(19) Europäisches Patentamt
European Patent Office
Office européen des brevets

(11) EP 1 243 271 A1

(12) EUROPEAN PATENT APPLICATION
published in accordance with Art. 158(3) EPC

(43) Date of publication:
**25.09.2002 Bulletin 2002/39**

(21) Application number: **00971740.6**

(22) Date of filing: **02.11.2000**

(51) Int Cl.[7]: **A61K 31/5025**, A61K 9/08, A61P 11/02, A61P 43/00, A61P 37/08 // (C07D521/00, 487:04)

(86) International application number:
**PCT/JP00/07719**

(87) International publication number:
**WO 01/034152 (17.05.2001 Gazette 2001/20)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.11.1999 JP 32107999**

(71) Applicant: **Takeda Chemical Industries, Ltd.**
**Osaka-shi, Osaka 541-0045 (JP)**

(72) Inventors:
- **NAGAYA, Hideaki**
**Toyonaka-shi, Osaka 561-0801 (JP)**
- **KAWANO, Yasuhiko**
**Suita-shi, Osaka 565-0806 (JP)**
- **KASHIHARA, Toshio**
**Suita-shi, Oaka 565-0872 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(54) **NASAL DROPS CONTAINING FUSED PYRIDAZINE DERIVATIVES**

(57) A nose drop containing the compound (I) represented by the formula

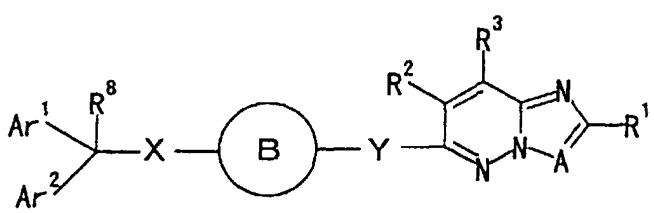

(I)

wherein $Ar^1$ and $Ar^2$ are each an aromatic group, $Ar^1$ and $Ar^2$ optionally form a condensed cyclic group together with the adjacent carbon atom, ring B is a nitrogen-containing heterocycle, X and Y are each a bond, an oxygen atom or S(O)p (p is 0 to 2), $NR^4$ ($R^4$ is H or a lower alkyl group) or a divalent linear lower hydrocarbon group optionally having substituents and containing 1 to 3 heteroatoms, A is N or $CR^7$ ($R^7$ is H, a halogen atom, a hydrocarbon group, an acyl group or a hydroxy group optionally having substituents), $R^1$, $R^2$ and $R^3$ are each H, a halogen atom, a hydrocarbon group, an acyl group or a hydroxy group optionally having substituents, and
$R^8$ is H, a hydroxy group optionally substituted by a lower alkyl group or a carboxyl group, provided that the nitrogen-containing heterocycle represented by ring B is not a heterocycle represented by the formula

EP 1 243 271 A1

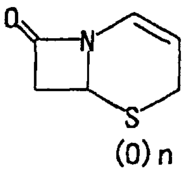

wherein n is 0 - 1, or a salt thereof or a prodrug thereof, exhibits a superior prophylactic or therapeutic effect on allergic rhinitis and the like.

## Description

### Technical Field

The present invention relates to a nose drop, which is useful as an agent for the prophylaxis or treatment of allergic rhinitis and the like.

### Background Art

WO98/49167 discloses that a compound of the formula

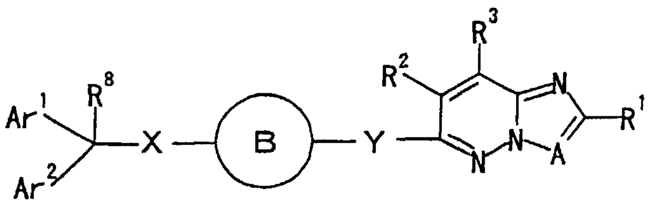

(I)

wherein $Ar^1$ and $Ar^2$ are each an aromatic group optionally having substituents, $Ar^1$ and $Ar^2$ optionally form a condensed cyclic group together with the adjacent carbon atom, ring B is a nitrogen-containing heterocycle optionally having substituents, X and Y are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, A is a nitrogen atom or $CR^7$ ($R^7$ is a hydrogen atom, a halogen atom, a hydrocarbon group optionally having substituents, an acyl group or a hydroxy group optionally having substituents), $R^1$, $R^2$ and $R^3$ are the same or different and each is a hydrogen atom, a halogen atom, a hydrocarbon group optionally having substituents, an acyl group or a hydroxy group optionally having substituents, and $R^8$ is a hydrogen atom, a hydroxy group optionally substituted by lower alkyl group or a carboxyl group, or a salt thereof is useful as an antihistaminic and/or eosinophil chemotaxis-inhibiting agent, an antiallergic agent, an agent for the prophylaxis or treatment of asthma, allergic conjunctivitis, allergic rhinitis, chronic urticaria or atopic dermatitis and the like. As concrete examples of the dosage form, tablets (including sugar-coated tablets and film-coated tablets), pills, capsules (including microcapsules), granules, fine subtilaes, powders, syrups, emulsions, suspensions, injectable preparations, inhalants and ointments are disclosed.

The development of a pharmaceutical preparation by which the above-mentioned compound or a salt thereof can efficiently exert a therapeutic effect on allergic rhinitis and the like has been desired.

### Disclosure of the Invention

The present inventors have intensively conducted various studies in an attempt to solve the above-mentioned various problems and found that, a compound represented by the formula

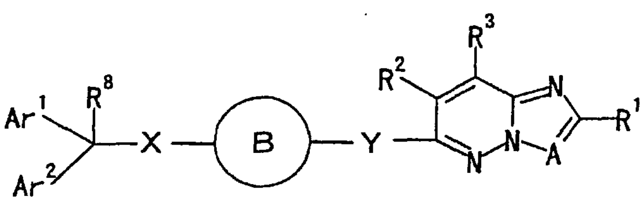

(I)

wherein

| | |
|---|---|
| $Ar^1$ and $Ar^2$ | are each an aromatic group optionally having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally |

having substituents, an acyl group or a hydroxy group optionally having substituents),

$R^1$, $R^2$ and $R^3$ are the same or different and each is a hydrogen atom, a halogen atom, a hydrocarbon group optionally having substituents, an acyl group or a hydroxy group optionally having substituents, and

$R^8$ is a hydrogen atom, a hydroxy group optionally substituted by a lower alkyl group or a carboxyl group,

provided that the nitrogen-containing heterocycle represented by ring B is not a heterocycle represented by the formula

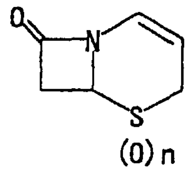

wherein n is 0 or 1, or a salt thereof or a prodrug thereof, having a great chemical structural characteristic in the presence of substituted piperidine or piperazine at the 6-position of [1,2,4]triazolo[1,5-b]pyridazine or imidazo[1,2-b]pyridazine skeleton containing a spacer, prepared into a nose drop unexpectedly exerts effectively a superior therapeutic effect on allergic rhinitis. The present inventors have further conducted investigation based on these findings and completed the present invention.

Accordingly, the present invention provides

[1] a nose drop agent containing a compound represented by the formula

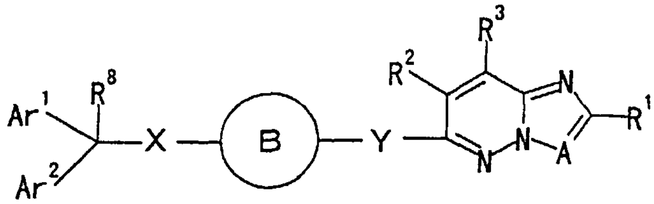

(I)

wherein

$Ar^1$ and $Ar^2$ are each an aromatic group optionally having substituents, $Ar^1$ and $Ar^2$ optionally form a condensed cyclic group together with the adjacent carbon atom,

ring B is a nitrogen-containing heterocycle optionally having substituents,

X and Y are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms,

A is a nitrogen atom or $CR^7$ ($R^7$ is a hydrogen atom, a halogen atom, a hydrocarbon group optionally having substituents, an acyl group or a hydroxy group optionally having substituents),

$R^1$, $R^2$ and $R^3$ are the same or different and each is a hydrogen atom, a halogen atom, a hydrocarbon group optionally having substituents, an acyl group or a hydroxy group optionally having substituents, and

$R^8$ is a hydrogen atom, a hydroxy group optionally substituted by a lower alkyl group or a carboxyl group,

provided that the nitrogen-containing heterocycle represented by ring B is not a heterocycle represented by the formula

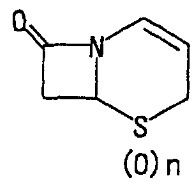

wherein n is 0 or 1, or a salt thereof or a prodrug thereof,

[2] the agent of [1], which is an antihistaminic and/or eosinophil chemotaxis-inhibiting agent,

[3] the agent of [1], which is an antiallergic agent,

[4] the agent of [1], which is an agent for the prophylaxis or treatment of allergic rhinitis,

[5] the agent of [1], which contains 2-[6-[3-[4-(diphenylmethoxy)piperidino]propylamino]imidazo[1,2-b]pyridazin-2-yl]-2-methylpropionic acid or a salt thereof,

[6] the agent of [5], wherein the 2-[6-[3-[4-(diphenylmethoxy)piperidino]propylamino]imidazo[1,2-b]pyridazin-2-yl]-2-methylpropionic acid is a dihydrate,

[7] a method for the prophylaxis or treatment of allergic rhinitis, which comprises administering, to a mammal, an effective amount of a nose drop containing a compound represented by the formula

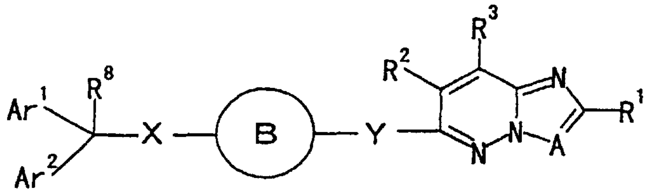 (I)

wherein

| | |
|---|---|
| $Ar^1$ and $Ar^2$ | are each an aromatic group optionally having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally having substituents, an acyl group or a hydroxy group optionally having substituents), |
| $R^1$, $R^2$ and $R^3$ | are the same or different and each is a hydrogen atom, a halogen atom, a hydrocarbon group optionally having substituents, an acyl group or a hydroxy group optionally having substituents, and |
| $R^8$ | is a hydrogen atom, a hydroxy group optionally substituted by a lower alkyl group or a carboxyl group, |

provided that the nitrogen-containing heterocycle represented by ring B is not a heterocycle represented by the formula

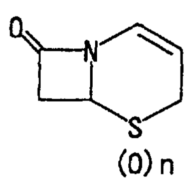

wherein n is 0 or 1, or a salt thereof or a prodrug thereof, and

[8] use of a compound represented by the formula

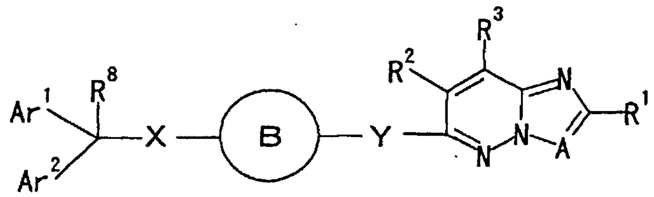

( I )

wherein

| | |
|---|---|
| $Ar^1$ and $Ar^2$ | are each an aromatic group optionally having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally having substituents, an acyl group or a hydroxy group optionally having substituents), |
| $R^1$, $R^2$ and $R^3$ | are the same or different and each is a hydrogen atom, a halogen atom, a hydrocarbon group optionally having substituents, an acyl group or a hydroxy group optionally having substituents, and |
| $R^8$ | is a hydrogen atom, a hydroxy group optionally substituted by a lower alkyl group or a carboxyl group, |

provided that the nitrogen-containing heterocycle represented by ring B is not a heterocycle represented by the formula

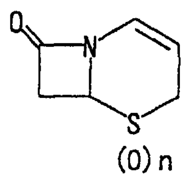

wherein n is 0 or 1, or a salt thereof or a prodrug for the production of a nose drop.

Furthermore, the present invention provides

[9] the agent of [1], wherein $Ar^1$ and $Ar^2$ are each

(1) a 6 to 14-membered monocyclic or condensed polycyclic aromatic hydrocarbon group (e.g., $C_{6-14}$ aryl group),

(2) a 5 to 8-membered monocyclic group containing, other than carbon atom, 1 or 2 kinds of 1 to 4 heteroatoms selected from nitrogen atom, sulfur atom and oxygen atom, or condensed aromatic heterocyclic group thereof or

(3) a monovalent group obtained by removing an optional hydrogen atom from the ring formed by condensation of the 6 to 14-membered monocyclic or condensed polycyclic aromatic hydrocarbon group of the above-mentioned (1) and the 5 to 8-membered monocyclic group containing, other than carbon atom, 1 or 2 kinds of 1 to 4 heteroatoms selected from nitrogen atom, sulfur atom and oxygen atom or condensed aromatic heterocyclic group thereof of the above-mentioned (2), which is optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy and (xxviii) $C_{7-16}$ aralkyloxy,

$Ar^1$ and $Ar^2$ optionally form, together with the adjacent carbon atom, a condensed cyclic group of the formula

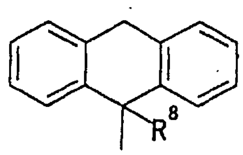 , 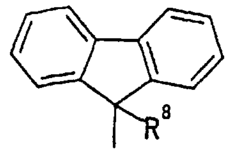 , 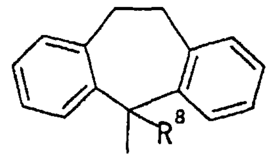 ,

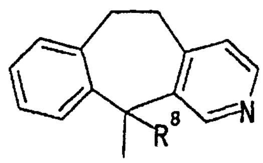 , 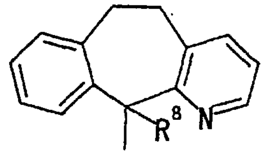 or 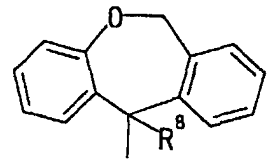

wherein $R^8$ is a hydrogen atom, a hydroxy group optionally substituted with $C_{1-6}$ alkyl or a carboxyl group, optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo,

ring B is a 3 to 13-membered nitrogen-containing heterocycle containing at least one nitrogen atom and optionally further containing 1 to 3 heteroatoms selected from nitrogen atom, oxygen atom and sulfur atom, which is optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkylcarbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo,

X and Y are the same or different and each is (1) a bond, (2) an oxygen atom, (3) S(O)p (p is an integer of 0 to 2), (4) $NR^4$ ($R^4$ is a hydrogen atom or a linear or branched $C_{1-6}$ alkyl group) or (5) a divalent linear $C_{1-6}$ hydrocarbon group optionally containing 1 to 3 heteroatoms selected from oxygen atom and sulfur atom and optionally having substituents selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo,

A is a nitrogen atom or $CR^7$ [$R^7$ is (1) a hydrogen atom, (2) a halogen atom, (3) a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy group, a $C_{6-14}$ aryl group or a $C_{7-16}$ aralkyl group, optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo,

(4) an acyl group represented by $-(C=O)-R^9$, $-SO_2-R^9$, $-SO-R^9$, $-(C=O)NR^{10}R^9$, $-(C=O)O-R^9$, $-(C=S)O-R^9$ or

-(C=S)$NR^{10}R^9$ ($R^9$ is (a) a hydrogen atom, (b) a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group, optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo or (c) -$OR^{11}$ ($R^{11}$ is a hydrogen atom, or a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo, and $R^{10}$ is a hydrogen atom or a $C_{1-6}$ alkyl group), or

(5) a group represented by -$OR^{12}$ ($R^{12}$ is a hydrogen atom, or a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo)],

$R^1$, $R^2$ and $R^3$ are each (1) a hydrogen atom, (2) a halogen atom, (3) a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo,

(4) an acyl group represented by -(C=O)-$R^{13}$, -$SO_2$-$R^{13}$, -SO-$R^{13}$, -(C=O)$NR^{14}R^{13}$, -(C=O)O-$R^{13}$, -(C=S)O-$R^{13}$ or -(C=S)$NR^{14}R^{13}$ ($R^{13}$ is (a) a hydrogen atom, (b) a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo, or

(c) a group represented by -$OR^{15}$ ($R^{15}$ is a hydrogen atom or a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene

ring optionally having 1 to 3 $C_{1-6}$ alkoxy groups, a $C_{6-14}$ aryl group or a $C_{7-16}$ aralkyl group, optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo, and $R^{14}$ is a hydrogen atom or a $C_{1-6}$ alkyl group, or
(5) a group represented by $-OR^{16}$ ($R^{16}$ is a hydrogen atom, or a $C_{1-6}$ alkyl group, a $C_{2-6}$ alkenyl group, a $C_{2-6}$ alkynyl group, a $C_{3-6}$ cycloalkyl group, a group obtained by condensing $C_{3-6}$ cycloalkyl group and benzene ring optionally having 1 to 3 $C_{1-6}$ alkoxy groups, a $C_{6-14}$ aryl group or a $C_{7-16}$ aralkyl group optionally substituted by a group selected from the group consisting of (i) halogen atom, (ii) $C_{1-6}$ alkylenedioxy, (iii) nitro, (iv) cyano, (v) optionally halogenated $C_{1-6}$ alkyl, (vi) optionally halogenated $C_{2-6}$ alkenyl, (vii) optionally halogenated $C_{2-6}$ alkynyl, (viii) $C_{3-6}$ cycloalkyl, (ix) $C_{1-6}$ alkoxy optionally having 1 to 3 halogen atoms, mono- or di-$C_{1-6}$ alkylamino or $C_{1-6}$ alkoxy-carbonyl, (x) optionally halogenated $C_{1-6}$ alkylthio, (xi) hydroxy, (xii) amino, (xiii) mono-$C_{1-6}$ alkylamino, (xiv) di-$C_{1-6}$ alkylamino, (xv) 5 or 6-membered cyclic amino, (xvi) $C_{1-6}$ alkyl-carbonyl, (xvii) carboxyl, (xviii) $C_{1-6}$ alkoxy-carbonyl, (xix) carbamoyl, (xx) thiocarbamoyl, (xxi) mono-$C_{1-6}$ alkyl-carbamoyl, (xxii) di-$C_{1-6}$ alkyl-carbamoyl, (xxiii) $C_{6-10}$ aryl-carbamoyl, (xxiv) sulfo, (xxv) $C_{1-6}$ alkylsulfonyl, (xxvi) $C_{6-10}$ aryl, (xxvii) $C_{6-10}$ aryloxy, (xxviii) $C_{7-16}$ aralkyloxy and (xxix) oxo, and
$R^8$ is a hydrogen atom, a hydroxy group optionally substituted with $C_{1-6}$ alkyl or a carboxyl group,

[10] the agent of [1], wherein $Ar^1$ and $Ar^2$ are each (1) a phenyl group optionally substituted by halogen atom or $C_{1-6}$ alkyl or (2) a 5 to 8-membered aromatic heterocyclic group containing, other than carbon atom, 1 to 4 heteroatoms selected from nitrogen atom, sulfur atom and oxygen atom, ring B is a ring represented by the formula

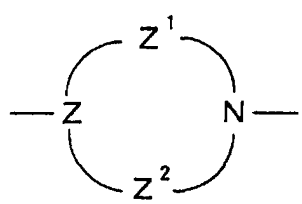

wherein Z is a nitrogen atom or a methyne group and $Z^1$ and $Z^2$ are each a linear $C_{1-4}$ alkylene group optionally substituted by hydroxy group, oxo group or $C_{1-6}$ alkyl group, X is a bond, an oxygen atom or NH, Y is a group represented by

(i) $C_{1-6}$ alkylene group,
(ii) $-(CH_2)p^1O-$,
(iii) $-(CH_2)p^1NH-$,
(iv) $-(CH_2)p^1S-$,
(v) $-(CH_2)q^1CH(OH)(CH_2)q^2O-$,
(vi) $-(CH_2)q^1CH(OH)(CH_2)q^2NH-$,
(vil) $-(CH_2)q^1CH(OH) (CH_2)q^2S-$,
(viil) $-(CH_2)p^1CONH-$,
(iX) $-COO(CH_2)p^1O-$,
(x) $-COO (CH_2)p^1NH-$,
(xi) $-COO(CH_2)p^1S-$,
(xii) $-(CH_2)q^1O(CH_2)q^2O-$,
(xiii)$-(CH_2)q^1O(CH_2)q^2NH-$ or
(xiv) $-(CH_2)q^1O(CH_2)q^2S-$ ($p^1$ is an integer of 1-6 and $q^1$ and $q^2$ are each an integer of 1-3), A is a nitrogen atom or $CR^{7'}$ ($R^{7'}$ is a hydrogen atom, a halogen atom, a $C_{1-6}$ alkyl group, a $C_{1-6}$ alkoxy-carbonyl group or a carboxyl group), $R^1$ is (1) a hydrogen atom, (2) a $C_{1-6}$ alkyl group optionally substituted by a group selected from the group consisting of (i) carboxyl, (ii) $C_{1-6}$ alkoxy-carbonyl, (iii) hydroxy and (iv) carbamoyl optionally having mono- or di-$C_{1-6}$ alkyl, (3) a $C_{6-14}$ aryl group, (4) a $C_{1-6}$ alkoxy group, (5) a $C_{1-6}$ alkoxy-carbonyl group, (6) a carboxyl group, (7) a carbamoyl group optionally having $C_{1-6}$ alkyl optionally substituted by carboxyl or

$C_{1-6}$ alkoxy-carbonyl, or (8) a $C_{3-6}$ cycloalkyl group optionally substituted by $C_{1-6}$ alkoxy-carbonyl, $R^2$ is a hydrogen atom, a $C_{1-6}$ alkyl group, a $C_{1-6}$ alkoxy-carbonyl group or a carboxyl group, $R^3$ is a hydrogen atom, and $R^8$ is a hydrogen atom or a hydroxy group,

[11] the agent of [1], wherein $Ar^1$ and $Ar^2$ are each a phenyl group, ring B is a ring of the formula

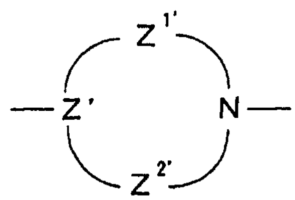

wherein Z' is a methyne group and $Z^{1'}$ and $Z^{2'}$ are methylene group or ethylene group, X is a bond or an oxygen atom, Y is a group represented by $-(CH_2)p^1NH-$ ($p^1$ is an integer of 1-6), A is $CR^{7''}$ ($R^{7''}$ is a hydrogen atom or a $C_{1-6}$ alkyl group) , $R^1$ is (1) a hydrogen atom, (2) a $C_{1-6}$ alkyl group (particularly, isopropyl group) optionally substituted by carboxyl or $C_{1-6}$ alkoxy-carbonyl or (3) a carbamoyl group optionally having $C_{1-6}$ alkyl optionally substituted by $C_{1-6}$ alkoxy-carbonyl, $R^2$ is a hydrogen atom, $R^3$ is a hydrogen atom and $R^8$ is a hydrogen atom,
[12] the agent of [1], wherein $Ar^1$ and $Ar^2$ are each an aromatic hydrocarbon group optionally having substituents,
[13] the agent of [1], wherein $Ar^1$ and $Ar^2$ are each a phenyl group optionally having substituents,
[14] the agent of [1], wherein $Ar^1$ and $Ar^2$ are each (1) a phenyl group optionally substituted by halogen atom or $C_{1-6}$ alkyl or (2) a 5 to 8-membered aromatic heterocyclic group containing, other than carbon atom, 1 to 4 heteroatoms selected from nitrogen atom, sulfur atom and oxygen atom,
[15] the agent of [1], wherein ring B is represented by a ring of the formula

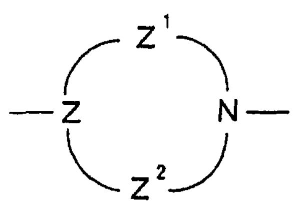

wherein Z is a nitrogen atom or a methyne group, $Z^1$ and $Z^2$ are each a linear $C_{1-4}$ alkylene group optionally substituted by hydroxy group, oxo group or $C_{1-6}$ alkyl group,
[16] the agent of [15], wherein $Z^1$ and $Z^2$ are each a linear $C_{1-2}$ alkylene group,
[17] the agent of [1], wherein X is a bond, an oxygen atom or NH,
[18] the agent of [1], wherein X is a bond or an oxygen atom,
[19] the agent of [1], wherein Y is a group represented by the formula

$$-(CH_2)m-Y^1-(CH_2)n-Y^2-$$

wherein $Y^1$ and $Y^2$ are the same or different and each is a bond, an oxygen atom, S(O)p (p is an integer of 0 to 2), $NR^4$ ($R^4$ is a hydrogen atom or a lower alkyl group), a carbonyl group, a carbonyloxy group or a group represented by the formula

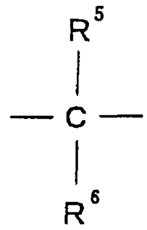

wherein $R^5$ and $R^6$ are the same or different and each is a hydroxy group or a $C_{1-4}$ alkyl group, and m and n are each an integer of 0 to 4 (provided the sum of m and n is not more than 6),
[20] the agent of [1], wherein Y is a group represented by

(i) $C_{1-6}$ alkylene group,
(ii) $-(CH_2)p^1O-$,
(iii) $-(CH_2)p^1NH-$,
(iv) $-(CH_2)p^1S-$,
(v) $-(CH_2)q^1CH(OH)(CH_2)q^2O-$,
(vi) $-(CH_2)q^1CH(OH)(CH_2)q^2NH-$,
(vii) $-(CH_2)q^1CH(OH)(CH_2)q^2S-$,
(viii) $-(CH_2)p^1CONH-$,
(ix) $-COO(CH_2)p^1O-$,
(x) $-COO(CH_2)p^1NH-$,
(xi) $-COO(CH_2)p^1S-$,
(xii) $-(CH_2)q^1O(CH_2)q^2O-$,
(xiii) $-(CH_2)q^1O(CH_2)q^2NH-$ or
(xiv) $-(CH_2)q^1O(CH_2)q^2S-$ ($p^1$ is an integer of 1-6 and $q^1$ and $q^2$ are each an integer of 1-3),

[21] the agent of [1], wherein $R^1$, $R^2$, $R^3$ and $R^7$ are the same or different and each is (1) a hydrogen atom, (2) a $C_{1-6}$ alkyl group optionally substituted by carboxyl group or $C_{1-6}$ alkoxy-carbonyl group, (3) a $C_{1-6}$ alkoxy group, (4) a $C_{1-6}$ alkoxy-carbonyl group or (5) a carboxyl group,
[22] the agent of [1], wherein $R^1$ is (1) a hydrogen atom, (2) a $C_{1-6}$ alkyl group optionally substituted by a group selected from the group consisting of (i) carboxyl, (ii) $C_{1-6}$ alkoxy-carbonyl, (iii) hydroxy and (iv) carbamoyl optionally having mono- or di-$C_{1-6}$ alkyl, (3) a $C_{6-14}$ aryl group, (4) a $C_{1-6}$ alkoxy group, (5) a $C_{1-6}$ alkoxy-carbonyl group, (6) a carboxyl group, (7) a carbamoyl group optionally having $C_{1-6}$ alkyl optionally substituted by carboxyl or $C_{1-6}$ alkoxy-carbonyl, or (8) a $C_{3-6}$ cycloalkyl group optionally substituted by $C_{1-6}$ alkoxy-carbonyl,
[23] the agent of [1], wherein $R^2$ is a hydrogen atom, a $C_{1-6}$ alkyl group, a $C_{1-6}$ alkoxy-carbonyl group or a carboxyl group,
[24] the agent of [1], wherein $R^3$ is a hydrogen atom,
[25] the agent of [1] wherein $R^7$ is a hydrogen atom, a halogen atom, a $C_{1-6}$ alkyl group, a $C_{1-6}$ alkoxy-carbonyl group or a carboxyl group,
[26] the agent of [1], wherein $R^8$ is a hydrogen atom or a hydroxy group,
[27] the agent of [1], wherein A is a nitrogen atom,
[28] the agent of [1], wherein A is $CR^{7'}$ ($R^{7'}$ is a hydrogen atom, a halogen atom, a $C_{1-6}$ alkyl group, a $C_{1-6}$ alkoxy-carbonyl group or a carboxyl group),
[29] the agent of [1], wherein A is CH,
[30] the agent of [1], wherein the compound is ethyl 2-[6-[3-[4-(diphenylmethoxy)piperidino]propylamino]imidazo [1,2-b]pyridazin-2-yl]-2-methylpropionate or a salt thereof,
[31] the agent of [1], wherein the compound is 2-[6-[3-[4-(diphenylmethoxy)piperidino]propylamino]imidazo[1,2-b] pyridazin-2-yl]-2-methylpropionic acid or a salt thereof,
[32] the agent of [1], wherein the compound is ethyl N-[6-[3-[4-(diphenylmethoxy)piperidino]propylamino]imidazo [1,2-b]pyridazine-2-carbonyl]glycinate or a salt thereof,
[33] the agent of [1], wherein the compound is ethyl 2-[6-[3-[4-(diphenylmethoxy)piperidino]propylamino]-3-methylimidazo[1,2-b]pyridazin-2-yl]-2-methylpropionate or a salt thereof,
[34] the agent of [1], wherein the compound is ethyl 2-[6-[3-[4-(diphenylmethylamino)piperidino]propylamino]imidazo[1,2-b]pyridazin-2-yl]-2-methylpropionate or a salt thereof,
[35] the agent of [1], wherein the compound is 2-[6-[3-[4-(diphenylmethoxy)piperidino]propylamino]-3-methylimidazo[1,2-b]pyridazin-2-yl]-2-methylpropionic acid or a salt thereof,
[36] the agent of [1], wherein the compound is a hydrate of the compound represented by the formula

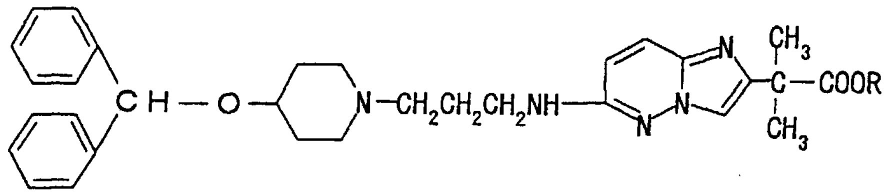

(I'')

wherein R is a hydrogen atom or ethyl group, or succinate or citrate of compound (I"),
[37] the agent of [1], wherein the compound is disuccinate of ethyl 2-[6-[3-[4-(diphenylmethoxy)piperidino]propylamino]-imidazo[1,2-b]pyridazin-2-yl]-2-methylpropionate, and
[38] the agent of [1], wherein the compound is citrate of ethyl 2-[6-[3-[4-(diphenylmethoxy)piperidino]propylamino]-imidazo[1,2-b]pyridazin-2-yl]-2-methylpropionate.

When compound (I) or a salt thereof contains an asymmetric carbon in the structure, any optically active form or racemate is encompassed in the scope of the present invention, and these compounds or salts thereof may be hydrates or anhydrates.

**Best Mode of Embodiment of the Invention**

In the above-mentioned formulas, $Ar^1$ and $Ar^2$ are each an "aromatic group optionally having substituents", and $Ar^1$ and $Ar^2$ may form a condensed cyclic group together with the adjacent carbon atom.
As the "aromatic group" represented by $Ar^1$ and $Ar^2$, for example,

(1) a monocyclic or condensed polycyclic aromatic hydrocarbon group, more specifically a 6 to 14-membered monocyclic or condensed polycyclic aromatic hydrocarbon group exemplified by $C_{6-14}$ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl, 9-phenanthryl and the like, and the like (preferably phenyl, tolyl, xylyl, biphenyl, 1-naphthyl, 2-naphthyl and the like, particularly preferably phenyl and the like), and the like, and
(2) a monocyclic group (preferably 5 to 8-membered) containing, other than carbon atom, preferably 1 or 2 kinds of 1 or more (e.g., 1 to 4, preferably 1 to 3) heteroatoms selected from nitrogen atom, sulfur atom and oxygen atom, or a condensed aromatic heterocyclic group thereof, more specifically aromatic heterocycle such as thiophene, benzo[b]thiophene, benzo[b]furan, benzimidazole, benzoxazole, benzothiazole, benzisothiazole, naphtho [2,3-b]thiophene, thianthrene, furan, isoindolizine, xanthrene, phenoxathiin, pyrrole, imidazole, triazole, thiazole, oxazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, isothiazole, phenothiazine, isoxazole, furazan, phenoxazine or isochroman and the like (preferably pyridine, thiophene or furan and the like, more preferably pyridine and the like), or
(3) a monovalent group obtained by removing an optional hydrogen atom from a ring formed by condensing these rings (preferably the aforementioned monocyclic heterocycle) with 1 or plural (preferably 1 or 2, more preferably 1) aromatic rings (e.g., the above-mentioned aromatic hydrocarbon group and the like, preferably benzene ring and the like), for example, the ring formed by condensation of the 6 to 14-membered monocyclic or condensed polycyclic aromatic hydrocarbon group of the above-mentioned (1) and the monocyclic group or a condensed aromatic heterocyclic group thereof containing, other than carbon atom, preferably 1 or 2 kinds of one or more heteroatoms selected from nitrogen atom, sulfur atom and oxygen atom, of the above-mentioned (2) and the like are used.

As the "aromatic group" of the "aromatic group optionally having substituents" represented by $Ar^1$ and $Ar^2$, for example, phenyl group and the like are preferable.
As the "substituent" of the aromatic group represented by $Ar^1$ and $Ar^2$, for example, (i) halogen atom (e.g., fluorine, chlorine, bromine, iodine), (ii) lower alkylenedioxy group (e.g., $C_{1-3}$ alkylenedioxy group such as methylenedioxy, ethylenedioxy and the like, and the like), (iii) nitro group, (iv) cyano group, (v) optionally halogenated lower alkyl group, (vi) optionally halogenated lower alkenyl group, (vii) optionally halogenated lower alkynyl group, (viii) lower cycloalkyl group (e.g., $C_{3-6}$ cycloalkyl group such as cyclopropyl, cyclobutyl, cyclopentyl, cyclohexyl and the like, and

the like), (ix) optionally substituted lower alkoxy group, (x) optionally halogenated lower alkylthio group, (xi) hydroxyl group, (xii) amino group, (xiii) mono-lower alkylamino group (e.g., mono-$C_{1-6}$ alkylamino group such as methylamino, ethylamino, propylamino, isopropylamino, butylamino and the like, and the like), (xiv) di-lower alkylamino group (e.g., di-$C_{1-6}$ alkylamino group such as dimethylamino, diethylamino, dipropylamino, dibutylamino and the like, and the like), (xv) 5 or 6-membered cyclic amino group (e.g., morpholino, piperazin-1-yl, piperidino, pyrrolidin-1-yl and the like), (xvi) lower alkyl-carbonyl group (e.g., $C_{1-6}$ alkyl-carbonyl group such as acetyl, propionyl and the like, and the like), (xvii) carboxyl group, (xviii) lower alkoxy-carbonyl group (e.g., $C_{1-6}$ alkoxy-carbonyl group such as methoxycarbonyl, ethoxycarbonyl, propoxycarbonyl, butoxycarbonyl and the like, and the like), (xix) carbamoyl group, (xx) thiocarbamoyl, (xxi) mono-lower alkyl-carbamoyl group (e.g., mono-$C_{1-6}$ alkyl-carbamoyl group such as methylcarbamoyl, ethylcarbamoyl and the like, and the like), (xxii) di-lower alkyl-carbamoyl group (e.g., di-$C_{1-6}$ alkylcarbamoyl group such as dimethylcarbamoyl, diethylcarbamoyl and the like, and the like), (xxiii) aryl-carbamoyl (e.g., $C_{6-10}$ aryl-carbamoyl such as phenylcarbamoyl, naphthylcarbamoyl and the like, and the like), (xxiv) sulfo group, (xxv) lower alkylsulfonyl group (e.g., $C_{1-6}$ alkylsulfonyl group such as methylsulfonyl, ethylsulfonyl and the like, and the like), (xxvi) aryl group (e.g., $C_{6-10}$ aryl group such as phenyl, naphthyl and the like, and the like), (xxvii) aryloxy group (e.g., $C_{6-10}$ aryloxy group such as phenoxy, naphthyloxy and the like, and the like), (xxviii) aralkyloxy group (e.g., $C_{7-16}$ aralkyloxy group such as benzyloxy and the like, and the like) and the like are used.

As the above-mentioned "optionally halogenated lower alkyl group", for example, lower alkyl group such as $C_{1-6}$ alkyl group (e.g., methyl, ethyl, propyl, isopropyl, butyl, isobutyl, sec-butyl, tert-butyl, pentyl, hexyl and the like, and the like) optionally having 1 to 3 halogen atoms, and the like are mentioned. Specific examples thereof include methyl, fluoromethyl, chloromethyl, difluoromethyl, trichloromethyl, trifluoromethyl, ethyl, 2-bromomethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the above-mentioned "optionally halogenated lower alkenyl group" and "optionally halogenated lower alkynyl group", for example, lower alkenyl group (e.g., $C_{2-6}$ alkenyl group such as vinyl, propenyl, isopropenyl, 2-buten-1-yl, 4-penten-1-yl, 5-hexen-1-yl, and the like, and the like) optionally having 1 to 3 halogen atoms, and lower alkynyl group (e.g., $C_{2-6}$ alkynyl group such as 2-butyn-1-yl, 4-pentyn-1-yl, 5-hexyn-1-yl, and the like, and the like), optionally having 1 to 3 halogen atoms (e.g., fluorine, chlorine, bromine, iodine), and the like are used.

As the above-mentioned "optionally substituted lower alkoxy group", for example, lower alkoxy group (e.g., $C_{1-6}$ alkoxy group such as methoxy, ethoxy, propoxy, isopropoxy, n-butoxy, isobutoxy, sec-butoxy, tert-butoxy and the like, and the like) optionally having 1 to 3 halogen atoms (e.g., fluorine, chlorine, bromine, iodine), mono- or di-lower alkylamino group (e.g., mono- or di-$C_{1-6}$ alkylamino group such as methylamino, dimethylamino, ethylamino, dimethylamino and the like, and the like) or lower alkoxy-carbonyl group (e.g., $C_{1-6}$ alkoxy-carbonyl group such as methoxycarbonyl, ethoxycarbonyl and the like, and the like), and the like are used.

As the above-mentioned "optionally halogenated lower alkylthio group", for example, lower alkylthio group (e.g., $C_{1-6}$ alkylthio group such as methylthio, ethylthio, n-propylthio, isopropylthio, n-butylthio, isobutylthio, sec-butylthio, tert-butylthio and the like, and the like) optionally having 1 to 3 halogen atoms (e.g., fluorine, chlorine, bromine, iodine), and the like are mentioned. Specific examples thereof include methylthio, difluoromethylthio, trifluoromethylthio, ethylthio, propylthio, isopropylthio, butylthio, 4,4,4-trifluorobutylthio, pentylthio, hexylthio and the like.

Specific examples of the condensed cyclic group formed by $Ar^1$ and $Ar^2$ together with the adjacent carbon atom include condensed cyclic group represented by

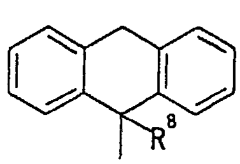 , 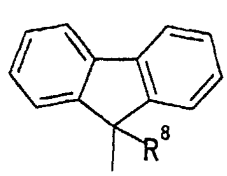 , 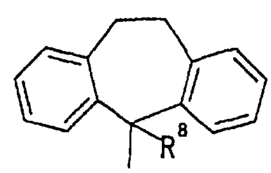 ,

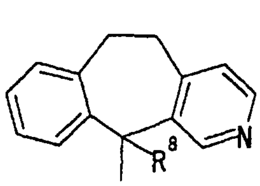 , 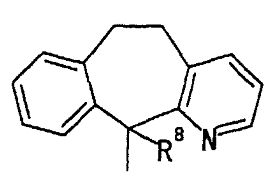 and 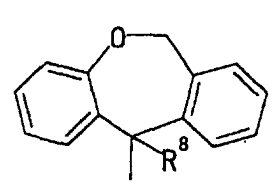

wherein $R^8$ is as defined above, and the like.

$Ar^1$ and $Ar^2$ are the same or different and each is preferably an aromatic hydrocarbon group optionally having substituents, such as a $C_{6-14}$ aromatic hydrocarbon group, more preferably a phenyl group optionally having substituents. More specifically, $Ar^1$ and $Ar^2$ are each preferably (1) a phenyl group optionally substituted by halogen atom or $C_{1-6}$ alkyl, (2) a 5 to 8-membered aromatic heterocyclic group containing, other than carbon atom, 1 to 4 heteroatoms selected from nitrogen atom, sulfur atom and oxygen atom, and the like.

In the above-mentioned formulas, ring B is a "nitrogen-containing heterocycle optionally having substituents". However, the nitrogen-containing heterocycle represented by ring B is not a heterocycle of the formula

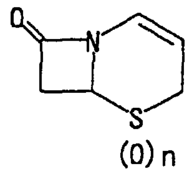

wherein n is 0 Or 1.

As the "nitrogen-containing heterocycle" represented by ring B, for example, a 3 to 13-membered nitrogen-containing heterocycle containing 1 nitrogen atom, and optionally containing 1 to 3 heteroatoms selected from, for example, nitrogen atom, oxygen atom, sulfur atom and the like, and the like are used. In the above-mentioned formulas, it is preferable to form a divalent group by removing one hydrogen atom each from nitrogen atom and the other atom of ring B. Specifically, for example, 3 to 9-membered (more preferably 3 to 6-membered) nitrogen-containing heterocyclic group of

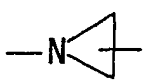 , 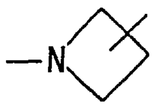 , 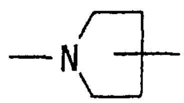 , 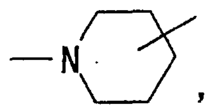 , 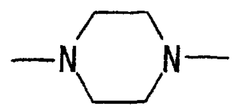

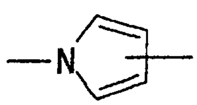 or 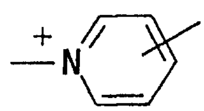

and the like, are preferable.

As the substituent of the nitrogen-containing heterocycle represented by ring B, for example, the "substituent" of the above-mentioned "aromatic group optionally having substituents" represented by $Ar^1$ and $Ar^2$, oxo group and the like are used.

Preferable examples of ring B include, for example, a ring represented by the formula

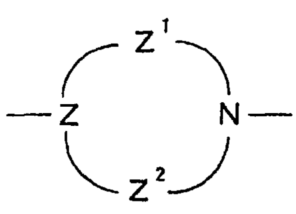

wherein Z is a nitrogen atom or a methyne group, and $Z^1$ and $Z^2$ are each a linear $C_{1-4}$ alkylene group optionally substituted by hydroxy group, oxo group or $C_{1-6}$ alkyl group, and the like.

As the "$C_{1-6}$ alkyl group", for example, linear or branched $C_{1-6}$ alkyl group such as methyl, ethyl, propyl, isopropyl, butyl, isobutyl, sec-butyl, tert-butyl, pentyl, hexyl and the like, and the like are used.

As the "linear $C_{1-4}$ alkylene group", for example, linear $C_{1-4}$ alkylene group represented by methylene, ethylene, propylene and butylene are used.

As the "linear $C_{1-4}$ alkylene group optionally substituted by hydroxy group, oxo group or $C_{1-6}$ alkyl group" which is represented by $Z^1$ and $Z^2$, an unsubstituted linear $C_{1-4}$ alkylene group and the like are preferably used, and an unsubstituted linear $C_{1-2}$ alkylene group is particularly preferable.

As ring B, piperidine, piperazine and the like are more preferably used.

In the above-mentioned formulas, X and Y are the same or different and each is (1) a bond, (2) an oxygen atom, (3) S(O)p (p is an integer of 0 to 2), (4) $NR^4$ ($R^4$ is a hydrogen atom or a lower alkyl group) or (5) a divalent linear lower hydrocarbon group optionally having substituents and containing 1 to 3 heteroatoms.

As the lower alkyl group represented by $R^4$, for example, linear or branched $C_{1-6}$ alkyl group such as methyl, ethyl, propyl, isopropyl, butyl, isobutyl, sec-butyl, tert-butyl, pentyl, hexyl and the like, and the like are used.

The "divalent linear lower hydrocarbon group optionally containing 1 to 3 heteroatoms" represented by X and Y, is a group obtained by removing one hydrogen atom bonded to each of the same or different carbon atoms of lower ($C_{1-6}$) hydrocarbon, namely 2 hydrogen atoms, which is, for example, a group optionally having a heteroatom selected from oxygen atom, sulfur atom and the like, in a hydrocarbon chain.

Specific examples of the "divalent linear lower hydrocarbon group" include

(i) $C_{1-6}$ alkylene group (e.g., $-CH_2-$, $-(CH_2)_2-$, $-(CH_2)_3-$, $-(CH_2)_4-$, $-(CH_2)_5-$, $-(CH_2)_6-$ and the like),
(ii) $C_{2-6}$ alkenylene group (e.g., $-CH=CH-$, $-CH=CH-CH_2-$, $-CH_2-CH=CH-CH_2-$, $-(CH_2)_2-CH=CH-CH_2-$, $-(CH_2)_2-CH=CH-(CH_2)_2-$, $-(CH_2)_3-CH=CH-CH_2-$ and the like),
(iii) $C_{2-6}$ alkynylene group (e.g., $-C\equiv C-$, $-C\equiv C-CH_2-$, $-CH_2-C\equiv C-CH_2-$, $-(CH_2)_2-C\equiv C-CH_2-$, $-(CH_2)_2-C\equiv C-(CH_2)_2-$, $-(CH_2)_3-C\equiv C-CH_2-$ and the like) and the like.

As the "substituent" of the "divalent linear lower hydrocarbon group optionally containing 1 to 3 heteroatoms" represented by X and Y, for example, the "substituent" of the above-mentioned "aromatic group optionally having substituents" represented by $Ar^1$ and $Ar^2$, oxo group and the like are used. Particularly, hydroxy group and oxo group are preferable.

As X, a bond, an oxygen atom or NH is preferable, and particularly, a bond or an oxygen atom is preferable.

As Y, preferred is, for example, a group of the formula

$$-(CH_2)m-Y^1-(CH_2)n-Y^2-$$

wherein $Y^1$ and $Y^2$ are the same or different and each is a bond, an oxygen atom, S(O)p (p is as defined above), $NR^4$ ($R^4$ is as defined above), a carbonyl group, a carbonyloxy group or a group of the formula

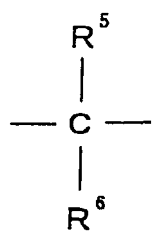

wherein $R^5$ and $R^6$ are the same or different and each is a hydroxy group or a $C_{1-4}$ alkyl group, and m and n are each an integer of 0 to 4 (provided that the sum of m and n is not more than 6, and the like.

As the "$C_{1-4}$ alkyl group" represented by $R^5$ and $R^6$, for example, linear or branched $C_{1-4}$ alkyl group such as methyl, ethyl, propyl, isopropyl, butyl, isobutyl, sec-butyl, tert-butyl and the like, and the like are used.

As Y, for example, a group represented by

(i) $C_{1-6}$ alkylene group,
(ii) $-(CH_2)p^1O-$,
(iii) $-(CH_2)p^1NH-$,
(iv) $-(CH_2)p^1S-$,
(v) $-(CH_2)q^1CH(OH)(CH_2)q^2O-$,
(vi) $-(CH_2)q^1CH(OH)(CH_2)q^2NH-$,
(vii) $-(CH_2)q^1CH(OH)(CH_2)q^2S-$,
(viii) $-(CH_2)p^1CONH-$,

(ix) $-COO(CH_2)p^1O-$,
(x) $-COO(CH_2)p^1NH-$,
(xi) $-COO(CH_2)p^1S-$,
(xii) $-(CH_2)q^1O(CH_2)q^2O-$,
(xiii) $-(CH_2)q^1O(CH_2)q^2NH-$ or
(xiv) $-(CH_2)q^1O(CH_2)q^2S-$ ($p^1$ is an integer of 1-6 and $q^1$ and $q^2$ are each an integer of 1-3) is preferable.

Of these, for example, Y is preferably a bond, $-(CH_2)_2-O-$, $-(CH_2)_3-O-$, $-(CH_2)_4-O-$, $-(CH_2)_6-O-$, $-(CH_2)_2-NH-$, $-(CH_2)_3-NH-$, $-(CH_2)_4-NH-$, $-(CH_2)_3-S-$, $-CH_2-CH(OH)-CH_2-O-$, $-(CH_2)_2-CO-NH-$, $-CH_2-CO-NH-$, $-CO-O-(CH_2)_2-O-$, $-CO-O-(CH_2)_3-O-$, $-(CH_2)_6-NH-$, - $(CH_2)_6-S-$, $-(CH_2)_2-O-(CH_2)_2-O-$, $-(CH_2)_2-O-(CH_2)_2-S-$ and the like.
In the above-mentioned formulas, A is a nitrogen atom or $CR^7$ ($R^7$ is a hydrogen atom, a halogen atom, a hydrocarbon group optionally having substituents, an acyl group or a hydroxy group optionally having substituents).
As the "halogen atom" represented by $R^7$, fluorine, chlorine, bromine and iodine are exemplified.
The "hydrocarbon group" represented by $R^7$ is, for example, a group obtained by removing one hydrogen atom from a hydrocarbon compound. Examples thereof include chain or cyclic hydrocarbon group such as alkyl group, alkenyl group, alkynyl group, cycloalkyl group, aryl group, aralkyl group and the like. Of these, chain (linear or branched) or cyclic hydrocarbon group having 1 to 16 carbon atoms and the like are preferable, and

a) alkyl group [preferably lower alkyl group (e.g., $C_{1-6}$ alkyl group such as methyl, ethyl, propyl, isopropyl, butyl, isobutyl, sec-butyl, tert-butyl, pentyl, hexyl and the like, and the like)],
b) alkenyl group [preferably lower alkenyl group (e.g., $C_{2-6}$ alkenyl group such as vinyl, allyl, isopropenyl, butenyl, isobutenyl, sec-butenyl and the like, and the like)],
c) alkynyl group [preferably lower alkynyl group (e.g., $C_{2-6}$ alkynyl group such as propargyl, ethynyl, butynyl, 1-hexynyl and the like, and the like)],
d) cycloalkyl group [preferably lower cycloalkyl group (e.g., $C_{3-6}$ cycloalkyl group such as cyclopropyl, cyclobutyl, cyclopentyl, cyclohexyl optionally condensed with benzene ring optionally having 1 to 3 lower alkoxy groups (e.g., $C_{1-6}$ alkoxy group such as methoxy and the like, and the like), and the like, and the like)],
e) aryl group (e.g., $C_{6-14}$ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl, 9-phenanthryl and the like, and the like, preferably phenyl group),
f) aralkyl group [preferably lower aralkyl group (e.g., $C_{7-16}$ aralkyl group such as benzyl, phenethyl, diphenylmethyl, 1-naphthylmethyl, 2-naphthylmethyl, 2-phenylethyl, 2-diphenylethyl, 1-phenylpropyl, 2-phenylpropyl, 3-phenylpropyl, 4-phenylbutyl, 5-phenylpentyl and the like, and the like, more preferably benzyl group)] and the like are preferable.

As the "substituent" of the "hydrocarbon group" represented by $R^7$, for example, the "substituent" of the above-mentioned "aromatic group optionally having substituents" represented by $Ar^1$ and $Ar^2$, oxo group and the like are used.
As the "acyl group" represented by $R^7$, for example, $-(C=O)-R^9$, $-SO_2-R^9$, $-SO-R^9$, $-(C=O)NR^{10}R^9$, $-(C=O)O-R^9$, $-(C=S)O-R^9$, $-(C=S)NR^{10}R^9$, ($R^9$ is a hydrogen atom, a hydrocarbon group optionally having substituents or a hydroxy group optionally having substituents, and $R^{10}$ is a hydrogen atom or a lower alkyl group (e.g., $C_{1-6}$ alkyl group such as methyl, ethyl, propyl, isopropyl, butyl, isobutyl, sec-butyl, tert-butyl, pentyl, hexyl and the like, and the like, particularly $C_{1-3}$ alkyl group such as methyl, ethyl, propyl, isopropyl and the like, and the like are preferable)) and the like are mentioned.
Of these, preferred are $-(C=O)-R^9$, $-SO_2-R^9$, $-SO-R^9$, $-(C=O)NR^{10}R^9$ and $-(C=O)O-R^9$, and $-(C=O)-R^9$ is more preferable.
The "hydrocarbon group" represented by $R^9$ is a group obtained by removing one hydrogen atom from a hydrocarbon compound. Examples thereof include chain (linear or branched) or cyclic hydrocarbon group such as alkyl group, alkenyl group, alkynyl group, cycloalkyl group, aryl group, aralkyl group and the like. Specific example thereof is the above-mentioned "hydrocarbon group" represented by $R^7$ and the like, and of these, chain or cyclic hydrocarbon group having 1 to 16 carbon atoms and the like are preferable, and lower ($C_{1-6}$) alkyl group is particularly preferable.
As the "substituent" that the "hydrocarbon group" represented by $R^9$ may have, for example, the "substituent" of the above-mentioned "aromatic group optionally having substituents" represented by $Ar^1$ and $Ar^2$, oxo group and the like are used.
As the "hydroxy group optionally having substituents" represented by $R^9$, for example, those similar to the "hydroxy group optionally having substituents" represented by $R^7$ to be mentioned later and the like are used.
As the "hydroxy group optionally having substituents" represented by $R^7$, for example, (1) a hydroxy group or (2) a hydroxy group having, for example, one aforementioned "hydrocarbon group optionally having substituents" and the like, instead of hydrogen atom of hydroxy group is used.

As $R^7$, (1) a hydrogen atom, (2) a $C_{1-6}$ alkyl group optionally substituted by carboxyl group or $C_{1-6}$ alkoxy-carboxyl, (3) a $C_{1-6}$ alkoxy group, (4) a $C_{1-6}$ alkoxy-carbonyl group or (5) a carboxyl group is preferable, and particularly, a hydrogen atom, a halogen atom, a $C_{1-6}$ alkyl group, a $C_{1-6}$ alkoxy-carbonyl group and a carboxyl group are preferable.

As A, a nitrogen atom or $CR^{7'}$ ($R^{7'}$ is a hydrogen atom, a halogen atom, a $C_{1-6}$ alkyl group, a $C_{1-6}$ alkoxy-carbonyl group or a carboxyl group) is preferable, and of these, nitrogen atom, CH and $C-CH_3$ are preferable, and nitrogen atom and CH are particularly preferable.

In the above-mentioned formulas, $R^1$, $R^2$ and $R^3$ are the same or different and each is a hydrogen atom, a halogen atom, a hydrocarbon group optionally having substituents, an acyl group or a hydroxy group optionally having substituents.

As the "halogen atom" represented by $R^1$, $R^2$ and $R^3$, fluorine, chlorine, bromine and iodine are exemplified.

As the "hydrocarbon group optionally having substituents" represented by $R^1$, $R^2$ and $R^3$, for example, the above-mentioned "hydrocarbon group optionally having substituents" represented by $R^7$, and the like are used.

As the "acyl group" represented by $R^1$, $R^2$ and $R^3$, for example, the above-mentioned "acyl group" represented by $R^7$, and the like are used.

As the "hydroxy group optionally having substituents" represented by $R^1$, $R^2$ and $R^3$, for example, the above-mentioned "hydroxy group optionally having substituents" represented by $R^7$, and the like are used.

$R^1$, $R^2$ and $R^3$ are the same or different and each is (1) a hydrogen atom, (2) a $C_{1-6}$ alkyl group optionally substituted by carboxyl group or $C_{1-6}$ alkoxy-carbonyl, (3) a $C_{1-6}$ alkoxy group, (4) a $C_{1-6}$ alkoxy-carbonyl group, (5) a carboxyl group or (6) a $C_{6-14}$ aryl group (particularly phenyl) is preferable, and (1) a hydrogen atom, (2) a $C_{1-6}$ alkyl group optionally substituted by carboxyl group and $C_{1-6}$ alkoxy-carbonyl, (3) a $C_{1-6}$ alkoxy group, (4) a $C_{1-6}$ alkoxy-carbonyl group or (5) a carboxyl group are more preferable.

As $R^1$, (1) a hydrogen atom, (2) a $C_{1-6}$ alkyl group optionally substituted by a group selected from the group consisting of (i) carboxyl, (ii) $C_{1-6}$ alkoxy-carbonyl, (iii) hydroxy or (iv) carbamoyl optionally having mono- or di-$C_{1-6}$ alkyl, (3) a $C_{6-14}$ aryl group, (4) a $C_{1-6}$ alkoxy group, (5) a $C_{1-6}$ alkoxy-carbonyl group, (6) a carboxyl group, (7) a carbamoyl group optionally having $C_{1-6}$ alkyl optionally substituted by carboxyl or $C_{1-6}$ alkoxy-carbonyl, or (8) a $C_{3-6}$ cycloalkyl group optionally substituted by $C_{1-6}$ alkoxy-carbonyl and the like are also preferable.

As $R^2$, a hydrogen atom, a $C_{1-6}$ alkyl group, a $C_{1-6}$ alkoxy-carbonyl group or a carboxyl group and the like are also preferable.

As $R^3$, a hydrogen atom is preferable.

In the above-mentioned formulas, $R^8$ is a hydroxy group optionally substituted by hydrogen atom or lower alkyl group.

In the above-mentioned the formulas, the "lower alkyl group" of "hydroxy group optionally substituted by lower alkyl group", which is represented by $R^8$ is, for example, $C_{1-6}$ alkyl group such as methyl, ethyl, propyl, isopropyl, butyl, isobutyl, sec-butyl, tert-butyl, pentyl, hexyl and the like, and the like.

As $R^8$, a hydrogen atom or a hydroxy group is preferable, and a hydrogen atom is particularly preferable.

As compound (I) of the present invention, a compound wherein $Ar^1$ and $Ar^2$ are each (1) a phenyl group optionally substituted halogen atom or $C_{1-6}$ alkyl or (2) a 5 to 8-membered aromatic heterocyclic group containing, other than carbon atom, 1 to 4 heteroatoms selected from nitrogen atom, sulfur atom and oxygen atom, ring B is a ring represented by the formula

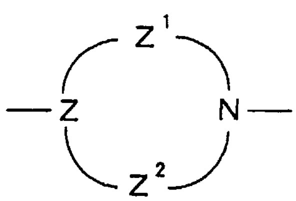

wherein Z is a nitrogen atom or a methyne group, and $Z^1$ and $Z^2$ are each a linear $C_{1-4}$ alkylene group optionally substituted by hydroxy group, oxo group or $C_{1-6}$ alkyl group, X is a bond, an oxygen atom or NH, Y is a group represented by

(i) $C_{1-6}$ alkylene group,
(ii) $-(CH_2)p^1O-$,
(iii) $-(CH_2)p^1NH-$,
(iv) $-(CH_2)p^1S-$,
(v) $-(CH_2)q^1CH(OH)(CH_2)q^2O-$,
(vi) $-(CH_2)q^1CH(OH)(CH_2)q^2NH-$,

(vii) $-(CH_2)q^1CH(OH)(CH_2)q^2S-$,
(viii) $-(CH_2)p^1CONH-$,
(ix) $-COO(CH_2)p^1O-$,
(x) $-COO(CH_2)p^1NH-$,
(xi) $-COO(CH_2)p^1S-$,
(xii) $-(CH_2)q^1O(CH_2)q^2O-$,
(xiii)$-(CH_2)q^1O(CH_2)q^2NH-$ or
(xiv) $-(CH_2)q^1O(CH_2)q^2S-$ ($p^1$ is an integer of 1-6, and $q^1$ and $q^2$ are each an integer of 1-3), A is a nitrogen atom or $CR^{7'}$ ($R^{7'}$ is a hydrogen atom, a halogen atom, a $C_{1-6}$ alkyl group, a $C_{1-6}$ alkoxy-carbonyl group or a carbonyl group), $R^1$ is (1) a hydrogen atom, (2) a $C_{1-6}$ alkyl group optionally substituted by a group selected from the group consisting of (i) carboxyl, (ii) $C_{1-6}$ alkoxy-carbonyl, (iii) hydroxy and (iv) carbamoyl optionally having mono- or di-$C_{1-6}$ alkyl, (3) a $C_{6-14}$ aryl group, (4) a $C_{1-6}$ alkoxy group, (5) a $C_{1-6}$ alkoxy-carbonyl group, (6) a carboxyl group, (7) a carbamoyl group optionally having $C_{1-6}$ alkyl optionally substituted by carboxyl or $C_{1-6}$ alkoxy-carbonyl, or (8) a $C_{3-6}$ cycloalkyl group optionally substituted by $C_{1-6}$ alkoxy-carbonyl, $R^2$ is a hydrogen atom, a $C_{1-6}$ alkyl group, a $C_{1-6}$ alkoxy-carbonyl group or a carboxyl group, $R^3$ is a hydrogen atom, $R^8$ is a hydrogen atom or a hydroxyl group is preferable.

particularly, a compound wherein $Ar^1$ and $Ar^2$ are each a phenyl group, ring B is a ring represented by the formula

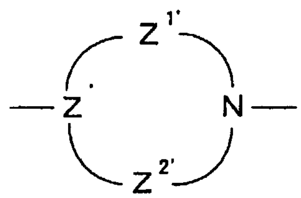

wherein Z' is a methyne group, and $Z^{1'}$ and $Z^{2'}$ are methylene group or ethylene group (preferably ethylene group), X is a bond, an oxygen atom or NH (preferably a bond or an oxygen atom), Y is a group represented by $-(CH_2)p^1NH-$ ($p^1$ is an integer of 1-6), A is $CR^{7''}$ ($R^{7''}$ is a hydrogen atom or a $C_{1-6}$ alkyl group), $R^1$ is (1) a hydrogen atom, (2) a $C_{1-6}$ alkyl group optionally substituted by carboxyl or $C_{1-6}$ alkoxy-carbonyl or (3) a carbamoyl group optionally having $C_{1-6}$ alkyl optionally substituted by $C_{1-6}$ alkoxy-carbonyl, $R^2$ is a hydrogen atom, $R^3$ is a hydrogen atom, and $R^8$ is a hydrogen atom, is preferable.

More specifically, the compounds produced in Reference Example B1 - Reference Example B138 to be mentioned later are used, and of those,

(1) ethyl 2-[6-[3-[4-(diphenylmethoxy)piperidino]-propylamino]imidazo[1,2-b]pyridazin-2-yl]-2-methylpropionate or a salt thereof (particularly, difumarate, disuccinate, citrate and the like),
(2) 2-[6-[3-[4-(diphenylmethoxy)piperidino]propylamino]-imidazo[1,2-b]pyridazin-2-yl]-2-methylpropionic acid or a salt thereof (particularly dihydrate),
(3) ethyl N-[6-[3-[4-(diphenylmethoxy)piperidino]-propylamino]imidazo[1,2-b]pyridazine-2-carbonyl]glycinate or a salt thereof,
(4) ethyl 2-[6-[3-[4-(diphenylmethoxy)piperidino]-propylamino]-3-methylimidazo[1,2-b]pyridazin-2-yl]-2-methylpropionate or a salt thereof (particularly dihydrochloride),
(5) ethyl 2-[6-[3-[4-(diphenylmethylamino)piperidino]-propylamino]imidazo[1,2-b]pyridazin-2-yl]-2-methylpropionate or a salt thereof,
(6) 2-[6-[3-[4-(diphenylmethoxy)piperidino]propylamino]-3-methylimidazo[1,2-b]pyridazin-2-yl]-2-methylpropionic acid or a salt thereof, and
(7) N-[6-[3-[4-(diphenylmethoxy)piperidino]propylamino]-3-methylimidazo[1,2-b]pyridazine-2-carbonyl]glycine or a salt thereof and the like are preferable.

The prodrug of the above-mentioned compound (I) or a salt thereof (hereinafter to be briefly referred to as compound (I) of the present invention) may be a compound that is converted to compound (I) by a reaction with an enzyme, gastric acid and the like in the body under physiological conditions. In other words, it may be a compound that undergoes enzymatic oxidation, reduction, hydrolysis and the like into compound (I) of the present invention, or a compound that undergoes hydrolysis and the like due to gastric acid and the like into compound (I) of the present invention.
Examples of the prodrug of compound (I) of the present invention include a compound wherein amino group

is acylated, alkylated or phosphorylated in compound (I) (e.g., a compound wherein amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated and the like, in compound (I) of the present invention); a compound wherein hydroxyl group is acylated, alkylated, phosphorylated or borated in compound (I) of the present invention (e.g., a compound wherein hydroxyl group is acetylated, palmitoylated, propanoylated, pivaloylated, succinylated, fumarylated, alanylated, dimethylaminomethylcarbonylated and the like, in compound (I) of the present invention); a compound wherein carboxyl group is esterified or amidated in compound (I) of the present invention (e.g., a compound wherein carboxyl group is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methyl amidated and the like, in compound (I) of the present invention); and the like. These compounds can be produced from compound (I) of the present invention by a method known *per se.*

The prodrug of compound (I) of the present invention may be a compound that is converted to compound of the present invention under the physiological conditions described in Iyakuhin no Kaihatsu (Development of Pharmaceutical Products), vol. 7, Molecule Design, pp. 163-198, Hirokawa Shoten (1990).

The production method of compound (I) or a salt thereof is explained in the following.

The compound (I) or a salt thereof can be produced by (A) reacting a compound represented by the formula

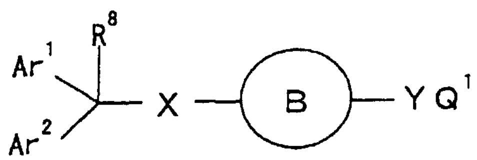

(II')

wherein $Q^1$ is a leaving group and other symbols are as defined above, or a salt thereof and a compound represented by the formula

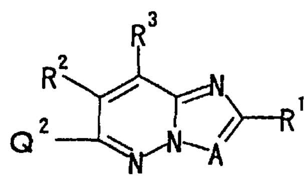

(III')

wherein $Q^2$ is a leaving group and other symbols are as defined above, or a salt thereof.

As the leaving group represented by $Q^1$, for example, an alkali metal such as sodium, potassium and the like, and the like are used. In addition, $Q^1$ may be a hydrogen atom.

As the leaving group represented by $Q^2$, for example, a halogen atom (e.g., chlorine, bromine, iodine and the like), a $C_{6-10}$ arylsulfonyloxy group (e.g., benzenesulfonyloxy, p-tolylsulfonyloxy and the like), a $C_{1-4}$ alkyl-sulfonyloxy group (e.g., methanesulfonyloxy and the like) and the like are used.

In this reaction, compound (II') or a salt thereof is generally used in an amount of about 1 to about 5 moles, preferably about 1 to about 2 moles, per mole of compound (III') or a salt thereof.

This condensation reaction is preferably carried out generally in the presence of a base. As the base, for example, alkali metal hydrides such as sodium hydride, potassium hydride and the like, alkali metal alkoxide such as sodium methoxide, sodium ethoxide and the like, alkali metal hydroxides such as sodium hydroxide, potassium hydroxide and the like, alkali metal carbonates such as sodium carbonate, potassium carbonate and the like, and the like are used.

This reaction can be also carried out in an inert solvent such as alcohols (e.g., methanol, ethanol and the like), ethers (e.g., dioxane, tetrahydrofuran and the like), aromatic hydrocarbons (e.g., such as benzene, toluene, xylene and the like), nitriles (e.g., acetonitrile and the like), amides (e.g., N,N-dimethylformamide, N,N-dimethylacetamide and the like), sulfoxides (e.g., dimethyl sulfoxide and the like) and the like.

The reaction temperature is generally about 10 to about 200°C, preferably about 50 to about 100°C.

The reaction time is generally about 30 minutes to about 24 hours, preferably about 1 to about 6 hours.

The compound (I) or a salt thereof can be produced by (B) reacting a compound represented by the formula

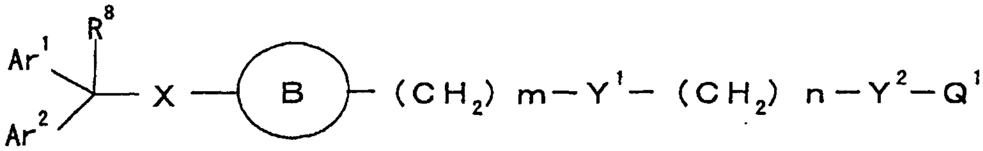

(IV)

wherein each symbol is as defined above, or a salt thereof and a compound represented by the formula

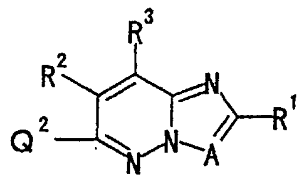

(III')

wherein each symbol is as defined above, or a salt thereof.

In this reaction, compound (IV) or a salt thereof is generally used in an amount of about 1 to about 5 moles, preferably about 1 to about 2 moles, per mole of compound (III') or a salt thereof. This condensation reaction is preferably carried out generally in the presence of a base. As the base, for example, alkali metal hydrides such as sodium hydride, potassium hydride and the like, alkali metal alkoxide such as sodium methoxide, sodium ethoxide and the like, alkali metal hydroxides such as sodium hydroxide, potassium hydroxide and the like, alkali metal carbonates such as sodium carbonate, potassium carbonate and the like, and the like are used.

This reaction can be also carried out in an inert solvent such as alcohols (e.g., methanol, ethanol and the like), ethers (e.g., dioxane, tetrahydrofuran and the like), aromatic hydrocarbons (e.g., benzene, toluene, xylene and the like), nitriles (e.g., acetonitrile and the like), amides (e.g., N,N-dimethylformamide, N,N-dimethylacetamide and the like), sulfoxides (e.g., dimethyl sulfoxide and the like), and the like.

The reaction temperature is generally about 10 to about 200°C, preferably about 50 to about 100°C.

The reaction time is generally about 30 minutes to about 24 hours, preferably about 1 to about 6 hours.

The compound (I) or a salt thereof can be produced by (C) reacting a compound represented by the formula

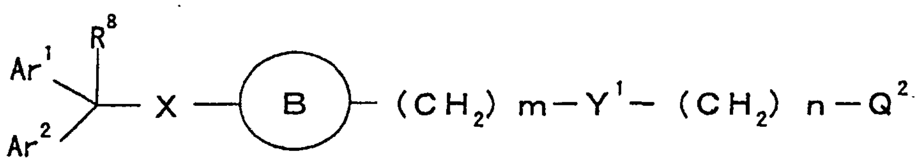

(V)

wherein each symbol is as defined above, or a salt thereof and a compound represented by the formula

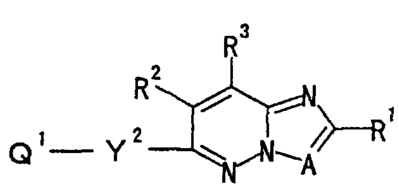

(VI')

wherein each symbol is as defined above, or a salt thereof.

In this reaction, compound (V) or a salt thereof is generally used in an amount of about 1 to about 5 moles, preferably about 1 to about 2 moles, per mole of compound (VI') or a salt thereof.

This condensation reaction is preferably carried out generally in the presence of a base. As the base, for example, alkali metal hydrides such as sodium hydride, potassium hydride and the like, alkali metal alkoxides such as sodium methoxide, sodium ethoxide and the like, alkali metal hydroxides such as sodium hydroxide, potassium hydroxide and the like, alkali metal carbonates such as sodium carbonate, potassium carbonate and the like, and the like are used.

This reaction can be also carried out in an inert solvent such as alcohols (e.g., methanol, ethanol and the like), ethers (e.g., dioxane, tetrahydrofuran and the like), aromatic hydrocarbons (e.g., benzene, toluene, xylene and the like), nitriles (e.g., acetonitrile and the like), amides (e.g., N,N-dimethylformamide, N,N-dimethylacetamide and the like), sulfoxides (e.g., dimethyl sulfoxide and the like), and the like.

The reaction temperature is generally about 10 to about 200°C, preferably about 50 to about 100°C.

The reaction time is generally about 30 minutes to about 24 hours, preferably about 1 to about 6 hours.

The compound (I) or a salt thereof can be produced by (D) reacting a compound represented by the formula

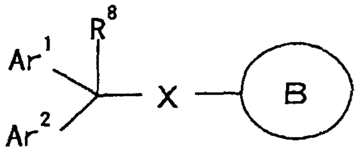

(VII)

wherein each symbol is as defined above, or a salt thereof and a compound represented by the formula

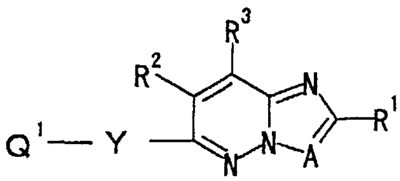

(VI)

wherein each symbol is as defined above, or a salt thereof.

In this reaction, compound (VII) or a salt thereof is generally used in an amount of about 1 to about 5 moles, preferably about 1 to about 2 moles, per mole of compound (VI) or a salt thereof.

This condensation reaction is preferably carried out generally in the presence of a base. As the base, for example, alkali metal hydrides such as sodium hydride, potassium hydride and the like, alkali metal alkoxides such as sodium methoxide, sodium ethoxide and the like, alkali metal hydroxides such as sodium hydroxide, potassium hydroxide and the like, alkali metal carbonates such as sodium carbonate, potassium carbonate and the like, and the like are used.

This reaction can be also carried out in an inert solvent such as alcohols (e.g., methanol, ethanol and the like), ethers (e.g., dioxane, tetrahydrofuran and the like), aromatic hydrocarbons (e.g., benzene, toluene, xylene and the like), nitriles (e.g., acetonitrile and the like), amides (e.g., N,N-dimethylformamide, N,N-dimethylacetamide and the like), sulfoxides (e.g., dimethyl sulfoxide and the like), and the like.

The reaction temperature is generally about 10 to about 200°C, preferably about 50 to about 100°C.

The reaction time is generally about 30 minutes to about 24 hours, preferably about 1 to about 6 hours.

The compound (I) or a salt thereof can be produced by, (E) reacting a compound represented by the formula

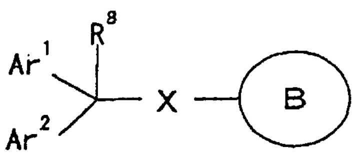

(VII)

wherein each symbol is as defined above, or a salt thereof and a compound represented by the formula

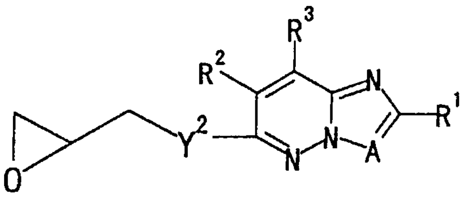

(VIII)

wherein each symbol is as defined above, or a salt thereof.

In this reaction, compound (VII) or a salt thereof is generally used in an amount of about 1 to about 5 moles, preferably about 1 to about 2 moles, per mole of compound (VIII) or a salt thereof.

This reaction can be also carried out in an inert solvent such as alcohols (e.g., methanol, ethanol and the like), ethers (e.g., dioxane, tetrahydrofuran and the like), aromatic hydrocarbons (e.g., benzene, toluene, xylene and the like), nitriles (e.g., acetonitrile and the like), amides (e.g., N,N-dimethylformamide, N,N-dimethylacetamide and the like), sulfoxides (e.g., dimethyl sulfoxide and the like) and the like.

The reaction temperature is generally about 10 to about 200°C, preferably about 50 to about 100°C.

The reaction time is generally about 30 minutes to about 24 hours, preferably about 1 to about 6 hours.

When the compound of the present invention thus obtained is a free compound, it can be converted to a salt by a conventional method. When it forms a salt, it can be converted to a free form or other salt by a conventional method. The compound of the present invention thus obtained or a salt thereof can be isolated and purified by any known procedures, such as solvent extraction, liquid-liquid transformation, phase transfer, salting out, crystallization, recrystallization, chromatography and the like. When the compound of the present invention or a salt thereof contains an optical isomer, it can be resolved into an R form or an S form by a conventional method for optical resolution.

As a salt of compound (I), for example, a salt with an inorganic salt (e.g., hydrochloric acid, phosphoric acid, hydrobromic acid, sulfuric acid and the like), a salt with an 行 organic acid (e.g., acetic acid, formic acid, propionic acid, fumaric acid, maleic acid, succinic acid, tartaric acid, citric acid, malic acid, oxalic acid, methanesulfonic acid, benzenesulfonic acid and the like), and the like are used. When compound (I) has, as a substituent, an acidic group such as carboxylic acid and the like, a salt with an inorganic base (e.g., alkali metal or alkaline earth metal such as sodium, potassium, calcium, magnesium and the like, and the like, or ammonia and the like) or an organic base (e.g., tri-$C_{1-3}$ alkylamine such as triethylamine and the like) may be formed.

The production methods of the starting material compounds (II) to (VIII) of the compound of the present invention and salts thereof are explained in the following. The salts of these starting material compounds are exemplified by those recited as the salts of compound (I).

The starting material compounds (II) and (IV) and salts thereof can be synthesized according to the method described in, for example, J. Med. Chem., vol. 32, p. 583 (1989) or a method similar thereto.

The starting material compound (III) and a salt thereof can be synthesized according to the method described in, for example, J. Org. Chem., vol. 39, p. 2143 (1974) or a method similar thereto.

The starting material compound (V) and a salt thereof can be synthesized according to the method described in, for example, JP-A-62-2739 and the like or a method similar thereto.

The starting material compounds (VI) and (VIII) and salts thereof can be synthesized according to the method described in, for example, JP-A-3-223287 and the like or a method similar thereto.

The starting material compound (VII) or a salt thereof can be synthesized according to the method described in, for example, J. Med. Chem., vol. 38, p. 2472 (1995) or a method similar thereto.

The starting material compound thus obtained and salts thereof can be isolated and purified by any known procedures, such as solvent extraction, liquid-liquid transformation, phase transfer, salting out, crystallization, recrystallization, chromatography and the like, but they may be used as a reaction mixture in the next step as a starting material without any isolation procedure.

When, in the aforementioned each reaction of the present invention and each reaction for the synthesis of the starting material compound, the starting material compound has an amino group, carboxyl group or hydroxyl group as a substituent, these groups may be protected by usual protective groups generally used in peptide chemistry and the like. The protective groups may be removed after the reaction as necessary, to give the desired compounds.

As the amino-protective group, for example, $C_{1-6}$ alkylcarbonyl (e.g., acetyl, ethylcarbonyl and the like), phenylcarbonyl, $C_{1-6}$ alkyl-oxycarbonyl (e.g., methoxycarbonyl, ethoxycarbonyl and the like), phenyloxycarbonyl, $C_{7-10}$ aralkyl-carbonyl (e.g., benzylcarbonyl and the like), trityl, phthaloyl, N,N-dimethylaminomethylene, all of which optionally having substituents, formyl, and the like are used. As these substituents, halogen atom (e.g., fluorine, chlorine, bromine, iodine and the like), $C_{1-6}$ alkyl-carbonyl (e.g., methylcarbonyl, ethylcarbonyl, butylcarbonyl and the like), nitro group and the like, are used, wherein the number of the substituents is about 1 to 3.

The carboxyl-protective group includes, for example, $C_{1-6}$ alkyl (e.g., methyl, ethyl, n-propyl, i-propyl, n-butyl, tert-butyl, etc.), phenyl, trityl, silyl, all of which optionally having substituents and the like are used. As these substituents, halogen atom (e.g., fluorine, chlorine, bromine, iodine and the like), formyl, $C_{1-6}$ alkyl-carbonyl (e.g., acetyl, ethylcarbonyl, butylcarbonyl and the like), nitro group and the like, are used, wherein the number of the substituents is about 1 to 3.

The hydroxyl-protective group includes, for example, $C_{1-6}$ alkyl (e.g., methyl, ethyl, n-propyl, i-propyl, n-butyl, tert-butyl and the like), phenyl, $C_{7-10}$ aralkyl (e.g., benzyl and the like), formyl, $C_{1-6}$ alkyl-carbonyl (e.g., acetyl, ethylcarbonyl and the like), phenyloxycarbonyl, benzoyl, $C_{7-10}$ aralkyl-carbonyl (e.g., benzylcarbonyl and the like), pyranyl, furanyl or silyl and the like, all of which optionally having substituents, are used. Examples of these substituents include halogen atom (e.g., fluorine, chlorine, bromine, iodine and the like), $C_{1-6}$ alkyl (e.g., methyl, ethyl, n-propyl and the like), phenyl, $C_{7-10}$ aralkyl (e.g., benzyl and the like), nitro group and the like, wherein the number of the substituents is about 1 to 4.

These protective groups may be removed by any method known *per se* or a similar method, such as treatment with acid, base, ultraviolet light, hydrazine, phenylhydrazine, sodium N-methyldithiocarbamate, tetrabutylammonium fluoride, palladium acetate and the like.

The compound (I), a salt thereof and a prodrug thereof have a superior antiallergic action, antihistaminic action, anti-inflammatory action, anti-PAF (platelet activating factor) action, eosinophil chemotaxis-inhibiting action and the like, and shows low toxicity (acute toxicity: LD50>2 g/kg). Therefore, they can be used as a safe antiallergic agent for mammal (e.g., human, mouse, dog, rat, bovine and the like).

The compound (I), a salt thereof and a prodrug thereof have, along with the antihistaminic action, an eosinophil chemotaxis-inhibiting action, and can be used for the prophylaxis or treatment of allergic disease, inflammatory diseases, skin diseases, respiratory diseases and the like in the aforementioned mammal.

Inasmuch as the compound (I), a salt thereof and a prodrug thereof can particularly inhibit nasal resistance, sneeze, nasal secretion, eye itching and ophthalmic inflammation, they are useful for the prophylaxis or treatment of the allergic or inflammatory diseases of nose and eye and the like.

The allergic diseases are exemplified by allergic skin diseases (e.g., eczema, eczema·dermatitis, contact dermatitis, itching, dry dermatitis, chronic urticaria, acute urticaria, prurigo, atopic dermatitis and the like), allergic rhinitis, allergic conjunctivitis, hypersensitivity pneumoitis and the like.

Examples of the skin diseases include allergic or inflammatory skin diseases such as atopic dermatitis, eczema, eczema·dermatitis, contact dermatitis, itching, dry dermatitis, chronic urticaria, acute urticaria, prurigo, dermatitis herpetiformis, psoriasis and the like.

Examples of the respiratory disease include asthma, eosinophilic pneumonia (PIE syndrome), chronic obstructive pulmonary disease (COPD) and the like.

Examples of the allergic or inflammatory diseases of nose include allergic rhinitis, upper airway hypersensitivity, pollinosis and the like.

Examples of the allergic or inflammatory diseases of eye include conjunctivitis (e.g., allergic conjunctivitis, giant papillary conjunctivitis, contact blepharoconjunctivitis and the like), vernal conjunctivitis, pollinosis and the like.

Of the above-mentioned diseases, compound (I), a salt thereof and a prodrug thereof are useful as an agent for the prophylaxis or treatment of the allergic or inflammatory diseases of nose and eye, such as conjunctivitis (e.g., allergic conjunctivitis, giant papillary conjunctivitis, contact blepharoconjunctivitis and the like), vernal conjunctivitis, allergic rhinitis, pollinosis and the like.

Moreover, the compound (I), a salt thereof and a prodrug thereof show decreased distribution in the brain as compared to previous condensed pyridazine compound.

These prophylactic or therapeutic agent can be orally or parenterally administered.

The preparation to be used in the present invention may contain, as an active ingredient, other pharmaceutical agent component besides the compound (I) or a salt thereof or a prodrug thereof.

Examples of such active ingredient of a pharmaceutical agent include antiasthmatic agent (e.g., theophylline, procaterol, ketotifen, azelastine, seratrodast and the like), antiallergic agent (e.g., ketotifen, terfenadine, azelastine, epinastine and the like), anti-inflammatory agent (e.g., diclofenac sodium, ibuprofen, indomethacin and the like), antibacterial agent (e.g., cefixime, cefdinir, ofloxacin, tosufloxacin and the like), antifungal agent (e.g., fluconazole, itraconazole and the like) and the like.

These components are free of any particular limitation as long as the object of the present invention can be achieved and can be admixed in a suitable mixing ratio. Specific examples of the dosage form include nose drop, eye drop, aerosol, eye ointment, plaster, suppository, troche, poultice, liniment, prolonged preparation, sustained-release preparation and the like.

A nose drop is particularly preferable for the prophylaxis or treatment of allergic rhinitis, pollinosis and the like and an eye drop is particularly preferable for the prophylaxis or treatment of conjunctivitis such as allergic conjunctivitis, giant papillary conjunctivitis, contact blepharoconjunctivitis and the like, vernal conjunctivitis and the like.

These preparations can be formulated using additives generally used for pharmaceutical preparations and according to a conventional method. For example, an eye drop can be produced according to the method described in General Notices of Japanese Pharmacopoeia and the like.

As additives usable for the nose drop and the eye drop of the present invention, for example, buffer agent, stabilizer, tackifier, suspending agent, preservative (antiseptics), isotonicity agent, solubilizer, pH adjusting agent, refrigerant and the like are used.

Examples of the buffer agent include sodium hydrogenphosphate, sodium dihydrogenphosphate, crystalline sodium dihydrogenphosphate, boric acid, borax, sodium carbonate, sodium L-glutamate, mixture of these, weak acid, weak base, a salt thereof, a mixture of these and the like.

Examples of the stabilizer include sulfur compounds such as sodium sulfite, sodium hydrogensulfite, sodium metahydrogensulfite, sodium thiosulfate, rongalite, thioglycerol, thioglycolic acid, thiolactic acid, cysteine, glutathione, thioacetic acid, methionine, thiosorbitol, thioglucose, thiourea and the like; inorganic acids and salts thereof, such as boric acid, borate, phosphoric acid, metaphosphoric acid, sodium carbonate, sodium hydrogencarbonate and the like; organic acid such as formic acid, oxalic acid, tartaric acid, citric acid, edetic acid and the like, and a salt thereof (e.g., sodium edetate); acid amide such as acetamide, diethylacetamide, nicotineamide, urea, barbital and the like; urea derivative; polyhydric alcohols such as glycol, propylene glycol, glycerine, polyethylene glycol, glucose, ascorbic acid and the like; saccharides; phenols such as phenol, thymol, quinone, coumarone, isocoumarone and the like; amino acid such as dibutylhydroxytoluene, glycine, glutamic acid, lysin, phenylalanine, casein, edestin and the like; protein and the like.

Examples of the tackifier or suspending agent include polyhydric alcohol such as glycerine, macrogol and the like; saccharides such as sorbitol, mannitol, sucrose and the like; celluloses such as methyl cellulose, carboxymethyl cellulose, hydroxypropylmethyl cellulose and the like; synthetic polymers such as polyvinyl alcohol, polyvinyl pyrrolidone, carboxyvinyl polymer and the like; chondroitin sulfate and the like.

Examples of the preservative (antiseptics) include p-oxybenzoic acid esters such as propyl p-oxybenzoate, butyl p-oxybenzoate and the like; parabens such as methyl paraben, ethyl paraben, propyl paraben, butyl paraben and the like; benzalkonium chloride; benzethonium chloride; chlorhexidine gluconate; invert soaps such as cetylpyridinium chloride and the like; alcohol derivatives such as chlorobutanol, phenylethyl alcohol, benzyl alcohol and the like; organic acid and a salt thereof such as sodium dehydroacetate, sorbic acid, sodium sorbate and the like; phenols such as p-chloromethoxyphenol, p-chlorometacresol and the like; organic mercury agents such as thimerosal, phenol mercury nitrate, phenyl mercury borate, phenyl mercury acetate, nitromezol and the like; polymixin B sulfate and the like.

Examples of the isotonicity agent include sodium chloride, calcium chloride, potassium chloride, conc. glycerine, propylene glycol, mannitol, sucrose and the like.

Examples of the solubilizer include polyvinylpyrrolidine K30, macrogol 4000, polyethylene glycol, propylene glycol, D-mannitol, benzyl benzoate, ethanol, trisaminomethane, cholesterol, triethanolamine, sodium carbonate, sodium citrate and the like.

Examples of the pH adjusting agent include sodium hydroxide and the like.

Examples of the refrigerant include 1-menthol, dl-camphor and the like.

The nose drop of the present invention can be produced as a solution or suspension using the aforementioned components according to a known method using, for example, manufacturing machine. It can be also used as a powder preparation upon trituration with a suitable excipient such as lactose and the like. Specifically, by uniformly dissolving or suspending the aforementioned components, or filling a powder preparation in a nose drop administration tool generally used such as accumulator (mechanical pump), pressure type (aerosol), squeeze type, inhalation type tool and the like, a product can be provided.

While the admixing ratio of the compound (I) or a salt thereof or a prodrug thereof in the preparation of the present invention varies depending on the form of the preparation, it is generally about 0.001 to about 30 wt%, preferably about 0.01 to about 10 wt%, more preferably about 0.05 to about 2 wt%, relative to the whole preparation.

While the dose of these preparations varies depending on the age, body weight, condition, administration route, administration frequency and the like, it is, for example, generally about 0.001 to about 30 mg/human, preferably about 0.01 to about 10 mg/human, more preferably about 0.05 to about 2 mg/human, in an active ingredient (compound (I) or a salt thereof or a prodrug thereof) for adult patients with allergic rhinitis per day, which is preferably instilled into the nose once or twice a day. Alternatively, the dose is, for example, generally about 0.001 to about 30 mg/human, preferably about 0.01 to about 10 mg/human, more preferably about 0.05 to about 2 mg/human, in an active ingredient (compound (I) or a salt thereof or a prodrug thereof) for adult patients with allergic conjunctivitis per day, which is preferably instilled into the eye once or twice a day

The present invention is explained in detail in the following by referring to Reference Examples, Examples and Experimental Examples, which are not to be construed as limitative.

The fraction containing the objective product in Reference Examples was detected by observation under TLC (Thin Layer Chromatography). In the TLC observation, $60F_{254}$ (Merck) was used as a TLC plate and a UV detector

was used for the detection method.

The powder X-ray diffraction analysis was performed as follows.

Using RINT 1100 (Rigaku) measuring apparatus, a sample was placed in a quartz (0 scattering) specimen holder for the measurement of XRPD pattern. A powder diffractometer equipped with a CuX-ray tube source, a first beam monochromator and a position sensitive detector (PSD) was used. The incident beam was made parallel using a 1·divergent slit. The X-ray tube source was set to 40 KV and 40 mA, and the sample was exposed to $CuK_{\alpha 1}$ radioactive rays. The XRPD data was measured at 3-35θ and 6.000./min. The most prominent peak was taken as 100% and the X ray peak reaching 30% or above thereof was depicted.

## Examples

### Reference Example B1

Production of 6-[3-[4-(diphenylmethyl)-1-piperazinyl]-propoxy][1,2,4]triazolo[1,5-b]pyridazine dihydrochloride

4-(Diphenylmethyl)-1-piperazinepropanol (466 mg) was dissolved in dried tetrahydrofuran (10 ml), followed by addition of sodium t-butoxide (173 mg). The mixture was refluxed under heating for 30 minutes. After the mixture was cooled, 6-chloro[1,2,4]triazolo[1,5-b]pyridazine (268 mg) was added to the mixture. The resulting mixture was refluxed under heating for 3 hours. After the mixture was cooled, ice-water was added thereto, followed by extraction with ethyl acetate. The extract was washed with brine, dried over magnesium sulfate and concentrated under reduced pressure. The residue was subjected to silica gel column chromatography and eluted with a mixture of ethyl acetate: methanol (10:1). Fractions containing the objective compound were collected and dissolved in ethyl acetate (10 ml), followed by addition of 4N HCl-ethyl acetate solution (0.7 ml). The resulting crystals were recrystallized from 95% aqueous alcohol to give the title compound (413 mg).
melting point: 251-253°C
elemental analysis for $C_{25}H_{30}N_6OCl_2$
Calculated (%): C,59.88 ; H,6.03 ; N,16.76
Found (%): C,59.76 ; H,6.09 ; N,16.80

### Reference Example B2

Production of 6-[3-[4-(diphenylmethoxy)piperidino]propoxy]-[1,2,4]triazolo[1,5-b]pyridazine fumarate

4-(Diphenylmethoxy)-1-piperidinepropanol (390 mg) was dissolved in dried tetrahydrofuran (10 ml), followed by addition of sodium t-butoxide (127 mg). The mixture was refluxed under heating for 30 minutes. After the mixture was cooled, 6-chloro[1,2,4]triazolo[1,5-b]pyridazine (215 mg) was added thereto. The resulting mixture was refluxed for 3 hours under heating. After the mixture was cooled, ice-water was added thereto, followed by extraction with ethyl acetate. The extract was washed with brine, dried over magnesium sulfate and concentrated under reduced pressure. The residue was subjected to silica gel column chromatography and eluted with a mixture of ethyl acetate:methanol: triethylamine (95:5:1). Fractions containing the objective compound were collected and dissolved in ethanol (10 ml). Fumaric acid (93 mg) was added to the solution to precipitate crystals. The resulting crystals were recrystallized from ethanol to give the title compound (218 mg).
melting point: 157-159°C
elemental analysis for $C_{30}H_{33}N_5O_6$
Calculated(%): C,64.39 ; H,5.94 ; N,12.51
Found(%): C,64.16 ; H,5.71 ; N,12.32

### Reference Example B3

Production of 6-[3-[4-(diphenylmethyl)-1-piperazinyl]-propoxy]-7-isopropyl[1,2,4]triazolo[1,5-b]pyridazine dihydrochloride

4-(Diphenylmethyl)-1-piperazinepropanol (466 mg) was dissolved in dried tetrahydrofuran (10 ml), followed by addition of sodium t-butoxide (173 mg). The mixture was refluxed under heating for 30 minutes. After the mixture was cooled, 6-chloro-7-isopropyl[1,2,4]triazolo[1,5-b]pyridazine (295 mg) was added thereto. The resulting mixture was refluxed for 3 hours under heating. After cooling, ice-water was added to the mixture, followed by extraction with ethyl acetate. The extract was washed with brine, dried over magnesium sulfate and concentrated under reduced pressure. The residue was subjected to silica gel column chromatography and eluted with a mixture of ethyl acetate:

methanol (10:1). Fractions containing the objective compound were collected and dissolved in ethanol (10 ml), followed by addition of 1N-HCl (3 ml). The mixture was concentrated under reduced pressure. The resulting crystals were recrystallized from ethyl acetate to give the title compound (582 mg).
melting point: 177°C
elemental analysis for $C_{28}H_{36}N_6OCl_2$
Calculated (%): C,59.89 ; H,6.82 ; N,14.97
Found (%): C,59.47 ; H,6.89 ; N,14.45

**Reference Example B4**

Production of 6-[3-[4-(diphenylmethoxy)piperidino]propoxy]-7-isopropyl[1,2,4]triazolo[1,5-b]pyridazine fumarate

4-(Diphenylmethoxy)-1-piperidinepropanol (488 mg) was dissolved in dried tetrahydrofuran (10 ml), followed by addition of sodium t-butoxide (173 mg). The mixture was refluxed under heating for 30 minutes. After the mixture was cooled, 6-chloro-7-isopropyl[1,2,4]triazolo[1,5-b]pyridazine (295 mg) was added thereto. The resulting mixture was refluxed for 3 hours under heating. After the mixture was cooled, ice-water was added thereto, followed by extraction with ethyl acetate. The extract was washed with brine, dried over magnesium sulfate and concentrated under reduced pressure. The residue was subjected to silica gel column chromatography and eluted with a mixture of ethyl acetate:methanol:triethylamine (95:5:1). Fractions containing the objective compound were collected and dissolved in ethanol (10 ml). Fumaric acid (98 mg) was added to the solution to precipitate crystals. The resulting crystals were recrystallized from ethanol to give the title compound (385 mg).
melting point: 163-165°C
elemental analysis for $C_{33}H_{39}N_5O_6$
Calculated (%): C,65.87 ; H,6.53 ; N,11.64
Found (%): C,65.77 ; H,6.46 ; N,11.71

**Reference Example B5**

Production of 6-[3-[4-(diphenylmethyl)-1-piperazinyl]-propylamino][1,2,4]triazolo[1,5-b]pyridazine

step A: Production of 6-(3-hydroxypropylamino)[1,2,4]-triazolo[1,5-b]pyridazine

6-Chloro[1,2,4]triazolo[1,5-b]pyridazine (928 mg) was dissolved in ethanol (10 ml). 3-Amino-1-propanol (1.23 g) was added to the solution. The mixture was refluxed under heating for 20 hours. After being cooled, the mixture was concentrated under reduced pressure to a half of its volume. The resulting precipitates were washed with ethanol and dried to give the title compound (835 mg).
melting point: 193-194°C
elemental analysis for $C_8H_{11}N_5O$
Calculated (%): C,49.73 ; H,5.74 ; N,36.25
Found (%): C,49.70 ; H,5.53 ; N,36.28

step B:

6-(3-Hydroxypropylamino)[1,2,4]triazolo[1,5-b]pyridazine (450 mg) was suspended in tetrahydrofuran (15 ml), and N-ethyldiisopropylamine (582 mg) and methanesulfonyl chloride (533 mg) were added to the suspension. The resulting mixture was stirred at room temperature for one hour. Ice-water and sodium chloride were added to the mixture, followed by extraction with ethyl acetate. The extract was washed with brine, dried over magnesium sulfate and concentrated under reduced pressure. The residue was dissolved in N,N-dimethylformamide (10 ml), followed by addition of 1-(diphenylmethyl)piperazine (504 mg), sodium iodide (298 mg) and potassium carbonate (276 mg). The mixture was stirred at 60°C for two hours. After the mixture was cooled, ice-water was added to the mixture, followed by extraction with ethyl acetate. The extract was washed with brine, dried over magnesium sulfate and concentrated under reduced pressure. The residue was subjected to silica gel column chromatography and eluted with a mixture of ethyl acetate:methanol:triethylamine (90:10:1). Fractions containing the objective compound were collected and concentrated. The resulting crystals were washed with ethyl ether and dried to give the title compound (281 mg).
melting point: 139-140°C
elemental analysis for $C_{25}H_{29}N_7 \cdot 0.5H_2O$
Calculated (%): C,68.78 ; H,6.93 ; N,22.46
Found (%): C,68.72 ; H,6.86 ; N,22.16

**Reference Example B6**

Production of 6-[3-[4-(diphenylmethoxy)piperidino]-propylamino][1,2,4]triazolo[1,5-b]pyridazine

6-(3-Hydroxypropylamino)[1,2,4]triazolo[1,5-b]pyridazine (290 mg) was suspended in tetrahydrofuran (10 ml). N-Ethyldiisopropylamine (388 mg) and methanesulfonyl chloride (344 mg) were added to the suspension, and the mixture was stirred at room temperature for one hour. Ice-water and sodium chloride were added to the mixture, followed by extraction with ethyl acetate. The extract was washed with brine, dried over magnesium sulfate and concentrated under reduced pressure. The residue was dissolved in N,N-dimethylformamide (5 ml), followed by addition of 4-(dipheylmethoxy)piperidine (352 mg), sodium iodide (208 mg) and potassium carbonate (193 mg). The mixture was stirred at room temperature for 15 hours and at 60°C for 3 hours. After the mixture was cooled, ice-water was added thereto, followed by extraction with ethyl acetate. The extract was washed with brine, dried over magnesium chloride and concentrated under reduced pressure. The residue was subjected to silica gel column chromatography and eluted with a mixture of ethyl acetate:methanol:triethylamine (90:10:1). Fractions containing the objective compound were concentrated. The resulting crystals were washed with ethyl ether and dried to give the title compound (209 mg).
melting point: 136-138°C
elemental analysis for $C_{26}H_{30}N_6O$
Calculated (%): C,70.56 ; H,6.83 ; N,18.99
Found (%): C,70.43 ; H,6.83 ; N,19.04

**Reference Example B7**

Production of 6-[3-[4-(diphenylmethyl)-1-piperazinyl]-propylthio][1,2,4]triazolo[1,5-b]pyridazine

step A: Production of 6-(3-bromopropylthio)[1,2,4]-triazolo[1,5-b]pyridazine

Methyl 3-mercaptopropionate (3.9 ml) was dissolved in methanol (40 ml), followed by addition of a 2N sodium methoxide solution in methanol (15 ml) and 6-chloro[1,2,4]-triazolo[1,5-b]pyridazine (1.55 g). The mixture was refluxed under heating for one hour. After being cooled, the mixture was concentrated under reduced pressure. Ethyl acetate was added to the residue. The resulting crystals were collected, washed with ethyl acetate and suspended in tetrahydrofuran (40 ml), followed by addition of 1,3-dibromopropane (3.06 ml). The mixture was refluxed under heating for two hours. After the mixture was cooled, ice-water was added thereto. The mixture was extracted with ethyl acetate. The extract was washed with brine, dried over magnesium sulfate and concentrated under reduced pressure. A mixed solvent of ethyl acetate:hexane (1:1) was added to the residue. The resulting crystals were collected and dried to give the title compound (1.97 g).
melting point: 133-135°C
elemental analysis for $C_8H_9N_4SBr$
Calculated (%): C,35.18 ; H,3.32 ; N,20.51
Found (%): C.35.11 ; H,3.13 ; N,20.43

step B:

6-(3-Bromopropylthio)[1,2,4]triazolo[1,5-b]pyridazine (546 mg) and 1-(diphenylmethyl)piperazine (505 mg) were dissolved in acetonitrile (15 ml), followed by addition of sodium iodide (373 mg) and potassium carbonate (277 mg). The mixture was stirred at 50-60°C for 15 hours, followed by addition of ice-water and extraction with ethyl acetate. The extract was washed with brine, dried over magnesium sulfate and concentrated under reduced pressure. The residue was subjected to silica gel column chromatography and eluted with a mixture of ethyl acetate:methanol (95: 5). Fractions containing the objective compound were collected and concentrated. The resulting crystals were collected, washed with ethyl ether and dried to give the title compound (507 mg).
melting point: 128-130°C
elemental analysis for $C_{25}H_{28}N_6S$
Calculated (%): C,67.54 ; H,6.35 ; N,18.90
Found (%): C,67.25 ; H,6.29 ; N,18.78

**Reference Example B8**

Production of 6-[3-[4-(diphenylmethoxy)piperidino]-propylthio][1,2,4]triazolo[1,5-b]pyridazine fumarate

6-(3-Bromopropylthio)[1,2,4]triazolo[1,5-b]pyridazine (546 mg) and 4-(diphenylmethoxy)piperidine (535 mg) were dissolved in acetonitrile (15 ml), followed by addition of sodium iodide (373 mg) and potassium carbonate (277 mg). The mixture was stirred at 50-60°C for 15 hours, followed by addition of ice-water after cooled and extraction with ethyl acetate. The extract was washed with brine, dried over magnesium sulfate and concentrated under reduced pressure. The residue was subjected to silica gel column chromatography and eluted with a mixture of ethyl acetate: methanol:triethylamine (95:5:1). Fractions containing the objective compound were collected and concentrated. The residue was dissolved in ethanol (20 ml), followed by addition of fumaric acid (159 mg). The resulting crystals were collected, washed with ethyl ether and dried to give the title compound (435 mg).
melting point: 185-187°C
elemental analysis for $C_{30}H_{33}N_5O_5S \cdot 0.5H_2O$
Calculated (%): C,61.63 ; H,5.86 ; N,11.98
Found (%): C,61.98 ; H,5.83 ; N,11.95

**Reference Example B9**

Production of 6-[3-[4-(diphenylmethyl)-1-piperazinyl]-propylthio]-7-isopropyl[1,2,4]triazolo[1,5-b]pyridazine

step A: Production of 6-(3-chloropropylthio)-7-isopropyl[1,2,4]triazolo[1,5-b]pyridazine

Methyl 3-mercaptopropionate (3.9 ml) was dissolved in methanol (40 ml), followed by addition of 2N sodium methoxide in methanol solution (15 ml) and 6-chloro-7-isopropyl[1,2,4]-triazolo[1,5-b]pyridazine (1.97 g). The mixture was refluxed under heating for 40 minutes. After being cooled, the mixture was concentrated under reduced pressure. Ethyl acetate was added to the residue. The resulting crystals were collected, washed with ethyl acetate and suspended in tetrahydrofuran (40 ml), followed by addition of 1-bromo-3-chloropropane (2 ml). The mixture was refluxed under heating for two hours. After the mixture was cooled, ice-water was added thereto, followed by extraction with ethyl acetate. The extract was washed with brine, dried over magnesium sulfate and concentrated under reduced pressure. The residue was subjected to silica gel column chromatography and eluted with a mixture of hexane:ethyl acetate (2: 3). Fractions containing the objective compound were collected and concentrated under reduced pressure. The resulting crystals were filtered and dried to give the title compound (2.39 g).
melting point: 82-83°C
elemental analysis for $C_{11}H_{15}N_4SCl$
Calculated (%): C,48.79 ; H,5.58 ; N,20.69
Found (%): C,48.79 ; H,5.53 ; N,20.87

step B:

6-(3-Chloropropylthio)-7-isopropyl[1,2,4]triazolo [1,5-b]pyridazine (542 mg) and 1-(diphenylmethyl)piperazine (555 mg) were dissolved in acetonitrile (15 ml), followed by addition of sodium iodide (447 mg) and potassium carbonate (277 mg). The mixture was refluxed under heating for 20 hours. After the mixture was cooled, ice-water was added thereto, followed by extraction with ethyl acetate. The extract was washed with brine, dried over magnesium sulfate and concentrated under reduced pressure. The residue was subjected to silica-gel column chromatography and eluted with ethyl acetate. Fractions containing the objective compound were collected and concentrated. The resulting crystals were recrystallized from a mixture of ethyl acetate and ethyl ether (1:1) and dried to give the title compound (607 mg).
melting point: 137-139°C
elemental analysis for $C_{28}H_{34}N_6S$
Calculated (%): C,69.10 ; H,7.04 ; N,17.27
Found (%): C,69.04 ; H,7.06 ; N,17.33

**Reference Example B10**

Production of 6-[3-[4-(diphenylmethoxy)piperidino]-propylthio]-7-isopropyl[1,2,4]triazolo[1,5-b]pyridazine fumarate

6-(3-Chlolopropylthio)-7-isopropyl[1,2,4]triazolo[1,5-b]pyridazine (542 mg) and 4-(diphenylmethoxy)piperidine (535 mg) were dissolved in acetonitrile (15 ml), followed by addition of sodium iodide (447 mg) and potassium carbonate (277 mg). The mixture was refluxed under heating for 15 hours. After the mixture was cooled, ice-water was added thereto, followed by extraction with ethyl acetate. The extract was washed with brine, dried over magnesium sulfate and concentrated under reduced pressure. The residue was subjected to silica-gel column chromatography, followed by elution with a mixture of ethyl acetate:methanol (95:5). Fractions containing the objective compound were collected and concentrated. The residue was dissolved in ethanol (20 ml), followed by addition of fumaric acid (196 mg). The resulting crystals were collected, washed with ethanol and dried to give the title compound (780 mg).
melting point: 164-165°C
elemental analysis for $C_{33}H_{39}N_5O_5S$
Calculated (%): C,64.16 ; H,6.36 ; N,11.34
Found (%) : C,64.45 ; H,6.49 ; N,11.67

**Reference Example B11**

Production of 6-[4-(diphenylmethoxy)piperidino]-[1,2,4]triazolo[1,5-b]pyridazine

4-(Diphenylmethoxy)piperidine (1.12 g) and 6-chloro[1,2,4]triazolo[1,5-b]pyridazine (558 mg) were dissolved in 1-butanol (25 ml), followed by addition of N-ethyldiisopropylamine (700 mg). The mixture was refluxed under heating for 17 hours. After being cooled, the mixture was concentrated under reduced pressure. Ice-water was added to the residue, followed by extraction with ethyl acetate. The extract was washed with brine, dried over magnesium sulfate and concentrated under reduced pressure. The residue was subjected to silica-gel column chromatography and eluted with a mixture of hexane:ethyl acetate (1:3). Fractions containing the objective compound were collected and recrystallized from ethanol to give the title compound (757 mg).
melting point: 137-139°C
elemental analysis for $C_{23}H_{23}N_5O$
Calculated (%): C,71.67 ; H,6.01 ; N,18.17
Found (%): C,71.75 ; H,5.90 ; N,18.34

**Reference Example B12**

Production of 6-[4-[4-(diphenylmethoxy)piperidino]-butylamino][1,2,4]triazolo[1,5-b]pyridazine

4-(Diphenylmethoxy)-1-piperidinebutanamine (1.83 g) and 6-chloro[1,2,4]triazolo[1,5-b]pyridazine (557 mg) were dissolved in 1-butanol (30 ml), followed by addition of N-ethyldiisopropylamine (931 mg). The mixture was refluxed under heating for 14 hours. After being cooled, the mixture was concentrated under reduced pressure, followed by addition of ice-water and extraction with ethyl acetate. The extract was washed with brine, dried over sodium sulfate and concentrated under reduced pressure. The residue was subjected to a silica-gel column chromatography and eluted with a mixture of ethyl acetate:methanol:triethylamine (45:5:1). Fractions containing the objective compound were collected and concentrated. The resulting crystals were collected, washed with ethyl ether and dried to give the title compound (149 mg).
melting point: 102-104°C
elemental analysis for $C_{27}H_{32}N_6O$
Calculated (%): C,71.03 ; H,7.06 ; N,18.41
Found (%): C,70.78 ; H,6.77 ; N,18.40

**Reference Example B13**

Production of 6-[2-[4-(diphenylmethoxy)piperidino]-ethylamino][1,2,4]triazolo[1,5-b]pyridazine

step A: Production of 6-(2-hydroxyethylamino)[1,2,4]-triazolo[1,5-b]pyridazine

2-Aminoethanol (2.01 g) and 6-chloro[1,2,4]-triazolo[1,5-b]pyridazine (2.03 g) were dissolved in ethanol (22 ml). The mixture was refluxed under heating for 20 hours. After being cooled, the mixture was concentrated under reduced pressure. The resulting crystals were collected and dried to give the title compound (1.48 g).
melting point: 219-221°C
elemental analysis for $C_7H_9N_5O$
Calculated (%): C,46.92 ; H,5.06 ; N,39.09
Found (%): C,46.67 ; H,5.00 ; N,38.93

step B:

6-(2-Hydroxyethylamino)[1,2,4]triazolo[1,5-b]pyridazine (1.25 g) was suspended in tetrahydrofuran (40 ml), followed by addition of N-ethyldiisopropylamine (1.81 g) and methanesulfonyl chloride (1.60 g). The mixture was stirred at room temperature for 45 minutes, followed by addition of ice-water and sodium chloride to be saturated therewith. The mixture was extracted with ethyl acetate. The extract was washed with brine, dried over magnesium sulfate and concentrated under reduced pressure. The residue was dissolved in N,N-dimethylformamide (21 ml), followed by addition of 4-(diphenylmethoxy)piperidine (1.79 g), sodium iodide (1.00 g) and potassium carbonate (927 mg). The mixture was stirred at room temperature for 15 hours and at 60°C for 1.5 hours, followed by addition of ice-water and extraction with ethyl ether. The extract was washed with brine dried over magnesium sulfate and concentrated under reduced pressure. The resulting crystals were collected, washed with ethyl ether and dried to give the title compound (1.13 g).
melting point: 152-154°C
elemental analysis for $C_{25}H_{28}N_6O$
Calculated (%): C,70.07 ; H,6.59 ; N,19.61
Found (%): C,69.66 ; H,6.40 ; N,20.03

**Reference Example B14**

Production of 6-[2-[4-(diphenylmethoxy)piperidino]-ethoxy][1,2,4]triazolo[1,5-b]pyridazine fumarate

4-(Diphenylmethoxy)-1-piperidineethanol (774 mg) was dissolved in dried tetrahydrofuran (20 ml), followed by addition of sodium tert-butoxide (263 mg). The mixture was refluxed under heating for 30 minutes. After the mixture was cooled, 6-chloro[1,2,4]triazolo[1,5-b]pyridazine (385 mg) was added thereto. The mixture was refluxed under heating for 6 hours. After the mixture was cooled, ice-water was added to the mixture, followed by extraction with ethyl acetate. The extract was washed with brine, dried over magnesium sulfate and concentrated under reduced pressure. The residue was subjected silica-gel column chromatography and eluted with a mixture of ethyl acetate:methanol (10:1). Fractions containing the objective compound were collected, dissolved in ethanol (10 ml) and crystallized by the addition of fumaric acid (216 mg). The resulting crystals were recrystallized from ethanol to give the title compound (420 mg).
melting point: 176-177°C
elemental analysis for $C_{29}H_{31}N_5O_6 \cdot H_2O$
Calculated (%): C,61.80 ; H,5.90 ; N,12.43
Found (%): C,61.72 ; H,5.65 ; N,12.03

**Reference Example B15**

Production of 7-tert-butyl-6-[2-[4-(diphenylmethoxy)-piperidino]ethoxy][1,2,4]triazolo[1,5-b]pyridazine

4-(Diphenylmethoxy)-1-piperidineethanol (740 mg) was dissolved in dried tetrahydrofuran (18 ml), followed by addition of sodium tert-butoxide (251 mg). The mixture was refluxed under heating for 25 minutes. After the mixture was cooled, 7-tert-butyl-6-chloro[1,2,4]triazolo[1,5-b]pyridazine (501 mg) was added thereto. The mixture was refluxed under heating for 2 hours. After the mixture was cooled, ice-water was added thereto, followed by extraction with ethyl acetate. The extract was washed with brine, dried over magnesium sulfate and concentrated under reduced pressure. The residue was subjected to silica-gel column chromatography and eluted with ethyl acetate. Fractions containing the objective compound were collected and recrystallized from ethyl acetate to give the title compound (380 mg).
melting point: 133-135°C
elemental analysis for $C_{29}H_{35}N_5O_2$
Calculated (%): C,71.73 ; H,7.26 ; N,14.42
Found (%): C,71.47 ; H,7.06 ; N,14.19

**Reference Example B16**

Production of 1-[4-(diphenylmethoxy)piperidino]-3-([1,2,4]triazolo[1,5-b]pyridazin-6-yloxy)-2-propanol

step A: Production of 6-(2-oxiranylmethoxy)[1,2,4]-triazolo[1,5-b]pyridazine

Glycidol (0.13 ml) and 6-chloro[1,2,4]triazolo[1,5-b]pyridazine (309 mg) were suspended in N,N-dimethylformamide (5 ml), followed by addition of 60% oily sodium hydride (80 mg) at room temperature. The mixture was stirred

for 3 hours, followed by addition of an aqueous sodium chloride solution and extraction with ethyl acetate. The extract was dried over magnesium sulfate and concentrated under reduced pressure. The residue was subjected to silica-gel column chromatography and eluted with ethyl acetate. Fractions containing the objective compound were collected and dried to give the title compound (170 mg).
$^1$H-NMR($CDCl_3$)δ ppm: 2.7-2.9(1H,m), 2.9-3.1(1H,m), 3.3-3.4(1H,m), 4.1-4.4(1H,m), 4.7-4.9(1H,m), 7.11(1H,d, J=9Hz), 8.02(1H,d,J=9Hz), 8.34(1H,s).

step B:

6-(2-Oxiranylmethoxy)[1,2,4]triazolo[1,5-b]pyridazine (171 mg) and 4-(diphenylmethoxy)piperidine (238 mg) were suspended in ethanol (8 ml). The suspension was stirred at 60°C for 5 hours and concentrated under reduced pressure. Ethyl acetate was added to the residue. The resulting crystals were collected, washed with ethyl ether and dried to give the title compound (327 mg).
melting point: 133-135°C
elemental analysis for $C_{26}H_{29}N_5O_3$
Calculated (%): C,67.96 ; H,6.36 ; N,15.24
Found (%): C,67.84 ; H,6.13 ; N,15.34

**Reference Example B17**

Production of 1-[4-(diphenylmethyl)-1-piperazinyl]-3-([1,2,4]triazolo[1,5-b]pyridazin-6-yloxy)-2-propanol dihydrochloride

6-(2-Oxiranylmethoxy)[1,2,4]triazolo[1,5-b]pyridazine (485 mg) and 1-(diphenylmethyl)piperazine (764 mg) were suspended in ethanol (30 ml). The mixture was stirred at 60°C for 15 hours and concentrated under reduced pressure. Ethyl acetate was added to the residue. The resulting crystals were collected, washed with ethyl ether and dissolved in ethyl acetate (20 ml). 4N HCl-ethyl acetate solution (5 ml) was added thereto, followed by concentration under reduced pressure. The resulting crystals were recrystallized from ethanol to give the title compound (392 mg).
melting point: 242°C (decomp.)
elemental analysis for $C_{25}H_{30}N_6O_2Cl_2 \cdot H_2O$
Calculated (%): C,56.08 ; H,6.02 ; N,15.69
Found (%): C,56.44 ; H,6.03 ; N,15.84

**Reference Example B18**

Production of 3-[4-(diphenylmethoxy)piperidino]-N-([1,2,4]triazolo[1,5-b]pyridazin-6-yl)propionamide

step A: 3-chloro-N-([1,2,4]triazolo[1,5-b]pyridazin-6-yl)propionamide

6-Amino[1,2,4]triazolo[1,5-b]pyridazine (0.80 g) was dissolved in N,N-dimethylacetamide (7 ml), followed by addition of 3-chloropropionylchloride (0.68 ml) under ice-cooling. The mixture was stirred at room temperature for 1 hour and poured into ice-water, followed by extraction with a mixture of ethyl acetate:tetrahydrofuran (1:1). The extract was washed with brine, dried over magnesium sulfate and concentrated under reduced pressure. Ethyl ether was added to the residue. The resulting crystals were collected by filtration and dried to give the title compound (0.875 g). $^1$H-NMR(DMSO-$d_6$)δ ppm: 2.99 (2H,t,J=7Hz) , 3.91(2H,t,J=7Hz) , 8.36, 8.43(each 1H,d,J=10Hz), 8.57(1H,s), 11.37 (1H,s).

step B:

3-Chloro-N-([1,2,4]triazolo[1,5-b]pyridazin-6-yl)propionamide (339 mg) and 4-(diphenylmethoxy)piperizine (401 mg) were dissolved in acetonitrile (15 ml), followed by addition of sodium iodide (447 mg) and potassium carbonate (249 mg). The mixture was stirred at room temperature for 15 hours, followed by addition of ice-water and extraction with ethyl acetate. The extract was washed with brine, dried over magnesium sulfate and concentrated under reduced pressure. The residue was subjected to a silica-gel column chromatography and eluted with a mixture of ethyl acetate: methanol (85:15). Fractions containing the objective compound were collected and concentrated. The resulting crystals were recrystallized from ethanol to give the title compound (495 mg).
melting point: 176-177°C
elemental analysis for $C_{26}H_{28}N_6O_2$

Calculated (%): C,68.40 ; H,6.18; N,18.41
Found (%): C,68.20 ; H,6.00 ; N,18.36

**Reference Example B19**

Production of 3-[4-(diphenylmethyl)-1-piperazinyl]-N-([1,2,4]triazolo[1,5-b]pyridazin-6-yl)propionamide

3-Chloro-N-([1,2,4]triazolo[1,5-b]pyridazin-6-yl)propionamide (339 mg) and 1-(diphenylmethyl)piperazine (379 mg) were dissolved in acetonitrile (15 ml), followed by addition of sodium iodide (447 mg) and potassium carbonate (249 mg). The mixture was stirred at room temperature for 15 hours and refluxed under heating for 8 hours. After the mixture was cooled, ice-water was added thereto, followed by extraction with ethyl acetate. The extract was washed with brine, dried over magnesium sulfate and concentrated under reduced pressure. The resulting crystals were recrystallized from ethanol to give the title compound (408 mg).
melting point: 176-177°C
elemental analysis for $C_{25}H_{27}N_7O$
Calculated (%): C,66.65 ; H,6.26 ; N,21.76
Found (%): C,66.36 ; H,6.16 ; N,21.95

**Reference Example B20**

Production of 6-[3-[4-(diphenylmethoxy)piperidino]-propylamino]-2-methyl[1,2,4]triazolo[1,5-b]pyridazine

6-Chloro-2-methyl[1,2,4]triazolo[1,5-b]pyridazine (655 mg) and 4-(diphenylmethoxy)-1-piperidinepropanamine (1.26 g) was suspended in 1-butanol (20 ml), followed by addition of N-ethyldiisopropylamine (1.94 ml). The mixture was refluxed under heating for 22 hours, followed by addition of ice-water and sodium hydrogen carbonate and extraction with ethyl acetate. The extract was washed with brine, dried over magnesium sulfate and concentrated under reduced pressure. The residue was subjected to silica-gel column chromatography and eluted with a mixture of ethyl acetate:methanol:triethylamine (50:5:1). Fractions containing the objective compound were collected and concentrated. The resulting crystals were washed with hexane and dried to give the title compound (547 mg). melting point: 119-120°C
elemental analysis for $C_{27}H_{32}N_6O$
Calculated (%): C,71.03 ; H,7.06 ; N,18.41
Found (%): C,70.91 ; H,6.95 ; N,18.18

**Reference Example B21**

Production of 6-[3-[4-(diphenylmethoxy)piperidino]propoxy]-2-methyl[1,2,4]triazolo[1,5-b]pyridazine

4-(Diphenylmethoxy)-1-piperidinepropanol (743 mg) was dissolved in dried tetrahydrofuran (17 ml), followed by addition of sodium tert-butoxide (241 mg). The mixture was heated to 60°C and stirred for 30 minutes. After the mixture was cooled, 6-chloro-2-methyl[1,2,4]triazolo[1,5-b]pyridazine (384 mg) was added thereto, followed by reflux under heating for 21 hours. After the mixture was cooled, ice-water was added thereto, followed by extraction with ethyl acetate. The extract was washed with brine, dried over magnesium sulfate and concentrated under reduced pressure. The residue was subjected to silica-gel column chromatography and eluted with a mixture of ethyl acetate: methanol:triethylamine (50:5:1). Fractions containing the objective compound were collected. The resulting crystals were washed with ethyl ether and dried to give the title compound (700 mg).
melting point: 134-136°C
elemental analysis for $C_{27}H_{31}N_5O_2$
Calculated (%): C,70.87 ; H,6.83 ; N,15.31
Found (%): C,70.67 ; H,6.94 ; N,15.34

**Reference Example B22**

Production of 6-[4-[4-(diphenylmethoxy)piperidino]-butoxy][1,2,4]triazolo[1,5-b]pyridazine fumarate

4-(Diphenylmethoxy)-1-piperidinebutanol (2.04 g) was dissolved in dried tetrahydrofuran (60 ml), followed by addition of 60% oily sodium hydride (480 mg). The mixture was refluxed under heating for 70 minutes. After the mixture was cooled, 6-chloro[1,2,4]triazolo[1,5-b]pyridazine (927 mg) and N,N-dimethylformamide (30 ml) were added thereto,

followed by reflux under heating for 18 hours. After the mixture was cooled, ice-water was added thereto, followed by extraction with ethyl acetate. The extract was washed with brine, dried over magnesium sulfate and concentrated under reduced pressure. The residue was subjected to silica-gel column chromatography and eluted with a mixture of ethyl acetate:methanol:triethylamine (50:5:1). The fractions containing the objective compound were collected. The obtained oily mixture was dissolved in ethanol, followed by addition of fumaric acid (80 mg). This mixture was concentrated under reduced pressure, and the residue was recrystallized from methanol to give the title compound (266 mg).
melting point: 159-161°C
elemental analysis for $C_{31}H_{35}N_5O_6$
Calculated (%): C,64.91 ; H,6.15 ; N,12.21
Found (%): C,64.72 ; H,6.10 ; N,12.06

**Reference Example B23**

Production of 2-[4-(diphenylmethoxy)piperidino]-N-([1,2,4]triazolo[1,5-b]pyridazin-6-yl)acetamide

step A: Production of 2-bromo-N-([1,2,4]triazolo[1,5-b]pyridazin-6-yl)acetamide

6-Amino[1,2,4]triazolo[1,5-b]pyridazine (1.32 g) was dissolved in N,N-dimethylacetamide (12 ml), followed by addition of bromoacetyl bromide (1.02 ml) under ice-cooling. The mixture was stirred at room temperature for 30 minutes and poured into ice-water. The resulting crystals were washed with water and ethyl acetate and dried to give the title compound (2.37 g).
melting point: 210°C (decomp.)
elemental analysis for $C_7H_6N_5OBr$
Calculated (%): C,32.83 ; H,2.36 ; N,27.35
Found (%): C,33.04 ; H,2.50 ; N,26.84

step B:

2-Bromo-N-([1,2,4]triazolo[1,5-b]pyridazin-6-yl)acetamide (605 mg) and 4-(diphenylmethoxy)piperidine (632 mg) were dissolved in acetonitrile (20 ml), followed by addition of potassium carbonate (391 mg). The mixture was stirred at room temperature for 3 hours, followed by addition of ice-water and extraction with ethyl acetate. The extract was washed with brine, dried over magnesium sulfate and concentrated under reduced pressure. The resulting crystals were collected by filtration, recrystallized from ethanol and dried to give the title compound (769 mg).
melting point: 158-160°C
elemental analysis for $C_{26}H_{26}N_6O_2$
Calculated (%): C,67.86 ; H,5.92 ; N,18.99
Found (%) : C,67.59 ; H,5.91 ; N,18.76

**Reference Example B24**

Production of 2-[4-(diphenylmethyl)-1-piperazinyl]-N-([1,2,4]triazolo[1,5-b]pyridazin-6-yl)acetamide

2-Bromo-N-([1,2,4]triazolo[1,5-b]pyridazin-6-yl)acetamide (636 mg) and 1-(diphenylmethyl)piperazine (627 mg) were dissolved in acetonitrile (20 ml), followed by addition of potassium carbonate (411 mg). The mixture was stirred at room temperature for 2 hours, followed by addition of ice-water and extraction with ethyl acetate. The extract was washed with aqueous sodium chloride saturated solution, dried over magnesium sulfate and concentrated under reduced pressure. The resulting crystals were collected by filtration and recrystallized from methanol to give the title compound (525 mg).
melting point: 203-204°C
elemental analysis for $C_{24}H_{25}N_7O$
Calculated (%): C,67.43 ; H,5.89 ; N,22.93
Found (%): C,67.22 ; H,5.87 ; N,22.97

**Reference Example B25**

Production of 6-[2-[4-(diphenylmethoxy)-piperidinocarbonyloxy]ethoxy][1,2,4]triazolo[1,5-b]pyridazine

step A: Production of 2-([1,2,4]triazolo[1,5-b]pyridazin-6-yloxy) ethanol

60% Oily sodium hydride (510 mg) was suspended in N,N-dimethylformamide (70 ml), followed by addition of 2-(tert-butyldiphenylsilyloxy)ethanol (3.83 g). The mixture was stirred at room temperature for 1 hour, followed by addition of 6-chloro[1,2,4]triazolo[1,5-b]pyridazine (1.98 g). The mixture was stirred at room temperature for 5 hours and poured into ice-water, followed by extraction with ethyl ether. The extract was washed with an aqueous sodium chloride solution, dried over magnesium sulfate and concentrated under reduced pressure. The residue was dissolved in tetrahydrofuran (40 ml), followed by addition of tetra-n-butylammonium fluoride trihydrate (2.02 g). The mixture was stirred at room temperature for 10 minutes and concentrated under reduced pressure. The residue was subjected to silica-gel column chromatography and eluted with a mixture of ethyl acetate:hexane (1:1). Fractions containing the objective compound were collected and concentrated to give the title compound (0.875 g).
$^1$H-NMR($CDCl_3$)δ ppm: 4.06(2H,t,J=5Hz), 4.5-4.7(2H,m), 7.10, 8.01(each 1H,d,J=10Hz), 8.34(1H,s).

step B:

2-([1,2,4]Triazolo[1,5-b]pyridazin-6-yloxy)ethanol (275 mg) was dissolved in tetrahydrofuran (12 ml), followed by addition of N,N'-carbonyldiimidazole (544 mg). The mixture was stirred at room temperature for 3 hours, followed by addition of 4-(diphenylmethoxy)piperidine (900 mg) and N-ethyldiisopropylamine (0.53 ml). The mixture was further stirred at room temperature for 13 hours and concentrated under reduced pressure. The residue was subjected to silica-gel column chromatography and eluted with ethyl acetate. Fractions containing the objective compound were collected and concentrated to give the title compound (490 mg).
melting point: 75-76°C
elemental analysis for $C_{26}H_{27}N_5O_4$
Calculated (%): C,65.95 ; H,5.75 ; N,14.79
Found (%): C,65.88 ; H,5.84 ; N,14.88

**Reference Example B26**

Production of 6-[2-[4-(diphenylmethyl)-1-piperazinylcarbonyloxy]ethoxy][1,2,4]triazolo[1,5-b]pyridazine

2-([1,2,4]Triazolo[1,5-b]pyridazin-6-yloxy)ethanol (450 mg) was dissolved in tetrahydrofuran (20 ml), followed by addition of N,N'-carbonyldiimidazole (649 mg). The mixture was stirred at room temperature for 3 hours, followed by addition of 1-(diphenylmethyl)piperazine (1.07 g) and N-ethyldiisopropylamine (0.73 ml). The mixture was stirred at 60°C for 17 hours and concentrated under reduced pressure. Ice-water was added to the residue, followed by extraction with ethyl acetate. The extract was washed with brine, dried over magnesium sulfate and concentrated under reduced pressure. The residue was subjected to silica-gel column chromatography and eluted with a mixture of ethyl acetate:hexane (1:1). Fractions containing the objective compound were collected and concentrated. The resulting crystals were recrystallized from ethyl acetate to give the title compound (464 mg).
melting point: 157-159°C
elemental analysis for $C_{25}H_{26}N_6O_3 \cdot 0.5H_2O$
Calculated (%): C,64.23 ; H,5.82 ; N,17.98
Found (%): C,64.32 ; H,5.50 ; N,17.56

**Reference Example B27**

Production of 6-[3-[4-(diphenylmethoxy)-piperidinocarbonyloxy]propoxy][1,2,4]triazolo[1,5-b]pyridazine

step A: Production of 1-[3-(tert-butyldiphenylsilyloxy)-propoxycarbonyl]-4-(diphenylmethoxy)piperidine

3-(tert-Butyldiphenylsilyloxy)propanol (2.12 g) was dissolved in tetrahydrofuran (20 ml), followed by addition of N,N'-carbonyldiimidazole (1.20 g). The mixture was stirred at room temperature for 20 minutes, followed by addition of 4-(diphenylmethoxy)piperidine (1.98 g) and N-ethyldiisopropylamine (1.28 ml). The mixture was stirred at room temperature for 23 hours and concentrated under reduced pressure. Ice-water was added to the residue, followed by extraction with ethyl acetate. The extract was washed with brine, dried over magnesium sulfate and concentrated under

reduced pressure. The residue was subjected to silica-gel column chromatography and eluted with a mixture of ethyl acetate:hexane (1:10). Fractions containing the objective compound were collected and concentrated to give the title compound (3.95 g).
$^1$H-NMR($CDCl_3$)δ ppm: 1.04(9H,s), 1.50-1.90(6H,m), 3.05-3.25(2H,m), 3.50-3.80(5H,m), 4.21(2H,t,J=7Hz), 5.51(1H, s), 7.2-7.8(20H,m).

step B : Production of 3-[4-(diphenylmethoxy)-piperidinocarbonyloxy]-1-propanol

1-[(3-tert-Butyldiphenylsilyloxy)propoxycarbonyl]-4-diphenylmethoxypiperidine (1.95 g) was dissolved in tetrahydrofuran (15 ml), followed by addition of tetra-n-butylammonium fluoride trihydride (2.02 g). The mixture was stirred at room temperature for 3 hours and concentrated under reduced pressure. The residue was subjected to silica-gel column chromatography and eluted with ethyl acetate. Fractions containing the objective compound were collected and concentrated to give the title compound (1.33 g).
$^1$H-NMR($CDCl_3$)δ ppm: 1.5-2.0(6H,m), 3.1-3.4(2H,m), 3.5-3.9(5H,m), 4.26(2H,t,J=6Hz), 5.52(1H,s), 7.1-7.5(10H,m).

step C:

3-[4-(Diphenylmethoxy)piperidinocarbonyloxy]-1-propanol (1.33 g) was dissolved in tetrahydrofuran (30 ml), followed by addition of sodium tert-butoxide (339 mg). The mixture was stirred at 60°C for 1.5 hours. After the mixture was cooled, 6-chloro[1,2,4]triazolo[1,5-b]pyridazine (496 mg) was added thereto. The mixture was refluxed under heating for two hours, followed by addition of ice-water and extraction with ethyl acetate. The extract was washed with an aqueous sodium chloride solution, dried over magnesium sulfate and concentrated under reduced pressure. The residue was subjected to silica-gel column chromatography and eluted with ethyl acetate. Fractions containing the objective compound were collected and concentrated to give the title compound (0.730 g).
melting point: 119-120°C
elemental analysis for $C_{27}H_{29}N_5O_4$
Calculated (%): C,66.51 ; H,6.00 ; N,14.36
Found (%): C,66.65 ; H,5.78 ; N,14.64

**Reference Example B28**

Production of 6-[3-[4-(diphenylmethyl)-1-piperazinylcarbonyloxy]propoxy][1,2,4]triazolo[1,5-b]pyridazine hydrochloride

step A: Production of 1-[3-(tert-butyldiphenylsilyloxy)-propoxycarbonyl]-4-(diphenylmethyl)piperazine

3-(tert-Butyldiphenylsilyloxy)propanol (1.71 g) was dissolved in tetrahydrofuran (16 ml), followed by addition of N,N'-carbonyldiimidazole (0.97 g). The mixture was stirred at room temperature for 20 minutes, followed by addition of 1-(diphenylmethyl)piperazine (1.51 g) and N-ethyldiisopropylamine (1.03 ml). The mixture was stirred at 60°C for 16 hours. After being cooled, the mixture was concentrated under reduced pressure. Ice-water was added to the residue, followed by extraction with ethyl acetate. The extract was washed with brine, dried over magnesium sulfate and concentrated under reduced pressure. The residue was subjected to silica-gel column chromatography and eluted with a mixture of ethyl acetate:hexane (1:10). Fractions containing the objective compound were collected and concentrated to give the title compound (2.53 g).
$^1$H-NMR($CDCl_3$)δ ppm: 1.03(9H,s), 1.7-2.0(2H,m), 2.2-3.6(8H,m), 3.71(2H,t,J=6Hz), 4.21(2H,t,J=6Hz), 4.21(1H,s), 7.1-7.7(20H,m).

step B : Production of 3-[4-(diphenylmethyl)-1-piperazinylcarbonyloxy]-1-propanol

1-[3-(tert-Butyldiphenylsilyloxy)propoxycarbonyl]-4-(diphenylmethyl)piperazine (2.50 g) was dissolved in tetrahydrofuran (12 ml), followed by addition of tetra-n-butylammonium fluoride trihydrate (1.46 g). The mixture was stirred at room temperature for 3 hours and concentrated under reduced pressure. The residue was subjected to silica-gel column chromatography and eluted with a mixture of ethyl acetate:hexane (1:1). Fractions containing the objective compound were collected and concentrated to give the title compound (1.51 g).
$^1$H-NMR($CDCl_3$)δ ppm: 1.7-2.0(6H,m), 2.2-3.6(8H,m), 3.64(2H,t,J=6Hz), 4.25(2H,t, J=6Hz), 4.24(1H,s), 7.1-7.5(10H, m).

step C:

3-[4-(Diphenylmethyl)-1-piperazinylcarbonyloxy-1-propanol (1.44 g) was dissolved in tetrahydrofuran (30 ml), followed by addition of sodium tert-butoxide (429 mg). The mixture was stirred at 60°C for 0.5 hour. After the mixture was cooled, 6-chloro[1,2,4]triazolo[1,5-b]pyridazine (627 mg) was added thereto. This mixture was refluxed under heating for three hours, followed by addition of ice-water and extraction with ethyl acetate. The extract was washed with an aqueous sodium chloride solution, dried over magnesium sulfate and concentrated under reduced pressure. The residue was subjected to silica-gel column chromatography and eluted with a mixture of ethyl acetate:hexane (3:1). Fractions containing the objective compound were collected and concentrated. The residue was dissolved in ethyl acetate (10 ml), followed by addition of an ethyl acetate solution of 4N HCl (0.32 ml). The mixture was concentrated under reduced pressure. The resulting crystals were recrystallized from ethanol to give the title compound (0.450 g).
melting point: 167-169°C
elemental analysis for $C_{26}H_{29}N_6O_3Cl\cdot 0.5H_2O$
Calculated (%): C,60.29 ; H,5.84 ; N,16.22
Found (%): C,60.52 ; H,5.96 ; N,16.05

**Reference Example B29**

Production of 6-[6-[4-(diphenylmethoxy)piperidino]-hexyloxy][1,2,4]triazolo[1,5-b]pyridazine fumarate

4-(Diphenylmethoxy)-1-piperidinehexanol (0.905 g) was dissolved in tetrahydrofuran (15 ml), followed by addition of 60% oily sodium hydride (118 mg). The mixture was refluxed under heating for 1 hour. After the mixture was cooled, 6-chloro[1,2,4]triazolo[1,5-b]pyridazine (381 mg) was added thereto. This mixture was refluxed under heating for 3 hours, followed by addition of ice-water and extraction with ethyl acetate. The extract was washed with an aqueous sodium chloride solution, dried over magnesium sulfate and concentrated under reduced pressure. The residue was subjected to silica-gel column chromatography and eluted with a mixture of ethyl acetate:methanol:triethylamine (50:5:1). Fractions containing the objective compound were collected and concentrated. The residue was dissolved in ethyl acetate (10 ml), followed by addition of a solution of fumaric acid (263 mg) in methanol (10 ml). The mixture was concentrated, and the residue was recrystallized from ethyl acetate to give the title compound (0.979 g).
melting point: 136-138°C
elemental analysis for $C_{33}H_{39}N_5O_6$
Calculated (%): C,65.87 ; H,6.53 ; N,11.64
Found (%): C,65.79 ; H,6.54 ; N,11.62

**Reference Example B30**

Production of 6-[6-[4-(diphenylmethyl)-1-piperazinyl]-hexyloxy][1,2,4]triazolo[1,5-b]pyridazine fumarate

4-(Diphenylmethyl)-1-piperazinehexanol (0.640 g) was dissolved in tetrahydrofuran (10 ml), followed by addition of 60% oily sodium hydride (145 mg). The mixture was refluxed under heating for 1 hour. After the mixture was cooled, 6-chloro[1,2,4]triazolo[1,5-b]pyridazine (281 mg) was added thereto. This mixture was refluxed under heating for 1.5 hours. Ice-water was added to the mixture, followed by extraction with ethyl acetate. The extract was washed with an aqueous sodium chloride solution, dried over magnesium sulfate and concentrated under reduced pressure. The residue was subjected to silica-gel column chromatography and eluted with ethyl acetate. Fractions containing the objective compound were collected and dissolved in ethyl acetate (10 ml), followed by addition of a solution of fumaric acid (140 mg) in methanol (10 ml). The mixture was concentrated, and the residue was recrystallized from ethanol to give the title compound (189 mg).
melting point: 149-151°C
elemental analysis for $C_{32}H_{38}N_6O_5\cdot 0.5H_2O$
Calculated (%) : C,64.52 ; H,6.60 ; N,14.11
Found (%) : C,64.95 ; H,6.64 ; N,13.91

**Reference Example B31**

Production of 6-[3-[4-(diphenylmethoxy)piperidino]propoxy]-2-phenyl[1,2,4]triazolo[1,5-b]pyridazine hydrochloride

4-(Diphenylmethoxy)-1-piperidinepropanol (487 mg) was dissolved in dried tetrahydrofuran (10 ml), followed by addition of sodium tert-butoxide (144 mg). The mixture was refluxed under heating for 40 minutes. After the mixture

was cooled, 6-chloro-2-phenyl[1,2,4]triazolo[1,5-b]pyridazine (315 mg) was added thereto. This mixture was refluxed under heating for 4 hours. After the mixture was cooled, ice-water was added thereto, followed by extraction with a mixture of ethyl acetate:tetrahydrofuran (2:1). The extract was washed with brine dried over magnesium sulfate and concentrated under reduced pressure. The residue was subjected to silica-gel column chromatography and eluted with a mixture of ethyl acetate and methanol (10:1). Fractions containing the objective compound were collected, concentrated and dissolved in ethyl acetate (10 ml), followed by addition of 4N HCl in ethyl acetate solution (0.25 ml) and concentrated under reduced pressure. The resulting crystals were recrystallized from ethanol to give the title compound (0.334 g).
melting point: 127-129°C
elemental analysis for $C_{32}H_{34}N_5O_2Cl{\cdot}H_2O$
Calculated (%): C,66.95 ; H,6.32 ; N,12.20
Found (%): C,67.01 ; H,6.46 ; N,12.27

**Reference Example B32**

Production of 6-[3-[4-(diphenylmethoxy)piperidino]-propylamino]-2-phenyl[1,2,4]triazolo[1,5-b]pyridazine

6-Chloro-2-phenyl[1,2,4]triazolo[1,5-b]pyridazine (365 mg) and 4-(diphenylmethoxy)-1-piperidinepropanamine (0.513 g) were suspended in 1-butanol (8 ml); N-ethyldiisopropylamine (0.54 ml) was added, followed by heating and refluxing for 19 hours. Ice water and sodium hydrogen carbonate were added thereto, followed by extraction with ethyl acetate; the extract was washed with brine and dried over magnesium sulfate. After the extract was concentrated under reduced pressure, the residue was subjected to silica gel column chromatography and eluted with ethyl acetate: methanol:triethylamine (50:5:1). The desired fractions were collected and concentrated; the resulting crystals were recrystallized from ethyl acetate to give the title compound (308 mg).
melting point: 170-172°C
elemental analysis for $C_{32}H_{34}N_6O{\cdot}0.5H_2O$
Calculated (%): C,72.84 ; H,7.69 ; N,15.93
Found (%): C,73.08 ; H,7.61 ; N,16.03

**Reference Example B33**

Production of 2-tert-butyl-6- [3-[4-(diphenylmethoxy)-piperidino]propoxy][1,2,4]triazolo[1,5-b]pyridazine fumarate

4-(Diphenylmethoxy)-1-piperidinepropanol (911 mg) was dissolved in dry tetrahydrofuran (20 ml); sodium tert-butoxide (296 mg) was added thereto, followed by heating and refluxing for 30 minutes. After cooling, 2-tert-butyl-6-chloro[1,2,4]triazolo[1,5-b]pyridazine (589 mg) was added thereto, followed by heating and refluxing for 6 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the residue was dissolved in 10 ml of ethyl acetate; a solution of fumaric acid (102 mg) in methanol (10 ml) was added thereto, followed by concentration. The residue was recrystallized from ethyl acetate to give the title compound (382 mg).
melting point: 170-172°C
elemental analysis for $C_{34}H_{41}N_5O_6$
Calculated (%): C,66.32 ; H,6.71 ; N,11.37
Found (%): C,66.15 ; H,6.74 ; N,11.28

**Reference Example B34**

Production of 2-tert-butyl-6-[3-[4-(diphenylmethoxy)-piperidino]propylamino][1,2,4]triazolo[1,5-b]pyridazine fumarate

2-tert-Butyl-6-chloro[1,2,4]triazolo[1,5-b]pyridazine (276 mg) and 4-(diphenylmethoxy)-1-piperidinepropanamine (0.425 g) were suspended in 1-butanol (8 ml); N-ethyidiisopropylamine (0.45 ml) was added thereto, followed by heating and refluxing for 40 hours. Ice water and sodium hydrogen carbonate were added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the residue was dissolved in ethyl acetate (10 ml); a solution of fumaric acid (40 mg) in methanol (5 ml) was added thereto, followed

by concentration. The residue was powdered by the addition of ethyl ether, filtered and collected to give the title compound (164 mg).
melting point: 80°C
elemental analysis for $C_{34}H_{42}N_6O_5 \cdot H_2O, 0.5Et_2O$
Calculated (%): C,64.55 ; H,7.37 ; N,12.55
Found (%): C,64.79 ; H,7.76 ; N,12.44

**Reference Example B35**

Production of 6-[6-[4-(diphenylmethoxy)piperidino]-hexylamino][1,2,4]triazolo[1,5-b]pyridazine

step A: Production of 6-[([1,2,4]triazolo[1,5-b]pyridazin-6-yl)amino]-1-hexanol

6-Chloro[1,2,4]triazolo[1,5-b]pyridazine (2.03 g) was dissolved in ethanol (20 ml); 6-amino-1-hexanol (3.85 g) was added thereto, followed by heating and refluxing for 19 hours. After cooling, the crystals obtained were collected by filtration, washed with ethanol and dried to give the title compound (3.64 g).
$^1H$-NMR($CDCl_3$)$\delta$ ppm: 1.3-1.8(8H,m), 3.46(2H,t,J=6Hz), 3.67(2H,q, J=6Hz), 4.58(1H,broad s), 6.71,7.78(each 1H,d, J=10Hz), 8.19(1H,s).

step B:

6-[([1,2,4]Triazolo[1,5-b]pyridazin-6-yl)amino]-1-hexanol (1.64 g) was suspended in tetrahydrofuran (40 ml); N-ethyldiisopropylamine (2.25 g) and methanesulfonyl chloride (2.0 g) were added thereto, followed by stirring at room temperature for 5.5 hours. Ice water and sodium chloride were added, followed by extraction with ethyl acetate:tetrahydrofuran (2:1); the extract was washed with brine and dried over magnesium sulfate. After the extract was concentrated under reduced pressure, the residue was dissolved in acetonitrile (14 ml); 4-(diphenylmethoxy)piperidine (743 mg), potassium iodide (457 mg) and potassium carbonate (380 mg) were added thereto, followed by stirring at 50°C for 16 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the crystals obtained were recrystallized from acetic ether:ethyl ether (1:1) to give the title compound (597 mg).
melting point: 97-98°C
elemental analysis for $C_{29}H_{36}N_6O$
Calculated (%): C,71.87 ; H,7.49 ; N,17.34
Found (%): C,71.77 ; H,7.37 ; N,17.36

**Reference Example B36**

Production of methyl 6-[3-[4-(diphenylmethoxy)piperidino]-propylamino][1,2,4]triazolo[1,5-b]pyridazine-2-carboxylate

Methyl 6-chloro[1,2,4]triazolo[1,5-b]pyridazine-2-carboxylate (0.92 g) and 4-(diphenylmethoxy)-1-piperidinepropanamine (1.40 g) were suspended in N,N-dimethylformamide (20 ml); N-ethyldiisopropylamine (1.49 ml) was added thereto, followed by heating and refluxing at 80°C for 15 hours. After cooling, ice water and sodium chloride were added thereto, followed by extraction with ethyl acetate:tetrahydrofuran (1:2); the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the residue was recrystallized from ethanol:ethyl acetate (1:2) to give the title compound (639 mg).
melting point: 93-96°C
elemental analysis for $C_{28}H_{32}N_6O_3 \cdot 0.5H_2O$
Calculated (%) :C,65.99 ; H,6.53 ; N,16.49
Found (%) :C,65.69 ; H,6.28 ; N,16.58

**Reference Example B37**

Production of 6-[6-[4-(diphenylmethyl)-1-piperazinyl]-hexylamino][1,2,4]triazolo[1,5-b]pyridazine

6-(6-Hydroxyhexylamino)[1,2,4]triazolo[1,5-b]pyridazine (1.64 g) was suspended in tetrahydrofuran (40 ml); N-ethyldiisopropylamine (2.25 g) and methanesulfonyl chloride (2.0 g) were added thereto, followed by stirring at room temperature for 1 hour. Ice water and sodium chloride were added thereto, followed by extraction with ethyl acetate; the extract was washed with brine and dried over magnesium sulfate. After the extract was concentrated under reduced pressure, the residue was dissolved in N,N-dimethylformamide (13 ml); 1-(diphenylmethyl)piperazine (694 mg), potassium iodide (456 mg) and potassium carbonate (379 mg) were added thereto, followed by stirring at room temperature for 2 hours and at 60°C for 4 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the crystals obtained were recrystallized from ethyl acetate and dried to give the title compound (702 mg).
melting point: 130-132°C
elemental analysis for $C_{28}H_{25}N_7$
Calculated (%): C,71.61 ; H,7.51 ; N,20.88
Found (%):C,71.39 ; H,7.39 ; N,21.04

**Reference Example B38**

Production of 6-[3-[4-(diphenylmethoxy)piperidino]propoxy]-imidazo[1,2-b]pyridazine fumarate

Sodium tert-butoxide (159 mg) was dissolved in N,N-dimethylformamide (15 ml); 4-(diphenylmethoxy)-1-piperidinepropanol (489 mg) was added thereto, followed by stirring at 60°C for 30 minutes. After cooling, 6-chloroimidazo [1,2-b]pyridazine (253 mg) was added thereto, followed by stirring at 80-90°C for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (90:10:1). The desired fractions were collected, dissolved in ethyl acetate (10 ml); a solution of fumaric acid (93 mg) in methanol (10 ml) was added thereto, followed by concentration. The crystals precipitated were collected by filtration, washed with ethyl acetate and dried to give the title compound (288 mg). melting point: 155-157°C
elemental analysis for $C_{31}H_{34}N_4O_6 \cdot H_2O$
Calculated (%): C,64.57 ; H,6.29 ; N,9.72
Found (%) : C,64.24 ; H,5.98 ; N,9.28

**Reference Example B39**

Production of 6-[3-[4-(diphenylmethoxy)piperidino]-propylamino]imidazo[1,2-b]pyridazine fumarate [2:3]

4-(Diphenylmethoxy)-1-piperidinepropanamine (325 mg) and 6-chloroimidazo[1,2-b]pyridazine (184 mg) were stirred at 180°C for 1 hour. After cooling, aqueous sodium hydrogen carbonate solution was added, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (90:10:1). The desired fractions were collected, dissolved in ethyl acetate (10 ml); a solution of fumaric acid (193 mg) in methanol (10 ml) was added thereto, followed by concentration. Acetone was added to the residue; the crystals precipitated were collected by filtration, washed with acetone and dried to give the title compound (246 mg).
melting point: 137-139°C
elemental analysis for $C_{33}H_{37}N_5O_7 \cdot 0.5H_2O$
Calculated (%): C,63.45 ; H,6.13 ; N,11.21
Found (%): C,63.66 ; H,6.00 ; N,11.12

**Reference Example B40**

Production of ethyl 2-[6-[3-[4-(diphenylmethoxy)piperidino]-propylamino]imidazo[1,2-b]pyridazin-2-yl]-2-methylpropionate difumarate

4-(Diphenylmethoxy)-1-piperidinepropanamine (4.2 g) and ethyl 2-[6-chloroimidazo[1,2-b]pyridazin-2-yl]-2-methylpropionate (1.76 g) were stirred at 190-200°C for 3.5 hours. After cooling, aqueous sodium hydrogen carbonate solution was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (100:5:1). The desired fractions were collected, dissolved in ethyl acetate (16 ml); a solution of fumaric acid (867 mg) in methanol (16 ml) was added thereto, followed by concentration. Acetone was added to the residue; the crystals precipitated were collected by filtration, washed with acetone and dried to give the title compound (2.30 g).
melting point: 126-128°C
elemental analysis for $C_{41}H_{49}N_5O_{11}$
Calculated (%): C,62.50 ; H,6.27 ; N,8.89
Found (%): C,62.28 ; H,6.15 ; N,8.97

**Reference Example B41**

Production of 6-[3-[4-(diphenylmethoxy)piperidino]propoxy]-2-methoxyimidazo[1,2-b]pyridazine

4-(Diphenylmethoxy)-1-piperidinepropanol (758 mg) was dissolved in N,N-dimethylformamide (40 ml); 60% oily sodium hydride (102 mg) was added, followed by stirring at 60°C for 40 minutes. After cooling, 6-chloro-2-methoxyimidazo[1,2-b]pyridazine (428 mg) was added thereto, followed by stirring at 100°C for 2.5 hours. After cooling, ice water and sodium chloride were added thereto, followed by extraction with ethyl acetate:tetrahydrofuran (1:2); the extract was washed with brine and dried over magnesium sulfate. After the extract was concentrated under reduced pressure, the residue was subjected to silica gel column chromatography and eluted with ethyl acetate:methanol:triethylamine (50:5:1). The desired fractions were collected and concentrated; the crystals precipitated were recrystallized from ethanol to give the title compound (499 mg).
melting point: 133-135°C
elemental analysis for $C_{28}H_{32}N_4O_3$
Calculated (%): C,71.16 ; H,6.83 ; N,11.86
Found (%): C,71.23 ; H,6.83 ; N,11.94

**Reference Example B42**

Production of 6-[3-[4-(diphenylmethyl)-1-piperazinyl]-propoxy]-2-methoxyimidazo[1,2-b]pyridazine

4- (Diphenylmethyl)-1-piperazinepropanol (251 mg) was dissolved in N,N-dimethylformamide (14 ml); 60% oily sodium hydride (36 mg) was added thereto, followed by stirring at 60°C for 30 minutes. After cooling, 6-chloro-2-methoxyimidazo[1,2-b]pyridazine (149 mg) was added thereto, followed by stirring at 90°C for 4.5 hours. After cooling, ice water and sodium chloride were added thereto, followed by extraction with ethyl acetate:tetrahydrofuran (1:2); the extract was washed with brine and dried over magnesium sulfate. After the extract was concentrated under reduced pressure, the residue was subjected to silica gel column chromatography and eluted with ethyl acetate. The desired fractions were collected; the crystals precipitated were recrystallized from ethyl acetate to give the title compound (99 mg).
melting point: 144-146°C
elemental analysis for $C_{27}H_{31}N_5O_2$
Calculated (%): C,70.87 ; H,6.83 ; N,15.31
Found (%): C,70.79 ; H,6.82 ; N,15.39

**Reference Example B43**

Production of 2-[6-[3-[4-(diphenylmethoxy)piperidino]-propylamino]imidazo[1,2-b]pyridazin-2-yl]-2-methylpropionic acid

Ethyl 2- [6-[3- [4- (diphenylmethoxy)-piperidino]propylamino]imidazo[1,2-b]pyridazin-2-yl]-2-methylpropionate (468 mg) was dissolved in ethanol (3 ml); 1 N aqueous solution of sodium hydroxide (2 ml) was added thereto, followed by stirring at room temperature for 15 hours. After the mixture was concentrated under reduced pressure, the residue was diluted with water and washed with ethyl acetate; the water layer was adjusted to pH 7 by the addition of 1 N hydrochloric acid, followed by extraction with ethyl acetate:tetrahydrofuran (1:1); the extract was washed with brine and dried over magnesium sulfate. After the extract was concentrated under reduced pressure, ethyl acetate was added to the residue; the crystals precipitated were collected by filtration, washed with ethyl acetate and dried to give the title compound (267 mg). It can be recrystallized from acetone.
melting point: 205-206°C
elemental analysis for $C_{31}H_{37}N_5O_3$
Calculated (%): C,70.56 ; H,7.07 ; N,13.27
Found (%): C,70.46 ; H,7.06 ; N,13.36

**Reference Example B44**

Production of tert-butyl 2-[6-[3-[4-(diphenylmethoxy)-piperidino]propoxy]imidazo[1,2-b]pyridazin-2-yl]-2-methylpropionate difumarate

60% Oily sodium hydride (70 mg) was dissolved in N,N-dimethylformamide (5 ml); 4-(diphenylmethoxy)-1-piperidinepropanol (570 mg) was added thereto, followed by stirring at room temperature under reduced pressure for 30 minutes. After tert-butyl 2-(6-chloroimidazo[1,2-b]pyridazin-2-yl)-2-methylpropionate (520 mg) was added thereto, followed by stirring at room temperature for 8 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (195:5:1). The desired fractions were collected and dissolved in ethyl acetate (5 ml); a solution of fumaric acid (233 mg) in methanol (10 ml) was added thereto, followed by concentration. The crystals precipitated were collected by filtration, washed with acetone and dried to give the title compound (631 mg). melting point: 162-164°C
elemental analysis for $C_{43}H_{52}N_4O_{12}$
Calculated (%): C,63.22 ; H,6.42 ; N,6.86
Found (%): C,62.91 ; H,6.36 ; N,6.90

**Reference Example B45**

Production of 2-[6-[3-[4-(diphenylmethoxy)piperidino]-propoxy]imidazo[1,2-b]pyridazin-2-yl]-2-methylpropionic acid

tert-Butyl 2-[6-[3-[4-(diphenylmethoxy)piperidino]-propoxy]imidazo[1,2-b]pyridazin-2-yl]-2-methylpropionate (818 mg) was dissolved in 1-butanol (8 ml); potassium hydroxide (393 mg) was added thereto, followed by stirring at 90°C for 14 hours. After cooling, 1 N hydrochloric acid (7 ml) was added to the water layer, followed by extraction with ethyl acetate; the extract was washed with brine and dried over magnesium sulfate. After the extract was concentrated under reduced pressure, ethyl acetate was added to the residue; the crystals precipitated were collected by filtration, washed with ethyl acetate and dried to give the title compound (465 mg).
melting point: 183-185°C
elemental analysis for $C_{31}H_{36}N_4O_4 \cdot 2.5H_2O$
Calculated (%): C,64.90 ; H,7.20 ; N,9.77
Found (%): C,65.15 ; H,6.73 ; N,9.52

**Reference Example B46**

Production of ethyl 2-[6-[3-[4-(diphenylmethoxy)piperidino]-propoxy]imidazo[1,2-b]pyridazin-2-yl]-2-methylpropionate difumarate

2-[6-[3-[4-(Diphenylmethoxy)piperidino]-propoxy]imidazo[1,2-b]pyridazin-2-yl]-2-methylpropionic acid (529 mg) was dissolved in N,N-dimethylformamide (3 ml); N-ethyldiisopropylamine (0.207 ml) and ethyl iodide (0.135 ml) were added thereto, followed by stirring at room temperature for 15 hours. Ice water was added to the reaction mixture, followed by extraction with ethyl acetate; the extract was washed with brine and dried over magnesium sulfate. After the extract was concentrated under reduced pressure, the residue was subjected to silica gel column chromatography and eluted with ethyl acetate:methanol:triethylamine (100:5:1). The desired fractions were collected and dissolved in ethyl acetate (3 ml); a solution of fumaric acid (153 mg) in methanol (3 ml) was added thereto, followed by concentration. The crystals precipitated were collected by filtration, washed with ethyl acetate and dried to give the title compound

(406 mg).
melting point: 116-122°C
elemental analysis for $C_{41}H_{48}N_4O_{12}\cdot 0.5H_2O$
Calculated (%): C,61.72 ; H,6.19 ; N,7.02
Found (%): C,61.61 ; H,6.11 ; N,6.85

**Reference Example B47**

Production of 6-[2-[2-[4-(diphenylmethyl)-1-piperazinyl]-ethoxy]ethoxy]-7-methyl[1,2,4]triazolo[1,5-b]pyridazine

60% Oily sodium hydride (260 mg) was suspended in tetrahydrofuran (20 ml); 2-[2-[4-(diphenylmethyl)-1-piperazinyl]ethoxy]ethanol (1.15 g) was added thereto, followed by heating and refluxing for 1 hour. After cooling, 6-chloro-7-methyl[1,2,4]triazolo[1,5-b]pyridazine (540 mg) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (10:10:1). The desired fractions were collected; the crystals precipitated were collected by filtration, washed with ethyl ether and dried to give the title compound (730 mg).
melting point: 71-72°C
elemental analysis for $C_{27}H_{32}N_6O_2$
Calculated (%): C,68.62 ; H,6.82 ; N,17.78
Found (%): C,68.35 ; H,6.71 ; N,17.79

**Reference Example B48**

Production of 6-[2-[2-[4-(diphenylmethyl)-1-piperazinyl]-ethoxy]ethoxy][1,2,4]triazolo[1,5-b]pyridazine dihydrochloride

60% Oily sodium hydride (100 mg) was suspended in tetrahydrofuran (20 ml); 2-[2-[4-(diphenylmethyl)-1-piperazinyl]ethoxy]ethanol (470 mg) was added thereto, followed by heating and refluxing for 1 hour. After cooling, 6-chloro [1,2,4]triazolo[1,5-b]pyridazine (200 mg) was added thereto, followed by heating and refluxing for 4.5 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (10:10:1). The desired fractions were collected and dissolved in ethyl acetate (5 ml); 4 N hydrogen chloride solution in ethyl acetate (0.83 ml) was added thereto; the crystals precipitated were collected by filtration, washed with ethyl ether and dried to give the title compound (0.54 g).
melting point: 182-184°C
elemental analysis for $C_{26}H_{32}N_6O_2Cl_2\cdot H_2O$
Calculated (%): C,56.83 ; H,6.24 ; N,15.29
Found (%): C,56.98 ; H,6.10 ; N,15.39

**Reference Example B49**

Production of 6-[4-[4-(diphenylmethyl)-1-piperazinyl]butoxy]-7-methyl[1,2,4]triazolo[1,5-b]pyridazine dihydrochloride

60% Oily sodium hydride (240 mg) was suspended in tetrahydrofuran (20 ml); 4-(diphenylmethyl)-1-piperazinebutanol (0.99 g) was added thereto, followed by heating and refluxing for 1 hour. After cooling, 6-chloro-7-methyl [1,2,4]triazolo[1,5-b]pyridazine (510 mg) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (20:20:1). The desired fractions were collected and dissolved in ethyl acetate (5 ml); 4 N hydrogen chloride solution in ethyl acetate (0.64 ml) was added thereto; the crystals precipitated were collected by filtration, washed with ethyl ether and dried to give the title compound (470 mg).
melting point: 190-192°C
elemental analysis for $C_{27}H_{34}N_6OCl_2\cdot 0.5AcOEt\cdot H_2O$
Calculated (%): C,58.88 ; H,6.82 ; N,14.21
Found (%): C,59.11 ; H,6.82 ; N,14.03

**Reference Example B50**

Production of 6-[2-[2-[4-(diphenylmethyl)-1-piperazinyl]-ethoxy]ethylthio][1,2,4]triazolo[1,5-b]pyridazine dihydrochloride

step A : Production of 6-[2-(2-bromoethoxy)ethylthio]-[1,2,4]triazolo[1,5-b]pyridazine

Methyl 3-mercaptopropionate (2.8 ml) was dissolved in methanol (10 ml); 2 N sodium methoxide solution in methanol (19.4 ml) and 6-chloro[(1,2,4)triazolo[1,5-b]pyridazine (1.0 g) were added thereto, followed by heating and refluxing for 1 hour. After cooling, the mixture was concentrated under reduced pressure; ethyl acetate was added to the residue; the crystals precipitated were collected, washed with ethyl acetate and suspended in tetrahydrofuran (20 ml); 2-bromoethyl ether (1.63 ml) was added thereto, followed by heating and refluxing for 2 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (20:20:1). The desired fractions were collected to give the title compound (0.60 g) as an oil. $^1$H-NMR($CDCl_3$)$\delta$ ppm: 3.49(2H,t,J=6Hz), 3.55(2H,t,J=6Hz), 3.86(2H,t,J=6Hz), 3.90(2H,t,J=6Hz), 7.22, 7.93(各 1H,d,J=9Hz), 8.37(1H,s).

step B:

6-[2-(2-Bromoethoxy)ethylthio][1,2,4]triazolo[1,5-b]pyridazine (890 mg) and 1-(diphenylmethyl)piperazine (740 mg) were dissolved in N,N-dimethylformamide (10 ml); potassium carbonate (490 mg) was added thereto, followed by stirring at room temperature for 24 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:methanol (10:1). The desired fractions were collected and concentrated; the residue was dissolved in ethyl acetate (5 ml); 4 N hydrogen chloride solution in ethyl acetate (1.64 ml) was added thereto; the precipitated crystals were collected by filtration, washed with ethyl ether and dried to give the title compound (1.13 g).
melting point: 188-189°C
elemental analysis for $C_{26}H_{32}N_6OSCl_2 \cdot H_2O$
Calculated (%): C,55.22 ; H,6.06 ; N,14.86
Found (%): C,55.49 ; H,6.02 ; N,15.08

**Reference Example B51**

Production of 6-[6-[4-(diphenylmethyl)-1-piperazinyl]hexyloxy]-7-methyl[1,2,4]triazolo[1,5-b]pyridazine dihydrochloride

60% Oily sodium hydride (210 mg) was suspended in tetrahydrofuran (15 ml); 4-(diphenylmethyl)-1-piperazinehexanol (0.91 g) was added thereto, followed by heating and refluxing for 1 hour. After cooling, 6-chloro-7-methyl [1,2,4]triazolo[1,5-b]pyridazine (440 mg) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (10:10:1). The desired fractions were collected and dissolved in ethyl acetate (5 ml); 4 N hydrogen chloride solution in ethyl acetate (1.44 ml) was added thereto; the crystals precipitated were collected by filtration, washed with ethyl ether and dried to give the title compound (1.06 g), which was then recrystallized from ethanol.
melting point: 170-172°C
elemental analysis for $C_{29}H_{38}N_6OCl_2 \cdot 0.5EtOH$
Calculated (%): C,62.06 ; H,7.11 ; N,14.47
Found (%) : C,61.77 ; H,6.94 ; N,14.33

**Reference Example B52**

Production of 6-[6-[4-(diphenylmethoxy)piperidino]hexyloxy]-7-methyl[1,2,4]triazolo[1,5-b]pyridazine hydrochloride

60% Oily sodium hydride (160 mg) was suspended in tetrahydrofuran (20 ml); 4-(diphenylmethoxy)-1-piperidinehexanol (1.24 g) was added thereto, followed by heating and refluxing for 1 hour. After cooling, 6-chloro-7-methyl[1,2,4]triazolo[1,5-b]pyridazine (570 mg) was added thereto, followed by heating and refluxing for 1 hour. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate-methanol (10:1). The desired fractions were collected and dissolved in ethyl acetate (5 ml); 4 N hydrogen chloride solution in ethyl acetate (0.54 ml) was added thereto; the crystals precipitated were collected by filtration, washed with ethyl ether and dried to give the title compound (0.70 g).
melting point: 208-209°C
elemental analysis for $C_{30}H_{38}N_5O_2Cl \cdot 0.8H_2O$
Calculated (%): C,65.45 ; H,7.25 ; N,12.72
Found (%): C,65.47 ; H,7.21 ; N,12.60

**Reference Example B53**

Production of 6-[2-[2-[4-(diphenylmethoxy)piperidino]-ethoxy]ethoxy]-7-methyl[1,2,4]triazolo[1,5-b]pyridazine

60% Oily sodium hydride (190 mg) was suspended in tetrahydrofuran (15 ml); 2-[2-[4-(diphenylmethoxy)-piperidino]ethoxy]ethanol (1.47 g) was added thereto, followed by heating and refluxing for 1 hour. After cooling, 6-chloro-7-methyl[1,2,4]triazolo[1,5-b]pyridazine (660 mg) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10:1). The desired fractions were collected; the crystals precipitated were collected by filtration, washed with ethyl ether and dried to give the title compound (1.23 g).
melting point: 80-82°C
elemental analysis for $C_{28}H_{33}N_5O_3$
Calculated (%): C,68.97 ; H,6.82 ; N,14.36
Found (%): C,68.75 ; H,6.70 ; N,14.57

**Reference Example B54**

Production of 6-[6-[4-(diphenylmethyl)-1-piperazinyl]-hexylthio]-7-methyl[1,2,4]triazolo[1,5-b]pyridazine dihydrochloride

step A: Production of 6-(6-bromohexylthio)-7-methyl[1,2,4]triazolo[1,5-b]pyridazine

Methyl 3-mercaptopropionate (5.57 g) was dissolved in methanol (20 ml); 2 N sodium methoxide solution in methanol (35.6 ml) and 6-chloro-7-methyl[1,2,4]triazolo[1,5-b]pyridazine (2.0 g) were added thereto, followed by heating and refluxing for 1 hour. After cooling, the mixture was concentrated under reduced pressure; ethyl acetate was added to the residue; the crystals precipitated were collected, washed with ethyl acetate and suspended in tetrahydrofuran (30 ml); 1,6-dibromohexane (3.65 ml) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, ethyl ether was added to the residue; the crystals precipitated were collected by filtration to give the title compound (2.42 g).
$^1$H-NMR($CDCl_3$)δ ppm: 1.49-1.54(4H,m), 1.75-1.95(4H,m), 2.40(3H,s), 3.31(2H,t,J=7Hz), 3.43(2H,t,J=7Hz), 7.72(1H, s), 8.30(1H,s).

step B:

6-(6-Bromohexylthio)-7-methyl[1,2,4]triazolo[1,5-b]pyridazine (1.0 g) and 1-(diphenylmethyl)piperazine (770 mg) were dissolved in N,N-dimethylformamide (10 ml); potassium carbonate (500 mg) was added thereto, followed by stirring at room temperature for 18 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (20:1). The desired fractions were collected and concentrated; the residue was dissolved in ethyl acetate (5 ml); 4 N hydrogen chloride solution in ethyl acetate (1.96 ml) was added thereto; the crystals precipitated were collected by filtration, washed with ethyl ether and dried to give the title compound (0.98 g).
melting point: 180-182°C
elemental analysis for $C_{29}H_{38}N_6SCl_2 \cdot 0.4H_2O$

Calculated (%): C,59.97 ; H.6.73 ; N,14.47
Found (%): C,60.17 ; H,6.55 ; N,14.62

**Reference Example B55**

Production of 6-[2-[2-[4-(diphenylmethyl)-1-piperazinyl]-ethoxy]ethylthio]-7-methyl[1,2,4]triazolo[1,5-b]pyridazine dihydrochloride

step A : Production of 6-[2-(2-bromoethoxy)ethylthio]-7-methyl[1,2,4]triazolo[1,5-b]pyridazine

Methyl 3-mercaptopropionate (5.57 g) was dissolved in methanol (20 ml); 2 N sodium methoxide solution in methanol (35.6 ml) and 6-chloro-7-methyl[1,2,4]triazolo[1,5-b]pyridazine (2.0 g) were added thereto, followed by heating and refluxing for 1 hour. After cooling, the mixture was concentrated under reduced pressure; ethyl acetate was added to the residue; the crystals precipitated were collected, washed with ethyl acetate and suspended in tetrahydrofuran (30 ml); 2-bromoethyl ether (2.98 ml) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane:ethyl acetate:methanol (30:30:1). The desired fractions were collected to give the title compound (2.06 g) as an oil.
$^1$H-NMR($CDCl_3$)δ ppm: 2.42(3H,s), 3.50(2H,t,J=6Hz), 3.56(2H,t,J=6Hz), 3.86(2H,t,J=6Hz), 3.91(2H,t,J=6Hz), 7.74 (1H,s), 8.30(1H,s).

step B:

6- [2- (2-Bromoethoxy) ethylthio] -7-methyl[1,2,4]triazolo[1,5-b]pyridazine (1.0 g) and 1-(diphenylmethyl)piperazine (790 mg) were dissolved in N,N-dimethylformamide (10 ml); potassium carbonate (520 mg) was added thereto, followed by stirring at room temperature for 23 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane: ethyl acetate:methanol (5:5:1). The desired fractions were collected and concentrated; the residue was dissolved in ethyl acetate (5 ml); 4 N hydrogen chloride solution in ethyl acetate (1.55 ml) was added thereto; the crystals precipitated were collected by filtration, washed with ethyl ether and dried to give the title compound (0.85 g), which was then recrystallized from ethanol.
melting point: 198-200°C
elemental analysis for $C_{27}H_{34}N_6OSCl_2$
Calculated (%): C,57.75 ; H,6.10 ; N,14.97
Found (%): C,57.53 ; H,6.00 ; N,14.93

**Reference Example B56**

Production of 6-[6-[4-(diphenylmethoxy)piperidino]hexylthio]-7-methyl[1,2,4]triazolo[1,5-b]pyridazine fumarate

6-(6-Bromohexylthio)-7-methyl[1,2,4]triazolo[1,5-b]pyridazine (1.0 g) and 4-(diphenylmethoxy)piperidine (810 mg) were dissolved in N,N-dimethylformamide (10 ml); potassium carbonate (500 mg) was added thereto, followed by stirring at room temperature for 24 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10: 1). The desired fractions were collected and concentrated; the residue was dissolved in ethanol (10 ml); fumaric acid (290 mg) was added thereto, followed by concentration. Ethyl ether was added to the residue; the crystals precipitated were collected by filtration, washed with ethyl ether and dried to give the title compound (1.43 g).
melting point: 137-138°C
elemental analysis for $C_{34}H_{41}N_5O_5S \cdot 0.5H_2O$
Calculated (%): C,63.73 ; H,6.61 ; N,10.93
Found (%): C,63.97 ; H,6.44 ; N,11.00

**Reference Example B57**

Production of 6-[2-[2-[4-(diphenylmethoxy)piperidino]-ethoxy]ethylthio]-7-methyl[1,2,4]triazolo[1,5-b]pyridazine fumarate

6-[2-(2-Bromoethoxy)ethylthio]-7-methyl[1,2,4]triazolo[1,5-b]pyridazine (1.09 g) and 4-(diphenylmethoxy)piperidine (840 mg) were dissolved in N,N-dimethylformamide (10 ml); potassium carbonate (520 mg) was added thereto, followed by stirring at room temperature for 23 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with dichloromethane: ethyl acetate:methanol (5:5:1). The desired fractions were collected; the residue was dissolved in 10 ml of ethanol; fumaric acid (200 mg) was added, followed by concentration. Ethyl ether was added to the residue; the crystals precipitated were collected by filtration, washed with ethyl ether and dried to give the title compound (0.78 g).
melting point: 119-122°C
elemental analysis for $C_{32}H_{37}N_5O_6S \cdot 0.5H_2O$
Calculated (%): C,61.13 ; H,6.09 ; N,11.14
Found (%): C,61.12 ; H,5.82 ; N,11.40

**Reference Example B58**

Production of 6-[2-[2-[4-(diphenylmethoxy)piperidino]-ethoxy]ethylthio][1,2,4]triazolo[1,5-b]pyridazine fumarate

6-[2-(2-Bromoethoxy)ethylthio][1,2,4]triazolo[1,5-b]pyridazine (1.35 g) and 4-(diphenylmethoxy)piperidine (1.19 g) were dissolved in N,N-dimethylformamide (15 ml); potassium carbonate (740 mg) was added thereto, followed by stirring at room temperature for 17 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10: 1). The desired fractions were collected; the residue was dissolved in ethanol (10 ml); fumaric acid (360 mg) was added thereto, followed by concentration. Ethyl ether was added to the residue; the crystals precipitated were collected by filtration, washed with ethyl ether and dried to give the title compound (1.64 g).
melting point: 110-111°C
elemental analysis for $C_{31}H_{35}N_5O_6S \cdot 0.5H_2O$
Calculated (%): C,60.57 ; H,5.90 ; N,11.39
Found (%): C,60.35 ; H,5.73 ; N,11.16

**Reference Example B59**

Production of 6-[2-[2-[4-(diphenylmethyl)-1-piperazinyl]-ethoxy]ethylthio]-7-isopropyl[1,2,4]triazolo[1,5-b]pyridazine dihydrochloride

step A : Production of 6-[2-(2-bromoethoxy)ethylthio]-7-isopropyl[1,2,4]triazolo[1,5-b]pyridazine

Methyl 3-mercaptopropionate (2.05 g) was dissolved in methanol (10 ml); 2 N sodium methoxide solution in methanol (7.64 ml) and 6-chloro-7-isopropyl[1,2,4]triazolo[1,5-b]pyridazine (1.0 g) were added thereto, followed by heating and refluxing for 1 hour. After cooling, the mixture was concentrated under reduced pressure; ethyl acetate was added to the residue; the crystals precipitated were collected, washed with ethyl acetate and suspended in tetrahydrofuran (15 ml); 2-bromoethyl ether (1.28 ml) was added thereto, followed by heating and refluxing for 2 hours. After cooling, ice water was added, followed by extraction with ethyl acetate; the extract was washed with brine and dried over magnesium sulfate. After the extract was concentrated under reduced pressure, the residue was subjected to silica gel column chromatography and eluted with ethyl acetate:methanol (20:1). The desired fractions were collected to give the title compound (0.98 g).
$^1$H-NMR($CDCl_3$)$\delta$ ppm: 1.35(6H,s), 3.15-3.30(1H,m), 3.50(2H,t,J=6Hz), 3.55(2H,t,J=6Hz), 3.86(2H,t,J=6Hz), 3.91(2H, t,J=6Hz), 7.80(1H,s), 8.31(1H,s).

step B:

6-[2-(2-Bromoethoxy)ethylthio]-7-isopropyl[1,2,4]triazolo[1,5-b]pyridazine (0.98 g) and 1-(diphenylmethyl) piperazine (720 mg) were dissolved in N,N-dimethylformamide (10 ml); potassium carbonate (470 mg) was added

thereto, followed by stirring at room temperature for 15 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate: methanol (10:1). The desired fractions were collected and concentrated; the residue was dissolved in ethyl acetate (5 ml); 4 N hydrogen chloride solution in ethyl acetate (1.45 ml) was added thereto; the crystals precipitated were collected by filtration, washed with ethyl ether and dried to give the title compound (1.04 g), which was then recrystallized from ethanol.
melting point: 143-145°C
elemental analysis for $C_{29}H_{38}N_6OSCl_2 \cdot H_2O$
Calculated (%): C,57.32 ; H,6.64 ; N,13.83
Found (%): C,57.20 ; H,6.43 ; N,13.89

**Reference Example B60**

Production of 6-[2-[2-[4-(diphenylmethyl)-1-piperazinyl]-ethoxy]ethylthio]-7-tert-butyl[1,2,4]triazolo[1,5-b]pyridazine

step A: Production of 6-[2-(2-bromoethoxy)ethylthio]-7-tert-butyl[1,2,4]triazolo[1,5-b]pyridazine

Methyl 3-mercaptopropionate (2.23 g) was dissolved in methanol (10 ml); 2 N sodium methoxide solution in methanol (7.2 ml) and 6-chloro-7-t-butyl[1,2,4]triazolo[1,5-b]-pyridazine (1.0 g) were added thereto, followed by heating and refluxing for 1 hour. After cooling, the mixture was concentrated under reduced pressure; ethyl acetate was added to the residue; the crystals precipitated were collected, washed with ethyl acetate and suspended in tetrahydrofuran (20 ml); 2-bromoethyl ether (1.19 ml) was added thereto, followed by heating and refluxing for 3 hours. After cooling, ice water was added, followed by extraction with ethyl acetate; the extract was washed with brine and dried over magnesium sulfate. After the extract was concentrated under reduced pressure, the residue was subjected to silica gel column chromatography and eluted with ethyl acetate:hexane (2:1). The desired fractions were collected to give the title compound (1.06 g).
$^1$H-NMR($CDCl_3$)δ ppm: 1.56(9H,s), 3.50(2H,t,J=6Hz), 3.58(2H,t,J=6Hz), 3.86(2H,t,J=6Hz), 3.92(2H,t,J=6Hz), 7.94 (1H,s), 8.32(1H,s).

step B:

6-[2-(2-Bromoethoxy)ethylthio]-7-tert-butyl[1,2,4]triazolo[1,5-b]pyridazine (1.06 g) and 1-(diphenylmethyl) piperazine (740 mg) were dissolved in N,N-dimethylformamide (10 ml); potassium carbonate (480 mg) was added thereto, followed by stirring at room temperature for 18 hours. Ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate: methanol (20:1). The desired fractions were collected and concentrated; the crystals precipitated were collected by filtration, washed with ethyl ether and dried to give the title compound (0.85 g).
melting point: 106-108°C
elemental analysis for $C_{30}H_{38}N_6OS$
Calculated (%): C,67.89 ; H,7.22 ; N,15.83
Found (%): C,67.65 ; H,7.33 ; N,15.98

**Reference Example B61**

Production of 6-[2-[2-[4-(diphenylmethyl)-1-piperazinyl]-ethoxy]ethoxy]-7-isopropyl[1,2,4]triazolo[1,5-b]pyridazine

60% oily sodium hydride (160 mg) was suspended in tetrahydrofuran (20 ml); 2-[2-[4-(diphenylmethyl)-1-piperazinyl]ethoxy]ethanol (1.20 g) was added thereto, followed by heating and refluxing for 1 hour. After cooling, 6-chloro-7-isopropyl[1,2,4]triazolo[1,5-b]pyridazine (610 mg) was added thereto, followed by heating and refluxing for 1 hour. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10:1). The desired fractions were collected; the crystals precipitated were collected by filtration, washed with ethyl ether and dried to give the title compound (790 mg).
melting point: 119-120°C
elemental analysis for $C_{29}H_{36}N_6O_2 \cdot 0.5H_2O$

Calculated (%): C,68.34 ; H,7.32 ; N,16.49
Found (%): C,68.64 ; H,7.31 ; N,16.54

**Reference Example B62**

Production of 7-tert-butyl-6-[2-[2-[4-(diphenylmethyl)-1-piperazinyl]ethoxy]ethoxy][1,2,4]triazolo[1,5-b]pyridazine dihydrochloride

60% Oily sodium hydride (150 mg) was suspended in tetrahydrofuran (20 ml); 2-[2-[4-(diphenylmethyl)-1-piperazinyl]ethoxy]ethanol (1.05 g) was added thereto, followed by heating and refluxing for 1 hour. After cooling, 7-tert-butyl-6-chloro[1,2,4]triazolo[1,5-b]pyridazine (650 mg) was added thereto, followed by heating and refluxing for 2 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10:1). The desired fractions were collected; the residue was dissolved in ethyl acetate (5 ml); 4 N hydrogen chloride solution in ethyl acetate (2.1 ml) was added thereto; the crystals precipitated were collected by filtration, washed with ethyl ether and dried to give the title compound (1.55 g).
melting point: 150-152°C
elemental analysis for $C_{30}H_{40}N_6O_2Cl_2 \cdot 0.5H_2O$
Calculated (%): C,60.39 ; H,6.92 ; N,14.09
Found (%): C,60.20 ; H,6.64 ; N,14.09

**Reference Example B63**

Production of 7-tert-butyl-6-[2-[2-[4-(diphenylmethoxy)-piperidino]ethoxy]ethoxy][1,2,4]triazolo[1,5-b]pyridazine fumarate

60% Oily sodium hydride (120 mg) was suspended in tetrahydrofuran (20 ml); 2-[2-4-(diphenylmethoxy)-piperidino]ethoxy]ethanol (0.94 g) was added thereto, followed by heating and refluxing for 1 hour. After cooling, 7-tert-butyl-6-chloro[1,2,4]triazolo[1,5-b]pyridazine (530 mg) was added thereto, followed by heating and refluxing for 3 hours. After cooling, ice water was added thereto, followed by extraction with ethyl acetate; the extract was washed with brine and dried over magnesium sulfate. After the extract was concentrated under reduced pressure, the residue was subjected to silica gel column chromatography and eluted with ethyl acetate:methanol (10:1). The desired fractions were collected; the residue was dissolved in ethanol (10 ml); fumaric acid (250 mg) was added thereto, followed by concentration. Ethyl ether was added to the residue; the crystals precipitated were collected by filtration, washed with ethyl ether and dried to give the title compound (1.17 g).
melting point: 80-82°C
elemental analysis for $C_{35}H_{43}N_5O_7 \cdot 1.3H_2O$
Calculated (%): C,62.82 ; H,6.87 ; N,10.46
Found (%): C,62.89 ; H,6.69 ; N,10.22

**Reference Example B64**

Production of ethyl 2-[6-[5-[4-(diphenylmethoxy)piperidino]-pentylamino]imidazo[1,2-b]pyridazin-2-yl]-2-methylpropionate difumarate

4-(Diphenylmethoxy)-1-piperidinepentanamine (1.41 g) and ethyl 2-[6-chloroimidazo[1,2-b]pyridazin-2-yl]-2-methylpropionate (0.536 g) were stirred at 190-200°C for 3.5 hours. After cooling, ethyl acetate:tetrahydrofuran (2:1) was added; the mixture was washed with aqueous sodium hydrogen carbonate solution and brine and dried over sodium sulfate. The extract was concentrated under reduced pressure; the residue was subjected to silica gel column chromatography and eluted with ethyl acetate:methanol:triethylamine (185:15:2). The desired fractions were collected, dissolved in ethanol (5 ml); a solution of fumaric acid (235 mg) in methanol (5 ml) was added thereto, followed by concentration. Ethyl ether was added to the residue; the resulting powder was collected by filtration, washed with ethyl ether and dried to give the title compound (0.629 g).
melting point: 138°C
elemental analysis for $C_{43}H_{53}N_5O_{11}$
Calculated (%): C,63.30 ; H,6.55 ; N,8.58
Found (%): C,64.24 ; H,6.92 ; N,8.42

**Reference Example B65**

Production of ethyl 2-[6-[3-[4-(diphenylmethoxy)piperidino]-2-hydroxypropylamino]imidazo[1,2-b]pyridazin-2-yl]-2-methylpropionate difumarate

1-Amino-3-[4-(diphenylmethoxy)piperidino]-2-propanol (0.511 g) and ethyl 2-(6-chloroimidazo[1,2-b]pyridazin-2-yl)-2-methylpropionate (0.268 g) were stirred at 190-200°C for 3 hours. After cooling, ethyl acetate:tetrahydrofuran (2:1) was added thereto; the mixture was washed with aqueous sodium hydrogen carbonate solution and brine and dried over magnesium sulfate. The extract was concentrated under reduced pressure; the residue was subjected to silica gel column chromatography and eluted with ethyl acetate:methanol:triethylamine (90:10:1). The desired fractions were collected, dissolved in ethyl acetate (5 ml); a solution of fumaric acid (82 mg) in methanol (5 ml) was added thereto, followed by concentration. Ethyl ether was added to the residue; the resulting powder was collected by filtration, washed with ethyl ether and dried to give the title compound (0.223 g).
melting point: 145°C
elemental analysis for $C_{41}H_{49}N_5O_{12}\cdot Et_2O$
Calculated (%): C,61.56 ; H,6.77 ; N,7.98
Found (%): C.61.39 ; H,6.49 ; N,7.91

**Reference Example B66**

Production of ethyl 2-[6-[3-[4-[bis(4-fluorophenyl)methoxy]-piperidino]propylamino]imidazo[1,2-b]pyridazin-2-yl]-2-methylpropionate difumarate

4-[Bis(4-fluorophenyl)methoxy]-1-piperidinepropanamine (1.62 g) and ethyl 2-(6-chloroimidazo[1,2-b]pyridazin-2-yl)-2-methylpropionate (0.803 g) were stirred at 190-200°C for 3 hours. After cooling, aqueous sodium hydrogen carbonate solution and saline were added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (90:10:1). The desired fractions were collected, dissolved in ethyl acetate (20 ml) ; a solution of fumaric acid (301 mg) in methanol (10 ml) was added thereto, followed by concentration. Acetone was added to the residue; the crystals precipitate were collected by filtration, washed with acetone and dried to give the title compound (0.966 g) .
melting point: 159-161°C
elemental analysis for $C_{41}H_{47}N_5O_{11}F_2\cdot 0.5H_2O$
Calculated (%): C,59.13 ; H,5.81 ; N,8.41
Found (%): C,58.94 ; H,5.84 ; N,8.34

**Reference Example B67**

Production of ethyl 6-[3-[4-(diphenylmethoxy)piperidino]-propylamino]imidazo[1,2-b]pyridazine-2-carboxylate difumarate

4-(Diphenylmethoxy)-1-piperidinepropanamine (686 mg) and ethyl 6-chloroimidazo[1,2-b]pyridazin-2-carboxylate (477 mg) were dissolved in N,N-dimethylformamide (7 ml); N-ethyldiisopropylamine (0.73 ml) was added thereto, followed by stirring in an oil bath (80°C) for 18.5 hours. After cooling, ice water and sodium chloride were added thereto, followed by extraction with ethyl acetate:tetrahydrofuran (1:1); the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, dissolved in ethyl acetate (5 ml); a solution of fumaric acid (95 mg) in ethanol (5 ml) was added thereto, followed by concentration. Acetone:ethyl ether (1:2) was added to the residue to cause recrystallization; the crystals precipitated were collected by filtration and washed with ethyl ether to give the title compound (211 mg).
melting point: 176-179°C
elemental analysis for $C_{38}H_{43}N_5O_{11}$
Calculated (%): C,61.20 ; H,5.81 ; N,9.39
Found (%): C,61.17 ; H,5.98 ; N,9.80

**Reference Example B68**

Production of isopropyl 2-[6-[3-[4-(diphenylmethoxy)piperidino]propoxy]imidazo[1,2-b]pyridazin-2-yl]-2-methylpropionate difumarate

4-(Diphenylmethoxy)piperidinepropanol (8.10 g) was dissolved in N,N-dimethylformamide (60 ml); 60% oily sodium hydride (1.11 g) was added thereto, followed by stirring at room temperature under reduced pressure for 1 hour. While the solution was ice cooled, isopropyl 2-(6-chloroimidazo [1,2-b]pyridazin-2-yl)-2-methylpropionate (7.79 g) was added thereto, followed by stirring at the same temperature for 4 hours. Ice water was added thereto, followed by extraction with ethyl acetate:tetrahydrofuran (1:1); the extract was washed with brine and dried over magnesium sulfate. The extract was concentrated under reduced pressure; the residue was subjected to silica gel column chromatography and eluted with ethyl acetate. The desired fractions were collected and concentrated under reduced pressure; the residue was dissolved in ethanol (10 ml); fumaric acid (476 mg) was added thereto, followed by concentration again. The residue was crystallized by the addition of ethyl acetate, collected by filtration, washed with ethyl acetate and dried to give the title compound (1.05 g).
melting point: 145-147°C
elemental analysis for $C_{42}H_{50}N_4O_{12}$
Calculated (%): C,62.83 ; H,6.28 ; N,6.98
Found (%): C.62.50 ; H,6.10 ; N,7.04

**Reference Example B69**

Production of ethyl 2-[6-[3-[4-[bis(4-methylphenyl)-methoxy]piperidino]propylamino]imidazo[1,2-b]pyridazin-2-yl]-2-methylpropionate difumarate

4-[Bis(4-methylphenyl)methoxy]-1-piperidinepropanamine (2.11 g) and ethyl 2-(6-chloroimidazo[1,2-b]pyridazin-2-yl)-2-methylpropionate (0.803 g) were stirred at 190-200°C for 3 hours. After cooling, aqueous sodium hydrogen carbonate solution was added, followed by extraction with ethyl acetate; the extract was washed with brine and dried over sodium sulfate. The extract was concentrated under reduced pressure; the residue was subjected to silica gel column chromatography and eluted with ethyl acetate:methanol:triethylamine (95:5:1). The desired fractions were collected, dissolved in ethyl acetate (20 ml); a solution of fumaric acid (358 mg) in methanol (20 ml) was added thereto, followed by concentration. Acetone was added to the residue; the crystals precipitated were collected by filtration, washed with acetone and dried to give the title compound (0.901 g).
melting point: 159-161°C
elemental analysis for $C_{43}H_{53}N_5O_{11}$
Calculated (%): C,63.30 ; H,6.55 ; N,8.56
Found (%): C,63.29 ; H,6.32 ; N,8.67

**Reference Example B70**

Production of N-[6-[3-[4-(diphenylmethoxy)piperidino]propylamino]imidazo[1,2-b]pyridazine-2-carbonyl]glycine ethyl ester

4-(Diphenylmethoxy)-1-piperidinepropanamine (1.90 g) and N-(6-chloroimidazo-[1,2-b]pyridazine-2-carbonyl)glycine ethyl ester (1.38 g) were dissolved in 1-methyl-2-pyrrolidone (15 ml); N-ethyldiisopropylamine (0.841 ml) was added thereto, followed by stirring in an oil bath (90-100°C) for 24 hours. After cooling, ice water and saline were added, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (95:5:1). The desired fractions were collected and recrystallized from ethyl acetate to give the title compound (1.28 g).
melting point: 172-174°C
elemental analysis for $C_{32}H_{38}N_6O_4 \cdot 0.5H_2O$
Calculated (%): C,66.30 ; H,6.78 ; N,14.50
Found (%): C,66.42 ; H,6.68 ; N,14.55

**Reference Example B71**

Production of N-[6-[3-[4-(diphenylmethoxy)piperidino]-propylamino]imidazo[1,2-b]pyridazine-2-carbonyl]glycine ethyl ester dihydrochloride

N-[6-[3-[4-(Diphenylmethoxy)piperidino] propylamino]imidazo[1,2-b]pyridazine-2-carbonyl]glycine ethyl ester (0.628 g) was dissolved in tetrahydrofuran (10 ml); 4 N hydrogen chloride solution in ethyl acetate (1.5 ml) was added thereto, followed by concentration under reduced pressure. To the residue, methanol (10 ml) was added, followed by concentration under reduced pressure. The crystals obtained were collected and washed with ethyl acetate to give the title compound (0.658 g).
melting point: 205°C
elemental analysis for $C_{32}H_{40}N_6O_4Cl_2$
Calculated (%): C,59.72 ; H,6.26 ; N,13.06
Found (%): C,59.74 ; H,6.41 ; N,12.63

**Reference Example B72**

Production of N-[6-[3-[4-(diphenylmethoxy)piperidino]-propylamino]imidazo[1,2-b]pyridazine-2-carbonyl]glycine

N-[6-[3-[4-(Diphenylmethoxy)piperidino]-propylamino]imidazo[1,2-b]pyridazine-2-carbonyl]glycine ethyl ester (0.810 g) was dissolved in ethanol (4 ml); a 1 N aqueous solution of sodium hydroxide (2 ml) was added thereto, followed by stirring at room temperature for 3 hours. The mixture was concentrated under reduced pressure; ice water and 1 N hydrochloric acid (2.1 ml) were added to the residue, followed by extraction with ethyl acetate:tetrahydrofuran (1:2); the extract was washed with brine and dried over magnesium sulfate. The extract was concentrated under reduced pressure; the residue was powdered by the addition of ethyl acetate, collected by filtration and washed with ethyl acetate to give the title compound (0.183 g).
melting point: 171°C
elemental analysis for $C_{30}H_{34}N_6O_4 \cdot 2H_2O \cdot AcOEt$
Calculated (%): C,61.25 ; H,6.95 ; N,12.60
Found (%): C,61.30 ; H,6.74 ; N,12.45

**Reference Example B73**

Production of 2-[6-[3-[4-(diphenylmethoxy)piperidino]-propylamino]imidazo[1,2-b]pyridazin-2-yl]-2-methylpropionamide dihydrochloride

4-(Diphenylmethoxy)-1-piperidinepropanamine (1.29 g) and 2-(6-chloroimidazo[1,2-b]pyridazin-2-yl)-2-methylpropionamide (0.478 g) were stirred at 190-200°C for 70 minutes. After cooling, aqueous sodium hydrogen carbonate solution was added thereto, followed by extraction with ethyl acetate. The extract was washed with saline and dried over magnesium sulfate. The extract was concentrated under reduced pressure; the residue was subjected to silica gel column chromatography and eluted with ethyl acetate:methanol:triethylamine (90:10:1). The desired fractions were collected; the residue was dissolved in ethyl acetate (10 ml); 4 N hydrogen chloride solution in ethyl acetate (1.5 ml) was added thereto, followed by concentration under reduced pressure. Ethyl acetate was added to the residue; the resulting powder was collected by filtration, washed with ethyl acetate and dried to give the title compound (0.823 g).
melting point: 191°C
elemental analysis for $C_{31}H_{40}N_6O_2Cl_2 \cdot AcOEt$
Calculated (%): C,64.11 ; H,7.38 ; N,12.82
Found (%): C,63.70 ; H,7.27 ; N,12.34

**Reference Example B74**

Production of 2-[6-[3-[4-(diphenylmethoxy)piperidino]-propylamino]imidazo[1,2-b]pyridazin-2-yl]-N,N,2-trimethylpropionamide dihydrochloride

4-(Diphenylmethoxy)-1-piperidinepropanamine (1.04 g) and 2-(6-chloroimidazo[1,2-b]pyridazin-2-yl)-N,N-2-trimethylpropionamide (0.426 g) were stirred at 190-200°C for 60 minutes. After cooling, aqueous sodium hydrogen carbonate solution was added thereto, followed by extraction with ethyl acetate. The extract was washed with saline and dried over magnesium sulfate. The extract was concentrated under reduced pressure; the residue was subjected

to silica gel column chromatography and eluted with ethyl acetate:methanol:triethylamine (85:15:1). The desired fractions were collected; the residue was dissolved in ethyl acetate (10 ml); 4 N hydrogen chloride solution in ethyl acetate (1.5 ml) was added thereto, followed by concentration under reduced pressure. The residue was recrystallized from acetone to give the title compound (0.823 g).
melting point: 183°C
elemental analysis for $C_{33}H_{44}N_6O_2Cl_2 \cdot 1.5H_2O$
Calculated (%): C,60.54 ; H,7.24 ; N,11.84
Found (%): C,60.48 ; H,7.28 ; N,11.90

**Reference Example B75**

Production of 2-[6-[3-[4-(diphenylmethoxy)piperidino]-propylamino]imidazo[1,2-b]pyridazin-2-yl]-2-methylpropanol

4-(Diphenylmethoxy)-1-piperidinepropanamine (1.29 g) and 2-(6-chloroimidazo[1,2-b]pyridazin-2-yl)-2-methylpropanol (0.451 g) were stirred at 190-200°C for 90 minutes. After cooling, aqueous sodium hydrogen carbonate solution was added thereto, followed by extraction with ethyl acetate. The extract was washed with saline and dried over magnesium sulfate. The extract was concentrated under reduced pressure; the residue was subjected to silica gel column chromatography and eluted with ethyl acetate:methanol:triethylamine (90:10:1). The desired fractions were collected; the residue was recrystallized from ethyl ether to give the title compound (0.465 g).
melting point: 105-108°C
elemental analysis for $C_{31}H_{39}N_5O_2 \cdot 0.5H_2O$
Calculated (%): C,71.24 ; H,7.71 ; N,13.40
Found (%): C,71.22 ; H,7.87 ; N,13.32

**Reference Example B76**

Production of N-[6-[3-[4-(diphenylmethoxy)piperidino]-propylamino]imidazo[1,2-b]pyridazine-2-carbonyl]-2,2-dimethylglycine ethyl ester dihydrochloride

4-(Diphenylmethoxy)-1-piperidinepropanamine (1.23 g) and N-(6-chloroimidazo[1,2-b]pyridazine-2-carbonyl)-2,2-dimethylglycine ethyl ester (1.18 g) were dissolved in N,N-dimethylformamide (15 ml); N-ethyldiisopropylamine (1.31 ml) was added thereto, followed by stirring at 70°C for 9.5 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated under reduced pressure and dissolved in ethyl acetate (5 ml); 4 N hydrogen chloride solution in ethyl acetate (0.28 ml) was added thereto, followed by concentration again. Ethyl acetate was added to the residue; the crystals precipitated were collected by filtration, washed with ethyl ether and dried to give 284 mg of the title compound.
melting point: 194-196°C
elemental analysis for $C_{34}H_{44}N_6O_4Cl_2$
Calculated (%): C,60.80 ; H,6.60 ; N,12.51
Found (%): C,60.82 ; H,6.67 ; N,12.77

**Reference Example B77**

Production of ethyl 2-[6-[3-[4-(diphenylmethyl)piperazino]-propylamino]imidazo[1,2-b]pyridazin-2-yl]-2-methylpropionate trihydrochloride

4-(Diphenylmethyl)-1-piperazinepropanamine (1.31 g) and ethyl 2-(6-chloroimidazo[1,2-b]pyridazin-2-yl)-2-methylpropionate (567 mg) were stirred at 185°C for 3 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated under reduced pressure and dissolved in ethyl acetate (5 ml); 4 N hydrogen chloride solution in ethyl acetate (0.80 ml) was added thereto, followed by concentration again. Ethanol was added to the residue; the crystals precipitated were collected by filtration, washed with ethanol:ethyl acetate (1:3) and dried to give the title compound (502 mg).

melting point: 190-193°C
elemental analysis for $C_{32}H_{43}N_6O_2Cl_3 \cdot H_2O$
Calculated (%) :C,57.53 ; H,6.79 ; N,12.58
Found (%) :C,57.27 ; H,6.52 ; N,12.55

**Reference Example B78**

Production of 2-[6-[4-[4-(diphenylmethoxy)piperidino]-butylamino]imidazo[1,2-b]pyridazin-2-yl]-2-methylpropionic acid

4-(Diphenylmethoxy)-1-piperidinebutanamine (1.56 g) and ethyl 2-(6-chloroimidazo[1,2-b]pyridazin-2-yl)-2-methylpropionate (617 mg) were stirred at 185°C for 3 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated under reduced pressure and dissolved in ethyl acetate (5 ml); 4 N hydrogen chloride solution in ethyl acetate (0.52 ml) was added thereto, followed by concentration. The residue was dissolved in ethanol (4 ml); 1 N aqueous solution of sodium hydroxide (4 ml) was added thereto, followed by stirring at room temperature for 4 hours; 2 N aqueous solution of sodium hydroxide (1 ml) was added thereto, followed by stirring at 50°C for 16 hours. The mixture was concentrated under reduced pressure; the residue was diluted with water and washed with ethyl acetate; the water layer was adjusted to pH 4.5 by the addition of 4 N hydrochloric acid and extracted with ethyl acetate:tetrahydrofuran (1:1); the extract was washed with brine and dried over magnesium sulfate. The extract was concentrated under reduced pressure; the residue was powdered by the addition of ethyl acetate, collected by filtration and dried to give the title compound (271 mg).
Amorphous
elemental analysis for $C_{32}H_{39}N_5O_3 \cdot 2.1H_2O$, 0.5AcOEt
Calculated (%): C,65.49 ; H,7.63 ; N,11.23
Found (%): C,65.23 ; H,7.29 ; N,11.19

**Reference Example B79**

Production of 2-[6-[2-[4-(diphenylmethoxy)piperidino]-ethylamino]imidazo[1,2-b]pyridazin-2-yl]-2-methylpropionic acid

step A: Production of isopropyl 2-[6-(2-hydroxyethylamino)-imidazo[1,2-b]pyridazin-2-yl]-2-methylpropionate

2-Aminoethanol (130 mg) and isopropyl 2-(6-chloroimidazo[1,2-b]pyridazin-2-yl)-2-methylpropionate (300 mg) were stirred at 170°C for 4 hours. After cooling, 2-aminoethanol (260 mg) was added thereto, followed by stirring at 170°C for 45 minutes. After cooling, water was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol (10:1). The desired fractions were collected and concentrated under reduced pressure; the crystals precipitated were collected by filtration and dried to give the title compound (145 mg).
$^1$H-NMR($CDCl_3$)δ ppm: 1.29(3H,s), 1.32(3H,s), 1.64(6H,s), 3.44(2H,td,J=4.6,6.1Hz), 3.88(2H,t,J=4.6Hz), 4.96-5.15 (1H,m), 5.43(1H,t,J=6.2Hz), 5.72(1H,d,J=9.7Hz), 6.98(1H,d,J=9.7Hz), 7.45(1H,s).

step B: Production of isopropyl 2-[6-[(2-methanesulfonyloxy)-ethylamino]imidazo[1,2-b]pyridazin-2-yl]-2-methylpropionate

Isopropyl 2-[6-(2-hydroxyethylamino)imidazo[1,2-b]pyridazin-2-yl]-2-methylpropionate (2.18 g) was suspended in tetrahydrofuran (20 ml); N-ethyldiisopropylamine (2.45 ml) and methanesulfonyl chloride (1.10 ml) were added thereto, followed by stirring at room temperature for 1 hour. Ice water was added thereto, followed by extraction with ethyl acetate; the extract was washed with brine and dried over magnesium sulfate. The extract was concentrated under reduced pressure and dried to give the title compound (2.37 g).
$^1$H-NMR($CDCl_3$)δ ppm: 1.19(3H,s), 1.22(3H,s), 1.63(6H,s), 3.40(3H,s), 3.74(2H,td,J=5.1,5.4Hz), 4.48(2H,t,J=5.1Hz), 4.76(1H,t,J=5.4Hz), 4.95-5.12(1H,m), 6.39(1H,d,J= 9.6Hz), 7.54(1H,s), 7.62(1H,d,J=9.6Hz).

step C: Production of isopropyl 2-[6-[2-[4-(diphenylmethoxy)-piperidino]ethylamino]imidazo[1,2-b]pyridazin-2-yl]-2-methylpropionate

Isopropyl 2-[6-[2-(methanesulfonyloxy) ethylamino]imidazo[1,2-b]pyridazin-2-yl]-2-methylpropionate (1.13 g) was dissolved in N,N-dimethylformamide (15 ml); 4-(diphenylmethoxy)piperidine (943 mg), potassium iodide (586 mg) and potassium carbonate (488 mg) were added thereto, followed by stirring at 60°C for 2 hours. Ice water was added thereto; the mixture was saturated with sodium chloride and extracted with ethyl acetate; the extract was washed with brine and dried over magnesium sulfate. The extract was concentrated under reduced pressure; the residue was subjected to silica gel column chromatography and eluted with ethyl acetate:methanol (10:1). The desired fractions were collected, concentrated under reduced pressure and dried to give the title compound (571 mg).
$^1$H-NMR($CDCl_3$)δ ppm: 1.18(3H,s), 1.21(3H,s), 1.60-1.20(4H,m), 1.62(6H,s), 2.10-2.30(2H,m), 2.59(2H,t,J=5.6Hz), 2.70-2.85(2H,m), 3.35 (2H,dt,J=5.3,5.6Hz), 3.35-3.55 (1H,m), 4.90-5.10(1H,m), 5.05(1H), 5.53(1H,s), 6.39(1H,d, J=9.4Hz), 7.16-7.39(10H,m), 7.54(1H,s), 7.57(1H,d,J=9.4Hz).

step D:

Isopropyl 2-[6-[2-[4-(diphenylmethoxy)-piperidino]ethylamino]imidazo[1,2-b]pyridazin-2-yl]-2-methylpropionate (565 mg) was dissolved in ethanol (4 ml); 1 N aqueous sodium hydroxide solution (2.04 ml) was added thereto, followed by refluxing for 20 hours. After cooling, the mixture was concentrated under reduced pressure; the residue was diluted with water and adjusted to pH 5.5 by the addition of 1 N hydrochloric acid. Ethyl acetate was added thereto; the crystals precipitated were collected by filtration, washed with water and ethyl acetate and dried to give the title compound (443 mg).
melting point: 194-198°C
elemental analysis for $C_{30}H_{35}N_5O_3 \cdot 2.5H_2O$
Calculated (%): C,64.50 ; H,7.22 ; N,12.54
Found (%): C,64.57 ; H,7.03 ; N,12.58

**Reference Example B80**

Production of [6-[3-[4-(diphenylmethoxy)piperidino]-propylamino]imidazo[1,2-b]pyridazin-2-yl]carboxylic acid

Ethyl [6-[3-[4-(diphenylmethoxy)piperidino] propylamino]imidazo[1,2-b]pyridazin-2-yl]carboxylate (876 mg) was dissolved in ethanol (5 ml); 1 N aqueous sodium hydroxide solution (1.9 ml) was added thereto, followed by stirring at room temperature for 3 hours. After the mixture was concentrated under reduced pressure, the residue was diluted with water and washed with ethyl acetate; the water layer was adjusted to pH 5 by the addition of 1 N hydrochloric acid. The crystals precipitated were collected by filtration, washed with water and ethyl acetate and dried to give the title compound (256 mg).
melting point: 152-155°C
elemental analysis for $C_{28}H_{31}N_5O_3 \cdot 1.5H_2O$
Calculated (%): C,65.61 ; H,6.69 ; N,13.66
Found (%): C,65.52 ; H,6.61 ; N,13.61

**Reference Example B81**

Production of ethyl 2-[3-chloro-6-[3-[4-(diphenyimethoxy)-piperidino]propoxy]imidazo[1,2-b]pyridazin-2-yl]-2-methylpropionate 0.5 fumarate

4-(Diphenylmethoxy)-1-piperidinepropanol (334 mg) was dissolved in 20 ml of N,N-dimethylformamide; 60% oily sodium hydride (45 mg) was added thereto, followed by stirring at room temperature under reduced pressure for 35 minutes. Ethyl 2-[3,6-dichloroimidazo[1,2-b]pyridazin-2-yl]-2-methylpropionate (310 mg) was then added thereto, followed by stirring at 0°C for 2 hours. Ice water was added thereto, followed by extraction with ethyl acetate; the extract was washed with brine and dried over magnesium sulfate. The extract was concentrated under reduced pressure; the residue was subjected to silica gel column chromatography and eluted with ethyl acetate. The desired fractions were collected; the crystals precipitated were dissolved in ethanol (5 ml); fumaric acid (160 mg) was added thereto, followed by concentration under reduced pressure. Ethyl acetate was added to the residue; the mixture was washed with aqueous sodium hydrogen carbonate solution and brine, dried over magnesium sulfate and concentrated under reduced pressure. Ethyl acetate was added to the residue to cause crystallization; the crystals precipitated were collected by filtration, washed with ethyl ether and dried to give the title compound (168 mg).

melting point: 186-188°C
elemental analysis for $C_{35}H_{41}N_4O_6Cl \cdot 0.5H_2O$
Calculated (%): C,63.87 ; H,6.43 ; N,8.51
Found (%): C,63.33 ; H,6.34 ; N,8.85

**Reference Example B82**

Production of ethyl 2-[3-chloro-6-[3-[4-(diphenylmethyl)-1-piperazinyl]propoxy]imidazo[1,2-b]pyridazin-2-yl]-2-methylpropionate dihydrochloride

4-(Diphenylmethyl)-1-piperazinepropanol (1.0 g) was dissolved in N,N-dimethylformamide (10 ml); 60% oily sodium hydride (142 mg) was added thereto, followed by stirring at room temperature under reduced pressure for 40 minutes. To the reaction mixture, 973 mg of ethyl 2-(3,6-dichloroimidazo[1,2-b]pyridazin-2-yl)-2-methylpropionate (973 mg) was added thereto, followed by stirring at 0°C for 2 hours. Ice water was added thereto, followed by extraction with ethyl acetate; the extract was washed with brine and dried over magnesium sulfate. The extract was concentrated under reduced pressure; the residue was subjected to silica gel column chromatography and eluted with hexane:ethyl acetate:triethylamine (50:50:1). The desired fractions were collected and concentrated under reduced pressure; the crystals precipitated were dissolved in ethyl acetate (5 ml); 4 N hydrogen chloride solution in ethyl acetate (1.01 ml) was added, followed by concentration again. The residue was recrystallized from methanol, collected by filtration, washed with ethyl acetate and dried to give the title compound (424 mg).
melting point: 203-205°C
elemental analysis for $C_{32}H_{40}N_5O_3Cl_3 \cdot H_2O$
Calculated (%): C,57.62 ; H,6.35 ; N,10.50
Found (%): C.57.60 ; H,6.37 ; N,10.15

**Reference Example B83**

Production of ethyl 2-[3-chloro-6-[3-[4-(diphenylmethoxy)-piperidino]propylamino]imidazo[1,2-b]pyridazin-2-yl]-2-methylpropionate dihydrochloride

4-(Diphenylmethoxy)-1-piperidinepropanamine (2.56 g) and ethyl 2-[3,6-dichloroimidazo[1,2-b]pyridazin-2-yl]-2-methylpropionate (1.19 g) were stirred at 160°C for 3 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected and dissolved in ethyl acetate (5 ml); 4 N hydrogen chloride solution in ethyl acetate (0.80 ml) was added thereto, followed by concentration. The residue was powdered by the addition of ether and dried to give the title compound (1.33 g).
Amorphous
elemental analysis for $C_{33}H_{42}N_5O_3Cl_3 \cdot 0.5H_2O$
Calculated (%): C,58.97 ; H,6.45 ; N,10.42
Found (%): C,58.98 ; H,6.64 ; N,10.42

**Reference Example B84**

Production of ethyl 2-[3-chloro-6-[3-[4-(diphenylmethyl)-1-piperazinyl]propylamino]imidazo[1,2-b]pyridazin-2-yl]-2-methylpropionate trihydrochloride

4-(Diphenylmethyl)-1-piperazinepropanamine (1.75 g) and ethyl 2-[3,6-dichloroimidazo[1,2-b]pyridazin-2-yl]-2-methylpropionate (854 mg) were stirred at 160°C for 4 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol (30:1). The desired fractions were collected, concentrated under reduced pressure and dissolved in ethyl acetate (5 ml); 4 N solution of hydrogen chloride in ethyl acetate (1.55 ml) was added, followed by concentration again. The crystals precipitated were washed by the addition of ethanol: ethyl acetate (1:3), collected by filtration and dried to give the title compound (628 mg).
melting point: 203-205°C
elemental analysis for $C_{32}H_{42}N_6O_2Cl_4 \cdot H_2O$

Calculated (%): C,54.71 ; H,6.31 ; N,11.96
Found (%): C.54.88 ; H,6.07 ; N,11.97

**Reference Example B85**

Production of 2-[3-chloro-6-[3-[4-(diphenylmethyl)-1-piperazinyl]propylamino]imidazo[1,2-b]pyridazin-2-yl]-2-methylpropionic acid

Ethyl 2-[3-chloro-6-[3-[4-(diphenylmethyl)-1-piperazino]propylamino]imidazo[1,2-b]pyridazin-2-yl]-2-methylpropionate trihydrochloride (633 mg) was dissolved in ethanol (6 ml); 2 N aqueous solution of sodium hydroxide (2.31 ml) was added thereto, followed by thermal refluxing for 1.5 hours. After cooling, the mixture was concentrated under reduced pressure; the residue was diluted with water and washed with ethyl acetate; the water layer was adjusted to pH 5 by the addition of 1 N hydrochloric acid. Methanol was added thereto; the crystals precipitated were collected by filtration, washed with water-ethyl acetate and dried to give the title compound (462 mg).
melting point: 184-186°C
elemental analysis for $C_{30}H_{35}N_6O_2Cl\cdot H_2O$
Calculated (%): C,63.76 ; H,6.60 ; N,14.87
Found (%): C,63.49 ; H,6.52 ; N,14.81

**Reference Example B86**

Production of 2-[6-[2-[4-(diphenylmethyl)-1-piperazinyl]ethylamino]imidazo[1,2-b]pyridazin-2-yl]-2-methylpropionic acid

step A: Production of isopropyl 2-[6-[2-[4-(diphenylmethyl)-1-piperazinyl]ethylamino]imidazo[1,2-b]pyridazin-2-yl]-2-methylpropionate

Isopropyl 2-[6-[2-(methanesulfonyloxy)ethylamino]-imidazo[1,2-b]pyridazin-2-yl]-2-methylpropionate (1.24 g) was dissolved in N,N-dimethylformamide (15 ml); 1-(diphenylmethyl)piperazine (977 mg), potassium iodide (642 mg) and potassium carbonate (535 mg) were added thereto, followed by stirring at room temperature for 1 hour and at 60°C for 1.5 hours. Ice water and sodium chloride were added thereto, followed by extraction with ethyl acetate; the extract was washed with brine and dried over magnesium sulfate. The extract was concentrated under reduced pressure; the residue was subjected to silica gel column chromatography and eluted with ethyl acetate. The desired fractions were collected, concentrated under reduced pressure and dried to give the title compound (570 mg).
$^1$H-NMR($CDCl_3$)$\delta$ ppm: 1.17(3H,s), 1.20(3H,s), 1.62(6H,s), 2.36-2.60(8H,m), 2.63(2H,t,J=5.8Hz), 3.37(2H,dt, J=5.6,5.8Hz), 4.24(1H,s), 4.37(1H), 4.90-5.10(1H,m), 6.38(1H,d,J=9.6Hz), 7.13-7.44(10H,m), 7.52(1H,s), 7.55(1H,d, J=9.4Hz).

step B:

Isopropyl 2-[6-[2-[4-(diphenylmethyl)-1-piperazino]ethylamino]imidazo[1,2-b]pyridazin-2-yl]-2-methylpropionate (565 mg) was dissolved in ethanol (4 ml); 1 N aqueous solution of sodium hydroxide (2.09 ml) was added thereto, followed by thermal refluxing for 19 hours. After cooling, the mixture was concentrated under reduced pressure; the residue was diluted with water and adjusted to pH 5 by the addition of 1 N hydrochloric acid. Ethyl acetate was added thereto; the crystals precipitated were collected, washed with water and methanol and recrystallized from N,N-dimethylformamide-ethyl acetate (5:1), collected by filtration, washed with ethyl acetate and dried to give the title compound (249 mg).
melting point: 192-194°C
elemental analysis for $C_{29}H_{34}N_6O_2\cdot 3.0H_2O$
Calculated (%): C,63.02 ; H,7.30 ; N,15.21
Found (%): C,62.99 ; H,6.72 ; N,15.01

**Reference Example B87**

Production of 2-[3-chloro-6-[3-[4-(diphenylmethoxy)-piperidino]propylamino]imidazo[1,2-b]pyridazin-2-yl]-2-methylpropionic acid

Ethyl 2-[3-chloro-6-[3-[4-(diphenylmethoxy)-piperidino]propylamino]imidazo[1,2-b] pyridazin-2-yl]-2-methylpropionate dihydrochloride (653 mg) was dissolved in ethanol (6 ml); 2 N aqueous solution of sodium hydroxide (1.97 ml) was added thereto, followed by thermal refluxing for 2.5 hours. After cooling, the mixture was concentrated under reduced pressure; the residue was diluted with water and washed with ethyl acetate; the water layer was adjusted to pH 4.5 by the addition of 1 N hydrochloric acid. Acetone was added; the crystals precipitated were collected by filtration, washed with water-acetone (5:1) and dried to give the title compound (465 mg).
melting point: 133-135°C
elemental analysis for $C_{31}H_{36}N_5O_3Cl \cdot H_2O$
Calculated (%): C,64.18 ; H,6.60 ; N,12.07
Found (%): C,64.16 ; H,6.64 ; N,12.33

**Reference Example B88**

Production of 2-[3-chloro-6-[3-[4-(diphenylmethyl)-1-piperazinyl]propoxy]imidazo[1,2-b]pyridazin-2-yl]-2-methylpropionic acid

Ethyl 2-[3-chloro-6-[3-[4-(diphenylmethyl)-1-piperazinyl]propoxy]imidazo[1,2-b]pyridazin-2-yl]-2-methylpropionate trihydrochloride (458 mg) was dissolved in 2-propanol (4 ml); 2 N aqueous solution of sodium hydroxide (1.34 ml) was added thereto, followed by stirring at 80°C for 1.5 hours, after which 2 N aqueous solution of sodium hydroxide (0.3 ml)was added, followed by thermal refluxing for 2 hours. After cooling, the mixture was concentrated under reduced pressure; the residue was diluted with water and washed with ethyl acetate; the water layer was adjusted to pH 4 by the addition of 1 N hydrochloric acid, followed by extraction with ethyl acetate; the extract was dried over sodium sulfate. The extract was concentrated under reduced pressure and crystallized by the addition of ethyl acetate-ethyl ether-hexane (2:5:1), collected by filtration, washed with ethyl ether and dried to give the title compound (125 mg).
melting point: 118-121°C
elemental analysis for $C_{30}H_{34}N_5O_3Cl \cdot 1.5H_2O$
Calculated (%): C,62.65 ; H,6.48 ; N,12.18
Found (%): C,62.95 ; H,6.47 ; N,11.76

**Reference Example B89**

Production of 2-[6-[3-[4-(diphenylmethoxy)piperidino]-propoxy]-7-methylimidazo[1,2-b]pyridazin-2-yl]-2-methylpropionic acid

To N,N-dimethylformamide (10 ml), 60% oily sodium hydride (0.16 g) and 4-(diphenylmethoxy)-1-piperidinepropanol (1.30 g) were added, followed by stirring at room temperature under reduced pressure for 1 hour. While the reaction mixture was cooled with ice water, isopropyl 2-(6-chloro-7-methylimidazo[1,2-b]pyridazin-2-yl)-2-methylpropionate (1.31 g) was added thereto, followed by stirring at room temperature for 1.5 hours. To the reaction mixture, ice water was added, followed by extraction with ethyl acetate; the extract was washed with saline and dried over magnesium sulfate. The extract was concentrated under reduced pressure; the residue was subjected to silica gel column chromatography and eluted with hexane:ethyl acetate (1:5). The desired fractions were collected to give isopropyl 2-[6-[3-[4-(diphenylmethoxy)piperidino]propoxy]-7-methylimidazo[1,2-b]pyridazin-2-yl]-2-methylpropionate (582 mg) as an oil. This oil was dissolved in ethanol (4 ml); 1 N aqueous solution of sodium hydroxide (2 ml) was added thereto and reaction mixture was heated under reflux for 7 hours. After cooling, the reaction mixture was concentrated under reduced pressure. 1 N hydrochloric acid (2 ml) was added to the residue under ice-cooling, followed by extraction with ethyl acetate:tetrahydrofuran (1:1); the extract was washed with brine and dried over magnesium sulfate. The extract was concentrated under reduced pressure; the residue was crystallized by the addition of a small amount of water and ethyl ether, collected by filtration, washed with ethyl ether and dried to give the title compound (0.413 g).
melting point: 122°C
elemental analysis for $C_{32}H_{38}N_4O_4 \cdot 1.5H_2O$
Calculated (%): C,67.47 ; H,7.25 ; N,9.83
Found (%): C,67.61 ; H,7.13 ; N,9.68

**Reference Example B90**

Production of 2-[6-[3-[4-(diphenylmethoxy)piperidino]-propylamino]-7-methylimidazo[1,2-b]pyridazin-2-yl]-2-methylpropionic acid dihydrochloride

4-(Diphenylmethoxy)-1-piperidinepropanamine (1.40 g) and isopropyl 2-(6-chloro-7-methylimidazo[1,2-b]pyridazin-2-yl)-2-methylpropionate (0.636 g) were stirred at 190-200°C for 3 hours. After cooling, aqueous sodium hydrogen carbonate solution was added thereto, followed by extraction with ethyl acetate; the extract was washed with saline and dried over magnesium sulfate. The extract was concentrated under reduced pressure; the residue was subjected to silica gel column chromatography and eluted with ethyl acetate:methanol:triethylamine (185:15:2). The desired fractions were collected to give isopropyl 2-[6-[3-[4-(diphenylmethoxy)piperidino]propylamino]-7-methylimidazo[1,2-b] pyridazin-2-yl]-2-methylpropionate (0.737 g) as an oil. This oil was dissolved in ethanol (6 ml); 1 N aqueous solution of sodium hydroxide (3.15 ml) was added, followed by thermal refluxing for 7 hours. After cooling, the mixture was concentrated under reduced pressure; under ice cooling conditions, 1 N hydrochloric acid (1.89 ml) was added thereto; the residue was washed with ethyl acetate. To the water layer, 1 N hydrochloric acid (1.89 ml) was added and the mixture was saturated with sodium chloride, followed by extraction with ethyl acetate:tetrahydrofuran (1:1); the extract was washed with brine and dried over magnesium sulfate. The extract was concentrated under reduced pressure; 1 N hydrochloric acid (1.89 ml) was added to the residue, followed by concentration to dryness under reduced pressure; the residue was crystallized by the addition of ethyl ether, collected by filtration, washed with ethyl ether and dried to give the title compound (0.445 g).
melting point: 202°C (decomp.)
elemental analysis for $C_{32}H_{41}N_5O_3Cl_2 \cdot 0.5H_2O$
Calculated (%): C,61.63 ; H,6.79 ; N,11.23
Found (%) : C,61.66 ; H,6.83 ; N,11.11

**Reference Example B91**

Production of pivaloyloxymethyl 2-[6-[3-[4-(diphenylmethoxy)piperidino]propylamino]imidazo[1,2-b]pyridazin-2-yl]-2-methylpropionate difumarate

Ethyl 2-[6-[3-(4-diphenylmethoxy)piperidino]-propylamino]imidazo[1,2-b]pyridazin-2-yl]-2-methylpropionate difumarate (1.36 g) was suspended in ethyl acetate (20 ml) and washed with aqueous sodium hydrogen carbonate solution; the ethyl acetate layer was dried over magnesium sulfate. The extract was concentrated under reduced pressure; the residue was dissolved in ethanol (8 ml); 1 N aqueous solution of sodium hydroxide (4.3 ml) was added thereto, followed by stirring at room temperature for 40 hours. The mixture was concentrated under reduced pressure; while the residue was cooled with ice, 1 N hydrochloric acid (4.3 ml) and saline were added, followed by extraction with ethyl acetate:tetrahydrofuran (1:1); the extract was washed with brine and dried over magnesium sulfate. The extract was concentrated under reduced pressure; the residue was dissolved in N,N-dimethylformamide (5 ml); chloromethyl pivalate (0.374 ml) and potassium carbonate (0.357 g) were added thereto, followed by stirring at room temperature for 20 hours. Ice water was added thereto, followed by extraction with ethyl acetate; the extract was washed with saline and dried over magnesium sulfate. The extract was concentrated under reduced pressure; the residue was subjected to silica gel column chromatography and eluted with ethyl acetate:methanol:triethylamine (185:15:2). The desired fractions were collected, dissolved in ethyl acetate (10 ml); a solution of fumaric acid (227 mg) in methanol (5 ml) was added thereto, followed by concentration. The residue was recrystallized from ethyl acetate to give the title compound (0.772 g).
melting point: 164-167°C
elemental analysis for $C_{45}H_{55}N_5O_{13}$
Calculated (%): C,61.84 ; H,6.34 ; N,8.01
Found (%) : C,61.83 ; H,6.30 ; N,8.10

**Reference Example B92**

Production of ethyl 2-[6-[4-[4-(diphenylmethoxy)piperidino]-butyl]imidazo[1,2-b]pyridazin-2-yl]-2-methylpropionate dihydrochloride

step A: Production of ethyl 2-[6-(4-chlorobutyl)imidazo[1,2-b]pyridazin-2-yl]-2-methylpropionate

1-Chloro-4-iodobutane (5.0 g) was dissolved in toluene-N,N-dimethylacetamide (50-5 ml); copper-activated zinc (2.24 g) was added thereto, followed by stirring at 80°C in a nitrogen atmosphere for 3.5 hours. After cooling, ethyl 2-(6-chloroimidazo[1,2-b]pyridazin-2-yl)-2-methylpropionate (3.06 g) and dichlorobis(triphenylphosphine)palladium (II) (160 mg) were added thereto, followed by stirring at 80°C for 4 hours. After cooling, water and ethyl acetate were added thereto; the insoluble substances were filtered off through Celite; after the water layer was separated, the organic layer was washed with brine and dried over magnesium sulfate. The extract was concentrated under reduced pressure; the residue was subjected to silica gel column chromatography and eluted with hexane:ethyl acetate (1:1). The desired

fractions were collected, concentrated under reduced pressure and dried to give the title compound (1.74 g).
$^1$H-NMR($CDCl_3$)δ ppm:1.23(3H,t,J=6.8Hz), 1.68(6H,s), 1.80-2.00(4H,m), 2.84(2H,t,J=7.2Hz), 3.59(2H,t,J=6.0Hz), 4.17(2H,q,J=7.1Hz), 6.89(1H,d,J=9.5Hz), 7.80(1H,s), 7.82(1H,d,J=9.2Hz).

step B:

Ethyl 2-[6-(4-chlorobutyl)imidazo[1,2-b]pyridazin-2-yl]-2-methylpropionate (828 mg) was dissolved in 10 ml of acetonitrile (10 ml); 4-(diphenylmethoxy)piperidine (752 mg), potassium iodide (552 mg) and potassium carbonate (460 mg) were added thereto, followed by stirring at 60°C for 4 hours, after which the mixture was thermally refluxed for 18 hours. After cooling, ice water was added, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (100:5:2). The desired fractions were collected, concentrated under reduced pressure, dissolved in ethyl acetate (5 ml); 4 N solution of hydrogen chloride in ethyl acetate (1.01 ml) was added thereto, followed by concentration under reduced pressure. The residue was powdered from ethyl ether, collected by filtration and dried to give the title compound (1.18 g).
Amorphous
elemental analysis for $C_{34}H_{44}N_4O_3Cl_2 \cdot H_2O$
Calculated (%): C,63.25 ; H,7.18 ; N,8.68
Found (%): C,63.10 ; H,7.43 ; N,8.64

**Reference Example B93**

Production of sodium 2-[6-[4-[4-(diphenylmethoxy)piperidino]-butyl]imidazo[1,2-b]pyridazin-2-yl]-2-methylpropionate

Ethyl 2-[6-[4-[4-(diphenylmethoxy)-piperidino]butyl]imidazo[1,2-b]pyridazin-2-yl]-2-methylpropionate dihydrochloride (631 mg) was dissolved in ethanol (4 ml); 1 N aqueous solution of sodium hydroxide (5.5 ml) was added thereto, followed by thermal refluxing for 3 hours. After cooling, the mixture was concentrated under reduced pressure; the residue was diluted with water and adjusted to pH 5.5 by the addition of 1 N hydrochloric acid. Acetone was added to cause crystallization; the crystals precipitated were washed with water-acetone (2:1) and dried to give the title compound (345 mg).
melting point: 177-179°C
elemental analysis for $C_{32}H_{37}N_4O_3Na \cdot 1.75H_2O$
Calculated (%): C,66.25 ; H,7.04 ; N,9.66
Found (%): C,66.13 ; H,6.93 ; N,9.81

**Reference Example B94**

Ethyl 2-[6-[4-[4-(diphenylmethyl)-1-piperazinyl]butyl]-imidazo[1,2-b]pyridazin-2-yl]-2-methylpropionate

Ethyl 2-[6-(4-chlorobutyl)imidazo[1,2-b]pyridazin-2-yl]-2-methylpropionate (921 mg) was dissolved in N,N-dimethylformamide (10 ml); 1-(diphenylmethyl)piperazine (789 mg), potassium iodide (433 mg) and potassium carbonate (520 mg) were added thereto, followed by stirring at 60°C for 5 hours. After cooling, ethyl acetate was added thereto; the mixture was washed with brine and dried over magnesium sulfate. The extract was concentrated under reduced pressure; the residue was subjected to silica gel column chromatography and eluted with ethyl acetate:triethylamine (50:1). The desired fractions were collected, concentrated under reduced pressure and crystallized from ethyl ether:hexane (1:1), collected by filtration, washed with hexane and dried to give the title compound (554 mg).
melting point: 105-106°C
elemental analysis for $C_{33}H_{41}N_5O_2 \cdot 0.5H_2O$
Calculated (%): C,72.23 ; H,7.71 ; N,12.76
Found (%): C,72.48 ; H,7.73 ; N,12,95

**Reference Example B95**

Production of 2-[6-[4-[4-(diphenylmethyl)-1-piperazinyl]-butyl]imidazo[1,2-b]pyridazin-2-yl]-2-methylpropionic acid

Ethyl 2-[6-[4-[4-(diphenylmethyl)piperazino] butyl]imidazo[1,2-b]pyridazin-2-yl]-2-methylpropionate (482 mg) was dissolved in ethanol (2 ml); 1 N aqueous solution of sodium hydroxide (1.8 ml) was added thereto, followed by thermal refluxing for 1 hour. After cooling, the mixture was concentrated under reduced pressure; the residue was

diluted with water and adjusted to pH 5 by the addition of 1 N hydrochloric acid. Ethyl acetate was added to cause crystallization; the crystals precipitated were washed with water-acetone (2:1) and dried to give the title compound (386 mg).
melting point: 108-110°C
elemental analysis for $C_{31}H_{37}N_5O_2,H_2O$
Calculated (%): C,70.30 ; H,7.42 ; N,13.22
Found (%) : C,70.22 ; H,7.73 ; N,13.32

**Reference Example B96**

Production of isopropyl 1-[6-[3-[4-(diphenylmethoxy)-piperidino]propylamino]imidazo[1,2-b]pyridazin-2-yl] cyclopentanecarboxylate dihydrochloride

4-(Diphenylmethoxy)-1-piperidinepropanamine (1.67 g) and isopropyl 1-(6-chloroimidazo[1,2-b]pyridazin-2-yl)cyclopentanecarboxylate (793 mg) were stirred at 165°C for 5.5 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (100:5:2). The desired fractions were collected, concentrated under reduced pressure and dissolved in ethyl acetate (5 ml); 4 N solution of hydrogen chloride in ethyl acetate (0.84 ml) was added thereto, followed by concentration. The residue was powdered by the addition of ethyl ether; the resulting powder was collected by filtration, washed with ethyl ether and dried to give the title compound (999 mg).
Amorphous
elemental analysis for $C_{36}H_{47}N_5O_3Cl_2 \cdot 0.5H_2O,0.5Et_2O$
Calculated (%): C,63.85 ; H,7.47 ; N,9.80
Found (%): C,63.83 ; H,7.54 ; N,9.83

**Reference Example B97**

Production of 1-[6-[3-[4-(diphenylmethoxy)piperidino]-propylamino]imidazo[1,2-b]pyridazin-2-yl] cyclopentanecarboxylic acid

Isopropyl 1-[6-[3-[4-(diphenylmethoxy)piperidino]-propylamino]imidazo[1,2-b]pyridazin-2-yl]-cyclopentanecarboxylate dihydrochloride (598 mg) was dissolved in ethanol (3 ml); 2 N aqueous solution of sodium hydroxide (2.24 ml) was added thereto, followed by thermal refluxing for 7 hours. After cooling, the mixture was concentrated under reduced pressure; the residue was diluted with water, washed with ethyl acetate and adjusted to pH 4.5 by the addition of 1 N hydrochloric acid. The mixture was saturated with sodium chloride and extracted with ethyl acetate-tetrahydrofuran (1:2); the extract was dried over magnesium sulfate. The extract was concentrated under reduced pressure, powdered by the addition of ethyl acetate:ethyl ether (1:1), washed with ethyl ether and dried to give the title compound (349 mg).
Amorphous
elemental analysis for $C_{33}H_{39}N_5O_3 \cdot 3.0H_2O$
Calculated (%): C,65.22 ; H,7.46 ; N,11.52
Found (%): C,65.19 ; H,7.17 ; N,11.29

**Reference Example B98**

Production of 1-[6-[3-[4-(diphenylmethoxy)piperidino]-propoxy]imidazo[1,2-b]pyridazin-2-yl]cyclopropanecarboxylic acid

step A: Production of isopropyl 1-[6-[3-[4-(diphenylmethoxy)-piperidino]propoxy]imidazo[1,2-b]pyridazin-2-yl] cyclopropanecarboxylate

4-(Diphenylmethoxy)-1-piperidinepropanol (1.14 g) was dissolved in N,N-dimethylacetamide (15 ml); 60% oily sodium hydride (140 mg) was added thereto, followed by stirring at room temperature under reduced pressure for 30 minutes. To the reaction mixture, isopropyl 1-(6-chloroimidazo[1,2-b]pyridazin-2-yl)cyclopropanecarboxylate (980 mg) was added under ice cooling conditions, followed by stirring at the same temperature for 4 hours. Ice water was added thereto, and the mixture was saturated with sodium chloride and extracted with ethyl acetate:tetrahydrofuran

(1:1); the extract was dried over magnesium sulfate. The extract was concentrated under reduced pressure; the residue was subjected to silica gel column chromatography and eluted with ethyl acetate. The desired fractions were collected and concentrated under reduced pressure to give the title compound (496 mg).
$^1$H-NMR($CDCl_3$)δ ppm:1.25(3H,s),1.28(3H,s),1.40-2.25(12H,m),2.43-2.55(2H,m),2.70-2.88(2H,s),3.36-3.55(1H,m), 4.33(2H,t,J=6.3Hz),4.98-5.18(1H,m),5.52(1H,s),6.58(1H,d,J=9.8Hz),7.15-7.40(10H,m),7.64(1H,d,J=9.4Hz),8.03(1H, s).

step B:

Isopropyl 1-[6-[3-[4-(diphenylmethoxy)-piperidino]propoxy]imidazo[1,2-b]pyridazin-2-yl]cyclopropanecarboxylate (490 mg) was dissolved in ethanol (2 ml); 2 N aqueous solution of sodium hydroxide (0.86 ml) was added thereto, followed by thermal refluxing for 2 hours. After cooling, the mixture was concentrated under reduced pressure; the residue was diluted with water and adjusted to pH 5 by the addition of 1 N hydrochloric acid. The mixture was extracted with ethyl acetate:tetrahydrofuran (1:3); the extract was washed with saline and dried over magnesium sulfate. The extract was concentrated under reduced pressure, powdered by the addition of ethyl ether, washed with ethyl ether and dried to give the title compound (382 mg).
Amorphous
elemental analysis for $C_{31}H_{34}N_4O_4 \cdot 2.0H_2O$
Calculated (%): C,66.17 ; H,6.81 ; N,9.96
Found (%): C,66.27 ; H,7.00 ; N,9.75

**Reference Example B99**

Production of isopropyl 1-[6-[3-[4-(diphenylmethoxy)-piperidino]propylamino]imidazo[1,2-b]pyridazin-2-yl] cyclopropanecarboxylate dihydrochloride

4-(Diphenylmethoxy)-1-piperidinepropanamine (2.72 g) and isopropyl 1-(6-chloroimidazo[1,2-b]pyridazin-2-yl)cyclopropanecarboxylate (1.27 g) were stirred at 165°C for 4.5 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated under reduced pressure and dissolved in ethyl acetate (5 ml); 4 N solution of hydrogen chloride in ethyl acetate (0.72 ml) was added thereto, followed by concentration again. The residue was crystallized by the addition of ethyl acetate-acetone (2:1), collected by filtration, washed with ethyl acetate and dried to give the title compound (714 mg).
melting point: 206-208°C
elemental analysis for $C_{34}H_{43}N_5O_3Cl_2 \cdot 0.5H_2O$
Calculated (%): C,62.86 ; H,6.83 ; N,10.78
Found (%): C,63.10 ; H,6.88 ; N,10.83

**Reference Example B100**

Production of 1-[6-[3-[4-(diphenylmethoxy)piperidino]-propylamino]imidazo[1,2-b]pyridazin-2-yl] cyclopropanecarboxylic acid

Isopropyl 1-[6-[3-[4-(diphenylmethoxy)-piperidino]propylamino]imidazo[1,2-b]pyridazin-2-yl]cyclopropanecarboxylate dihydrochloride (554 mg) was dissolved in ethanol (3 ml); 2 N aqueous solution of sodium hydroxide (1.73 ml) was added thereto, followed by thermal refluxing for 1.5 hours. After cooling, the mixture was concentrated under reduced pressure; the residue was diluted with water, washed with ethyl acetate and adjusted to pH 5.5 by the addition of 1 N hydrochloric acid. The mixture was crystallized by the addition of acetone, washed with acetone and dried to give the title compound (321 mg).
melting point: 115-117°C
elemental analysis for $C_{31}H_{35}N_5O_3 \cdot H_2O$
Calculated (%): C,68.49 ; H,6.86 ; N,12.88
Found (%): C,68.24 ; H,6.89 ; N,12.93

**Reference Example B101**

Production of ethyl 2-[6-[3-[4-(diphenylmethoxy)piperidino]-propyl]imidazo[1,2-b]pyridazin-2-yl]-2-methylpropionate dihydrochloride

step A: Production of ethyl 2-[6-[3-(tetrahydropyranyl-2-oxy)propyl]imidazo[1,2-b]pyridazin-2-yl]-2-methylpropionate

2-(3-Iodopropoxy)tetrahydropyrane (10.6 g) was dissolved in toluene-N,N-dimethylacetamide (106-10.6 ml); copper-activated zinc (3.87 g) was added thereto, followed by stirring at 80°C in a nitrogen atmosphere for 3 hours. After cooling, ethyl 2-(6-chloroimidazo[1,2-b]pyridazin-2-yl)-2-methylpropionate (5.28 g) and dichlorobis(triphenylphosphine)palladium (II) (277 mg) were added thereto, followed by stirring at 80°C for 14 hours. After cooling, ice water and ethyl acetate were added thereto; the insoluble substances were filtered off through Celite; after the filtrate was extracted with ethyl acetate, the extract was washed with brine and dried over magnesium sulfate. The extract was concentrated under reduced pressure; the residue was subjected to silica gel column chromatography and eluted with hexane:ethyl acetate (1:1). The desired fractions were collected, concentrated under reduced pressure and dried to give the title compound (2.64 g).
$^1$H-NMR($CDCl_3$)δ ppm: 1.23(3H,t,J=7.0Hz), 1.68(6H,s), 1.40-1.95(6H,m), 1.98-2.15(2H,m), 2.87-2.96(2H,m), 3.40-3.56(2H,m), 3.75-3.94(2H,m), 4.17(2H,q,J=7.1Hz), 4.54-4.62(1H,broad t), 6.91(1H,d,J=9.2Hz), 7.79(1H,s), 7.80 (1H,d,J=9.0Hz).

step B: Production of ethyl 2-[6-(3-hydroxypropyl)-imidazo[1,2-b]pyridazin-2-yl]-2-methylpropionate

Ethyl 2-[6-[3-(tetrahydropyranyl-2-oxy)propyl]imidazo[1,2-b]pyridazin-2-yl]-2-methylpropionate (3.67 g) was dissolved in ethanol (38 ml); p-toluenesulfonic acid monohydrate (2.40 g) was added thereto, followed by stirring at room temperature for 24 hours. After the ethanol was distilled off under reduced pressure, the residue was diluted with water and extracted with ethyl acetate and tetrahydrofuran. The extract was washed with brine and dried over magnesium sulfate. The extract was concentrated under reduced pressure; the residue was subjected to silica gel column chromatography and eluted with ethyl acetate. The desired fractions were collected, concentrated under reduced pressure and dried to give the title compound (2.05 g).
$^1$H-NMR($CDCl_3$)δ ppm: 1.23(3H,t,J=7.2Hz), 1.68(6H,s), 1.95-2.10(2H,m), 2.94(2H,t,J=7.5Hz), 3.74(2H,q,J=7.1Hz), 4.17(2H,q,J=7.1Hz), 6.91(1H,d,J=9.0Hz), 7.80(1H,s), 7.82(1H,d,J=9.2Hz).

step C: Production of ethyl 2-[6-[3-(methanesulfonyloxy)-propyl]imidazo[1,2-b]pyridazin-2-yl]-2-methylpropionate

Ethyl 2-[6-(3-hydroxypropyl)imidazo[1,2-b]pyridazin-2-yl]-2-methylpropionate (2.04 g) was suspended in tetrahydrofuran (40 ml); under ice cooling conditions, N-ethyldiisopropylamine (2.41 ml) and methanesulfonyl chloride (0.83 ml) were added thereto, followed by stirring at room temperature for 15 minutes. Ice water was added thereto, followed by extraction with ethyl acetate; the extract was washed with brine and dried over magnesium sulfate. The extract was concentrated under reduced pressure and dried to give the title compound (2.78 g).
$^1$H-NMR($CDCl_3$)δ ppm: 1.23(3H,t,J=7.1Hz), 1.68(6H,s), 2.15-2.35(2H,m), 2.97(2H,t,J=7.5Hz), 3.03(3H,s), 4.17(2H,q, J=7.4Hz), 4.34(2H,t,J=6.2Hz), 6.89(1H,d,J=9.2Hz), 7.80(1H,s), 7.84(1H,d,J=10Hz).

step D:

Ethyl 2-[6-[3-(methanesulfonyloxy)propyl]imidazo[1,2-b]pyridazin-2-yl]-2-methylpropionate (1.32 g) was dissolved in N,N-dimethylformamide (15 ml); 4-(diphenylmethoxy)piperidine (1.15 g), potassium iodide (712 mg) and potassium carbonate (593 mg) were added thereto, followed by stirring at 60°C for 2 hours. After cooling, ice water was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1); the desired fractions were collected, concentrated under reduced pressure, dissolved in ethyl acetate (5 ml); 4 N solution of hydrogen chloride in ethyl acetate (1.6 ml) was added thereto, followed by concentration under reduced pressure. The residue was powdered from ethyl ether, collected by filtration and dried to give the title compound (1.55 g). Amorphous
elemental analysis for $C_{33}H_{42}N_4O_3Cl_2 \cdot 0.5H_2O$
Calculated (%): C,63.66 ; H,6.96 ; N,9.00
Found (%): C,63.61 ; H,6.94 ; N,9.07

**Reference Example B102**

Production of 2-[6-[3-[4-(diphenylmethoxy)piperidino]-propyl]imidazo[1,2-b]pyridazin-2-yl]-2-methylpropionic acid

Ethyl 2-[6-[3-[4-(diphenylmethoxy) piperidino]propyl]imidazo[1,2-b]pyridazin-2-yl]-2-methylpropionate dihydrochloride (905 mg) was dissolved in ethanol (6 ml); 1 N aqueous solution of sodium hydroxide (5.9 ml) was added thereto, followed by thermal refluxing for 2 hours. After cooling, the mixture was concentrated under reduced pressure; the residue was diluted with water and adjusted to pH 4.5 by the addition of 1 N hydrochloric acid. The mixture was crystallized by the addition of acetone, washed with acetone and dried to give the title compound (476 mg).
melting point: 195-205°C
elemental analysis for $C_{31}H_{36}N_4O_3 \cdot 0.3H_2O$
Calculated (%): C,71.87 ; H,7.12 ; N,10.81
Found (%): C,71.95 ; H,6.94 ; N,10.73

**Reference Example B103**

Production of ethyl 2-[6-[3-[4-(diphenylmethyl)-1-piperazinyl]propyl]imidazo[1,2-b]pyridazin-2-yl]-2-methylpropionate trihydrochloride

Ethyl 2-[6-[3-(methanesulfonyloxy)propyl]imidazo[1,2-b]pyridazin-2-yl]-2-methylpropionate (1.41 g) was dissolved in N,N-dimethylformamide (15 ml); 1-(diphenylmethyl)piperazine (1.16 g), potassium iodide (760 mg) and potassium carbonate (633 mg) were added thereto, followed by stirring at 60°C for 2 hours. Ice water was added thereto, followed by extraction with ethyl acetate; the extract was washed with brine and dried over magnesium sulfate. The extract was concentrated under reduced pressure; the residue was subjected to silica gel column chromatography and eluted with ethyl acetate:triethylamine (50:1). The desired fractions were collected, concentrated under reduced pressure, dissolved in ethyl acetate (5 ml); 4 N solution of hydrogen chloride in ethyl acetate (2.4 ml) was added thereto, followed by concentration again. The residue was recrystallized from acetone:ethyl acetate (1:1), collected by filtration and dried to give of the title compound (1.39 g).
melting point: 183-185°C
elemental analysis for $C_{32}H_{42}N_5O_2Cl_3 \cdot H_2O$
Calculated (%): C,58.85 ; H,6.79 ; N,10.72
Found (%): C,58.82 ; H,6.52 ; N,10.67

**Reference Example B104**

Production of 2-[6-[3-[4-(diphenylmethyl)-1-piperazinyl]-propyl]imidazo[1,2-b]pyridazin-2-yl]-2-methylpropionic acid

Ethyl 2-[6-[3-[4-(diphenylmethyl)1-piperazino]propyl]imidazo[1,2-b]pyridazin-2-yl]-2-methylpropionate trihydrochloride (1.08 g) was dissolved in ethanol (8 ml); 1 N aqueous solution of sodium hydroxide (8.5 ml) was added thereto, followed by thermal refluxing for 2 hours. After cooling, the mixture was concentrated under reduced pressure; the residue was diluted with water and adjusted to pH 4.5 by the addition of 1 N hydrochloric acid. The mixture was crystallized by the addition of acetone, washed with water-acetone (2:1) and dried to give the title compound (435 mg).
melting point: 176-178°C
elemental analysis for $C_{30}H_{35}N_5O_2 \cdot 0.5H_2O$
Calculated (%): C,71.12 ; H,7.16 ; N,13.82
Found (%): C,70.79 ; H,6.86 ; N,13.87

**Reference Example B105**

Production of ethyl 2-[6-[3-[4-(diphenylmethoxy)piperidino]-propylamino]-3-methylimidazo[1,2-b]pyridazin-2-yl]-2-methylpropionate dihydrochloride

4-(Diphenylmethoxy)-1-piperidinepropanamine (2.38 g) and ethyl 2-(6-chloro-3-methylimidazo[1,2-b]pyridazin-2-yl)-2-methylpropionate (1.03 g) were stirred at 160°C for 7.5 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated under reduced pressure and dissolved in ethyl acetate (5 ml); 4 N solution of hydrogen

chloride in ethyl acetate (0.96 ml) was added thereto, followed by concentration again. The residue was powdered by the addition of ethyl ether, collected by filtration and dried to give the title compound (666 mg).
Amorphous
elemental analysis for $C_{34}H_{45}N_5O_3Cl_2 \cdot 1.5H_2O$
Calculated (%): C,60.98 ; H,7.22 ; N,10.46
Found (%): C,60.70 ; H,6.95 ; N,10.34

**Reference Example B106**

Production of ethyl [6-[3-[4-(diphenylmethoxy)-piperidino]propylamino]-2-methylimidazo[1,2-b]pyridazin-3-yl] carboxylate

4-(Diphenylmethoxy)-1-piperidinepropanamine (1.98 g) and ethyl (6-chloro-2-methylimidazo[1,2-b]pyridazin-3-yl)carboxylate (1.46 g) were dissolved in 1-methyl-2-pyrrolidone (15 ml); N-ethyldiisopropylamine (1.05 ml) was added thereto, followed by stirring at 120°C for 40 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected and concentrated; ethyl ether-hexane (1:2) was added to the residue; the crystals precipitated were collected by filtration, washed with hexane and dried to give the title compound (412 mg).
melting point: 117-119°C
elemental analysis for $C_{31}H_{37}N_5O_3$
Calculated (%): C,70.56 ; H,7.07 ; N,13.27
Found (%): C,70.16 ; H,6.93 ; N,13.01

**Reference Example B107**

Production of [6-[3-[4-(diphenylmethoxy)piperidino]-propylamino]-2-methylimidazo[1,2-b]pyridazin-3-yl]carboxylic acid

Ethyl [6-[3-[4-(diphenylmethoxy)-piperidino]propylamino]-2-methylimidazo[1,2-b]pyridazin-3-yl]carboxylate (770 mg) was dissolved in ethanol (5 ml); 1 N aqueous solution of sodium hydroxide (3.2 ml) was added thereto, followed by stirring at room temperature for 3.5 hours. After cooling, the mixture was concentrated under reduced pressure; the residue was diluted with water, washed with ethyl acetate and adjusted to pH 4.5 by the addition of 1 N hydrochloric acid. The crystals precipitated were collected by filtration, washed with water and ethyl acetate and dried to give the title compound (265 mg).
melting point: 101-103°C
elemental analysis for $C_{29}H_{33}N_5O_3 \cdot 0.5H_2O$
Calculated (%): C,68.48 ; H,6.74 ; N,13.77
Found (%): C,68.63 ; H,6.77 ; N,13.91

**Reference Example B108**

Production of ethyl 2-[6-[3-[4-(diphenylmethylamino)piperidino]propylamino]imidazo[1,2-b]pyridazin-2-yl]-2-methylpropionate

4-(Diphenylmethylamino)-1-piperidinepropanamine (3.12 g) and ethyl 2-(6-chloroimidazo[1,2-b]pyridazin-2-yl)-2-methylpropionate (1.72 g) were stirred at 180°C for 3 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected and concentrated under reduced pressure; the residue was crystallized by the addition of ethyl ether:hexane (1:3), collected by filtration, washed with hexane and dried to give the title compound (1.83 g). melting point: 115-117°C
elemental analysis for $C_{33}H_{42}N_6O_2$
Calculated (%): C,71.45 ; H,7.63 ; N,15.15
Found (%): C,71.40 ; H,7.70 ; N,14.94

**Reference Example B109**

Production of 2-[6-[3-[4-(diphenylmethylamino)-piperidino]propylamino]imidazo[1,2-b]pyridazin-2-yl]-2-methylpropionic acid

Ethyl 2-[6-[3-[4-(diphenylmethylamino)-piperidino]propylamino]imidazo[1,2-b]pyridazin-2-yl]-2-methylpropionate (612 mg) was dissolved in ethanol (5 ml); 1 N aqueous solution of sodium hydroxide (2.2 ml) was added thereto, followed by thermal refluxing for 6 hours. After cooling, the mixture was concentrated under reduced pressure; the residue was diluted with water, washed with ethyl acetate and adjusted to pH 5 by the addition of 1 N hydrochloric acid. The mixture was saturated with sodium chloride and extracted with tetrahydrofuran; the extract was dried over magnesium sulfate. The extract was concentrated under reduced pressure, powdered by the addition of ethyl ether, collected by filtration and dried to give 503 mg of the title compound (503 mg).
Amorphous
elemental analysis for $C_{31}H_{38}N_6O_2{\cdot}2.7H_2O{\cdot}0.8Et_2O$
Calculated (%): C,64.93 ; H,7.87 ; N,13.28
Found (%): C,64.99 ; H,7.72 ; N,12.85

**Reference Example B110**

Production of ethyl 2-[6-[3-[4-(diphenylmethoxy)-piperidino]propylamino]imidazo[1,2-b]pyridazin-2-yl]-2-ethylbutyrate dihydrochloride

4-(Diphenylmethoxy)-1-piperidinepropanamine (3.03 g) and ethyl 2-(6-chloroimidazo[1,2-b]pyridazin-2-yl)-2-ethylbutyrate (1.38 g) were stirred at 160°C for 1.5 hours, then at 180°C for 2 hours. After the mixture was cooled to 90°C, ethanol and aqueous sodium hydrogen carbonate solution were added thereto, followed by extraction with ethyl' acetate; the extract was washed with brine and dried over magnesium sulfate. The extract was concentrated under reduced pressure; the residue was subjected to silica gel column chromatography and eluted with ethyl acetate: methanol:triethylamine (100:5:2). The desired fractions were collected, concentrated under reduced pressure and dissolved in ethyl acetate (5 ml); 4 N solution of hydrogen chloride in ethyl acetate (1.4 ml) was added thereto, followed by concentration again. The residue was powdered by the addition of ethyl ether and dried to give the title compound (893 mg).
Amorphous
elemental analysis for $C_{35}H_{47}N_5O_3Cl_2{\cdot}Et_2O$
Calculated (%): C,64.10 ; H,7.86 ; N,9.58
Found (%): C,63.78 ; H,7.57 ; N,9.96

**Reference Example B111**

Production of N-[3-chloro-6-[3-[4-(diphenylmethoxy)-piperidino]propylamino]imidazo[1,2-b]pyridazine-2-carbonyl] glycine ethyl ester

4-(Diphenylmethoxy)-1-piperidinepropanamine (0.649 g) and N-(3,6-dichloroimidazo[1,2-b]pyridazine-2-carbonyl)glycine ethyl ester (0.53 g) were dissolved in 1-methyl-2-pyrrolidone (7 ml); N-ethyldiisopropylamine (0.345 ml) was added thereto, followed by stirring in an oil bath (90-100°C) for 24 hours. After cooling, ice water was added thereto, followed by extraction with ethyl acetate; the extract was washed with saline and dried over magnesium sulfate. The extract was concentrated under reduced pressure; the residue was subjected to silica gel column chromatography and eluted with ethyl acetate:methanol:triethylamine (185:15:2). The desired fractions were collected and recrystallized from ethyl acetate to give the title compound (0.711 g).
melting point: 178-180°C
elemental analysis for $C_{32}H_{37}N_6O_4Cl$
Calculated (%): C,63.51 ; H,6.16 ; N,13.89
Found (%): C,63.56 ; H,6.21 ; N,13.78

**Reference Example B112**

Production of N-[6-[3-[4-(diphenylmethoxy)-piperidino]propylamino]imidazo[1,2-b]pyridazine-2-carbonyl]-β-alanine ethyl ester

4-(Diphenylmethoxy)-1-piperidinepropanamine (0.649 g) and N-(6-chloroimidazo[1,2-b]pyridazine-2-carbonyl)-β-alanine ethyl ester (0.594 g) were dissolved in 1-methyl-2-pyrrolidone (7 ml); N-ethyldiisopropylamine (0.345 ml) was added thereto, followed by stirring in an oil bath (90-100°C) for 24 hours. After cooling, ice water was added thereto, followed by extraction with ethyl acetate; the extract was washed with saline and dried over magnesium sulfate. The extract was concentrated under reduced pressure; the residue was subjected to silica gel column chromatography and eluted with ethyl acetate:methanol:triethylamine (370:30:4). The desired fractions were collected and recrystallized from ethyl ether to give the title compound (0.347 g).
melting point: 83-86°C
elemental analysis for $C_{33}H_{40}N_6O_4$
Calculated (%): C,67.79 ; H,6.90 ; N,14.37
Found (%): C,68.05 ; H,6.87 ; N,14.38

**Reference Example B113**

Production of sodium 2-[6-[3-[4-(diphenylmethoxy)-piperidino]propylamino]imidazo[1,2-b]pyridazin-2-yl]-2-methylpropionate

To a solution of 2-[6-[3-[4-(diphenylmethoxy)-piperidino]propylamino]imidazo[1,2-b]pyridazin-2-yl]-2-methylpropionic acid (528 mg) in methanol (2 ml), a 2 N aqueous solution of sodium hydroxide (0.47 ml) was added, followed by stirring at room temperature for 5 minutes. This solution was diluted with 2-propanol and concentrated under reduced pressure; the residue was dissolved in 2-propanol and again concentrated under reduced pressure. To this residue, 2-propanol and ethyl ether were added; the resulting powder was collected by filtration to give the title compound (474 mg).
Amorphous
elemental analysis for $C_{31}H_{36}N_5O_3Na \cdot 0.5H_2O$
Calculated (%): C,66.65 ; H,6.68 ; N,12.54
Found (%): C,66.45 ; H,6.54 ; N,12.53

**Reference Example B114**

Production of 6-[5-[4-(diphenylmethoxy)piperidino]-pentylamino][1,2,4]triazolo[1,5-b]pyridazine

4-(Diphenylmethoxy)-1-piperidinepentanamine (0.705 g) and 6-chloro[1,2,4]triazolo[1,5-b]pyridazine (0.309 g) were stirred at 135-140°C for 1.5 hours. After cooling, aqueous sodium hydrogen carbonate solution was added thereto, followed by extraction with ethyl acetate; the extract was washed with saline and dried over sodium sulfate. The extract was concentrated under reduced pressure; the residue was subjected to silica gel column chromatography and eluted with ethyl acetate:methanol:triethylamine (95:5:1). The desired fractions were collected, recrystallized from ethyl ether and dried to give the title compound (0.629 g).
melting point: 96-98°C
elemental analysis for $C_{28}H_{34}N_6O \cdot H_2O$
Calculated (%): C,70.12 ; H,7.36 ; N,17.52
Found (%): C,70.29 ; H,7.19 ; N,17.62

**Reference Example B115**

Production of 1-[4-(diphenylmethoxy)piperidino]-3-[([1,2,4]triazolo[1,5-b]pyridazin-6-yl)amino]-2-propanol

1-Amino-3-[4-(diphenylmethoxy)piperidino]-2-propanol (0.675 g) and 6-chloro[1,2,4]triazolo [1,5-b]pyridazine (0.335 g) were stirred at 135-140°C for 3 hours. After cooling, aqueous sodium hydrogen carbonate solution was added thereto, followed by extraction with ethyl acetate-tetrahydrofuran (2:1); the extract was washed with saline and dried over sodium sulfate. The extract was concentrated under reduced pressure; the residue was subjected to silica gel column chromatography and eluted with ethyl acetate:methanol:triethylamine (90:10:1). The desired fractions were collected, recrystallized from ethyl acetate and dried to give the title compound (0.509 g).

melting point: 82-87°C
elemental analysis for $C_{26}H_{30}N_6O_2 \cdot H_2O$
Calculated (%): C,65.53 ; H,6.77 ; N,17.63
Found (%): C,65.36 ; H,6.50 ; N,17.25

**Reference Example B116**

Production of tert-butyl [6-[3-[4-(diphenylmethoxy)-piperidino]propylamino][1,2,4]triazolo[2,5-b]pyridazin-2-yl] carboxylate

4-(Diphenylmethoxy)-1-piperidinepropanamine (563 mg) and tert-butyl (6-chloro[1,2,4]triazolo[1,5-b]pyridazin-2-yl)carboxylate (442 mg) were dissolved in pyridine (5 ml), followed by stirring at 80°C for 13.5 hours. After cooling, water was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected and concentrated under reduced pressure; the residue was crystallized by the addition of ethyl acetate, collected by filtration, washed with ethyl ether and dried to give the title compound (365 mg).
melting point: 133-135°C
elemental analysis for $C_{31}H_{38}N_6O_3$
Calculated (%): C,68.61 ; H,7.06 ; N,15.49
Found (%): C,68.18 ; H.6.81 ; N,15.46

**Reference Example B117**

Production of [6-[3-[4-(diphenylmethoxy)piperidino]-propylamino][1,2,4]triazolo[1,5-b]pyridazin-2-yl]carboxylic acid

4-(Diphenylmethoxy)-1-piperidinepropanamine (2.33 g) and (6-chloro[1,2,4]triazolo[1,5-b]pyridazin-2-yl)carboxylic acid (714 mg) were stirred at 175°C for 30 minutes. After cooling, the reaction mixture was crystallized by the addition of water-ethyl acetate-ethanol (2:2:1), collected by filtration, washed with water-ethyl acetate-ethyl ether (2:1:2) and dried to give the title compound (598 mg). melting point: 135-138°C
elemental analysis for $C_{27}H_{30}N_6O_3 \cdot 0.5H_2O$
Calculated (%): C,65.44 ; H,6.31 ; N,16.96
Found (%): C,65.76 ; H,6.13 ; N,16.97

**Reference Example B118**

Production of methyl [6-[3-[4-(diphenylmethoxy)-piperidino]propylamino][1,2,4]triazolo[1,5-b]pyridazin-7-yl] carboxylate

4-(Diphenylmethoxy)-1-piperidinepropanamine (1.42 g) and methyl (6-chloro[1,2,4]triazolo[1,5-b]pyridazin-7-yl)carboxylate (929 mg) were dissolved in N,N-dimethylformamide (20 ml); N-ethyldiisopropylamine (1.51 ml) was added thereto, followed by stirring at 70°C for 6 hours. After cooling, water was added thereto; the mixture was saturated with sodium chloride and extracted with ethyl acetate:tetrahydrofuran (1:1); the extract was dried over magnesium sulfate. The extract was concentrated under reduced pressure; the residue was subjected to silica gel column chromatography and eluted with ethyl acetate:methanol:triethylamine (50:5:1). The desired fractions were collected and concentrated; the residue was crystallized by the addition of ethyl acetate:ethyl ether:hexane (1:2:1), collected by filtration, washed with hexane and dried to give the title compound (905 mg).
melting point: 120-122°C
elemental analysis for $C_{28}H_{32}N_6O_3$
Calculated (%): C,67.18 ; H,6.44 ; N,16.79
Found (%): C,67.11 ; H,6.54 ; N,16.87

**Reference Example B119**

Production of [6-[3-[4-(diphenylmethoxy)piperidino]-propylamino][1,2,4]triazolo[1,5-b]pyridazin-7-yl]carboxylic acid

Methyl [6-[3-[4-(diphenylmethoxy)piperidino]-propylamino][1,2,4]triazolo[1,5-b]pyridazin-7-yl]carboxylate (1.58 g) was dissolved in ethanol (10 ml); 1 N aqueous solution of sodium hydroxide (8.0 ml) was added thereto,

followed by stirring at room temperature for 1.5 hours. After cooling, the mixture was concentrated under reduced pressure; the residue was diluted with water and washed with ethyl acetate; 1 N hydrochloric acid was added to adjust to pH 4.5. The mixture was saturated with sodium chloride and extracted with tetrahydrofuran; the extract was dried over magnesium sulfate. The crystals obtained by concentration under reduced pressure were washed with ethyl ether, collected by filtration and dried to give the title compound (788 mg).
melting point: 207-209°C
elemental analysis for $C_{27}H_{30}N_6O_3 \cdot 0.5H_2O$
Calculated (%): C,65.44 ; H,6.30 ; N,16.96
Found (%): C,65.17 ; H,6.19 ; N,16.90

**Reference Example B120**

Production of N-[6-[3-[4-(diphenylmethoxy)piperidino]-propylamino][1,2,4]triazolo[1,5-b]pyridazine-2-carbonyl]glycine ethyl ester

4-(Diphenylmethoxy)-1-piperidinepropanamine (1.41 g) and N-(6-chloro[1,2,4]triazolo[1,5-b]pyridazine-2-carbonyl)glycine ethyl ester (1.23 g) were dissolved in N,N-dimethylpropylamine (17 ml); N-ethyldiisopropylamine (1.50 ml) was added thereto, followed by stirring at room temperature for 28 hours, then at 60°C for 19 hours. After cooling, aqueous sodium hydrogen carbonate solution was added thereto, followed by extraction with ethyl acetate: tetrahydrofuran (1:1); the extract was dried over magnesium sulfate. The extract was concentrated under reduced pressure; the residue was subjected to silica gel column chromatography and eluted with ethyl acetate:methanol: triethylamine (50:5:1). The desired fractions were collected, concentrated, recrystallized by the addition of tetrahydrofuran, collected by filtration, washed with ethyl ether and dried to give the title compound (987 mg).
melting point: 175-177°C
elemental analysis for $C_{31}H_{37}N_7O_4$
Calculated (%): C,64.12 ; H,6.60 ; N,16.88
Found (%): C,63.99 ; H,6.52 ; N,16.85

**Reference Example B121**

Production of N-[6-[3-[4-(diphenylmethoxy)piperidino]-propylamino][1,2,4]triazolo[1,5-b]pyridazine-2-carbonyl]-2,2-dimethylglycine ethyl ester fumarate

4-(Diphenylmethoxy)-1-piperidinepropanamine (1.56 g) and N-(6-chloro[1,2,4]triazolo[1,5-b]pyridazine-2-carbonyl)-2,2-dimethylglycine ethyl ester (1.50 g) were dissolved in N,N-dimethylformamide (20 ml); N-ethyldiisopropylamine (1.65 ml) was added, followed by stirring at 70°C for 16 hours. After cooling, aqueous sodium hydrogen carbonate solution was added thereto, followed by extraction with ethyl acetate; the extract was washed with brine and dried over magnesium sulfate. The extract was concentrated under reduced pressure; the residue was subjected to silica gel column chromatography and eluted with ethyl acetate:methanol:triethylamine (50:5:1). The desired fractions were collected and concentrated; the oil obtained (880 mg) was dissolved in ethanol (5 ml), followed by the addition of fumaric acid (170 mg) and concentration; the resulting residue was powdered by the addition of ethyl ether, washed with ethyl ether, collected by filtration and dried to give the title compound (931 mg).
Amorphous
elemental analysis for $C_{37}H_{45}N_7O_8 \cdot H_2O \cdot 0.5Et_2O$
Calculated (%): C,60.76 ; H,6.80 ; N,12.72
Found (%): C,60.71 ; H,6.85 ; N,12.34

**Reference Example B122**

Production of isopropyl [6-[3-[4-(diphenylmethoxy)-piperidino]propoxy][1,2,4]triazolo[1,5-b]pyridazin-2-yl]carboxylate

4-(Diphenylmethoxy)-1-piperidinepropanol (653 mg) was dissolved in N,N-dimethylformamide (10 ml); 60% oily sodium hydride (88 mg) was added thereto, followed by stirring at room temperature under reduced pressure for 1.5 hours. To the reaction mixture, isopropyl (6-chloro[1,2,4]triazolo[1,5-b]pyridazin-2-yl)carboxylate (483 mg) was added under ice cooling conditions, followed by stirring at the same temperature for 3.5 hours. Ice water was added thereto, followed by extraction with ethyl acetate:tetrahydrofuran (1:1); the extract was washed with brine and dried over magnesium sulfate. The extract was concentrated under reduced pressure; the residue was subjected to silica gel column chromatography and eluted with ethyl acetate:methanol (10:1). The desired fractions were collected and concentrated;

the crystals precipitated were washed with ethyl ether, collected by filtration and dried to give the title compound (462 mg).
melting point: 126-127°C
elemental analysis for $C_{30}H_{35}N_5O_4$
Calculated (%): C,68.03 ; H,6.66 ; N,13.22
Found (%): C.68.01 ; H,6.79 ; N,13.42

**Reference Example B123**

Production of [6-[3-[4-(diphenylmethoxy)piperidino]-propoxy][1,2,4]triazolo[1,5-b]pyridazin-2-yl]carboxylic acid

Isopropyl [6-[3-[4-(diphenylmethoxy)piperidino]-propoxy][1,2,4]triazolo[1,5-b]pyridazin-2-yl]carboxylate (1.85 g) was dissolved in tetrahydrofuran (18 ml); 1 N aqueous solution of sodium hydroxide (3.8 ml) was added thereto, followed by stirring at room temperature for 3.5 hours. After cooling, the mixture was concentrated under reduced pressure; the residue was diluted with water and washed with ethyl acetate; 1 N hydrochloric acid was added thereto to adjust pH 4.5. The mixture was crystallized by the addition of ethanol:acetone (1:2), collected by filtration, washed with water and ethyl acetate and dried to give the title compound (1.33 g).
melting point: 173-177°C
elemental analysis for $C_{27}H_{29}N_5O_4 \cdot 2.5H_2O$
Calculated (%): C,60.89 ; H,6.43 ; N,13.15
Found (%): C,60.86 ; H,6.21 ; N,13.06

**Reference Example B124**

Production of N-[6-[3-[4-(diphenylmethoxy)piperidino]-propylamino][1,2,4]triazolo[1,5-b]pyridazine-2-carbonyl]-2,2-dimethylglycine

N-[6-[3-[4-(Diphenylmethoxy)piperidino]-propylamino][1,2,4]triazolo[1,5-b]pyridazine-2-carbonyl]-2,2-dimethylglycine ethyl ester (1.71 g) was dissolved in ethanol (6 ml); 1 N aqueous solution of sodium hydroxide (4.5 ml) was added thereto, followed by stirring at room temperature for 2 hours. Under ice cooling conditions, 1 N hydrochloric acid was added thereto to adjust the mixture to pH 5. The crystals obtained were collected by filtration, washed with water and ethyl acetate and dried to give the title compound (1.24 g). melting point: 247-249°C
elemental analysis for $C_{31}H_{37}N_7O_4 \cdot H_2O$
Calculated (%): C,63.14 ; H,6.67 ; N,16.63
Found (%): C,63.09 ; H,6.81 ; N,16.70

**Reference Example B125**

Production of N-[6-[3-[4-(diphenylmethoxy)piperidino]-propylamino][1,2,4]triazolo[1,5-b]pyridazine-2-carbonyl]glycine

N-[6-[3-[4-(Diphenylmethoxy)piperidino]-propylamino][1,2,4]triazolo[1,5-b]pyridazine-2-carbonyl]glycine ethyl ester (928 mg) was dissolved in ethanol (7 ml); 1 N aqueous solution of sodium hydroxide (2.2 ml) was added thereto, followed by stirring at room temperature for 1.5 hours. The mixture was concentrated under reduced pressure; the residue was diluted with water; 1 N hydrochloric acid was added to adjust the mixture to pH 4.5. The crystals obtained were collected by filtration, washed with water, acetone and ethyl acetate and dried to give the title compound (443 mg).
melting point: 256-258°C
elemental analysis for $C_{29}H_{33}N_7O_4 \cdot 1.5H_2O$
Calculated (%): C,61.04 ; H,6.36 ; N,17.18
Found (%): C,61.29 ; H,6.28 ; N,17.35

**Reference Example B126**

Production of N-[6-[3-[4-(diphenylmethoxy)piperidino]-propoxy][1,2,4]triazolo[1,5-b]pyridazine-2-carbonyl]-2,2-dimethylglycine ethyl ester 1.5 fumarate

[6-[3-[4-(Diphenylmethoxy)piperidino]-propoxy][1,2,4]triazolo[1,5-b]pyridazin-2-yl]carboxylic acid (986 mg) and N-ethyldiisopropylamine (0.38 ml) were suspended in N,N-dimethylformamide (10 ml); N,N'-carbonyldiimidazole

(361 mg) was added thereto, followed by stirring at room temperature for 3 hours. After ethyl 2-aminoisobutyrate hydrochloride (372 mg) was added thereto, the mixture was stirred at room temperature for 43 hours, then at 60°C for 5 hours. Ice water was added thereto, followed by extraction with ethyl acetate:tetrahydrofuran (1:1); the extract was washed with brine and dried over magnesium sulfate. The extract was concentrated under reduced pressure; the residue was subjected to silica gel column chromatography and eluted with ethyl acetate:methanol:triethylamine (50:1:1). The desired fractions were collected and concentrated under reduced pressure, after which it was dissolved in ethanol (5 ml); fumaric acid (139 mg) was added thereto, followed by concentration. Ethanol:ethyl acetate (1:3) was added to cause crystallization; the crystals precipitated were washed with ethyl ether, collected by filtration and dried to give the title compound (581 mg).
melting point: 127-130°C
elemental analysis for $C_{39}H_{46}N_6O_{11}$
Calculated (%): C,60.45 ; H,5.98 ; N,10.85
Found (%): C,60.06 ; H,5.91 ; N,10.80

**Reference Example B127**

Production of N-[6-[3-[4-(diphenylmethoxy)piperidino]-propylamino]-3-methylimidazo[1,2-b]pyridazine-2-carbonyl] glycine ethyl ester

4-(diphenylmethoxy)-1-piperidinepropanamine (1.17 g) and N-(6-chloro-3-methylimidazo[1,2-b]pyridazine-2-carbonyl)glycine ethyl ester (0.891 g) were dissolved in 1-methyl-2-pyrrolidone (10 ml); N-ethyldiisopropylamine (0.517 ml) was added thereto, followed by stirring in an oil bath (90-100°C) for 15 hours. After cooling, ice water was added thereto, followed by extraction with ethyl acetate; the extract was washed with saline and dried over magnesium sulfate. The extract was concentrated under reduced pressure; the residue was subjected to silica gel column chromatography and eluted with ethyl acetate:methanol:triethylamine (90:10:1). The desired fractions were collected and recrystallized from ethyl acetate:ethyl ether (1:1) to give the title compound (0.629 g).
melting point: 158-160°C
elemental analysis for $C_{33}H_{40}N_6O_4$
Calculated (%): C,67.79 ; H,6.90 ; N,14.37
Found (%): C,67.52 ; H,6.92 ; N,14.13

**Reference Example B128**

Production of 6-[3-[4-(diphenylmethoxy)piperidino]-propylamino]-2-isopropylimidazo[1,2-b]pyridazine hydrochloride

A mixture of 4-(diphenylmethoxy)-1-piperidinepropanamine (2.60 g), 6-chloro-2-isopropylimidazo[1,2-b]pyridazine (0.783 g) and potassium iodide (0.133 g) was stirred at 190°C in a nitrogen atmosphere for 5 hours. The reaction mixture was cooled to 100°C; ethanol (2 ml) was added drop by drop, after which the mixture was cooled to room temperature. To this mixture, an aqueous solution of sodium hydrogen carbonate (0.40 g) was added, followed by 2 extractions with ethyl acetate. The organic layers combined were washed with water, filtered and concentrated under reduced pressure. The residue was subjected to silica gel column chromatography (ethyl acetate, then ethyl acetate:methanol:triethylamine 500:25:1 and then 50:5:1). The desired fractions were collected, concentrated and dissolved in methanol; a 10% solution of hydrogen chloride/methanol (3 ml) was added, followed by concentration under reduced pressure, to give the title compound (1.13 g).
Amorphous
elemental analysis for $C_{30}H_{38}N_5OCl \cdot 0.75H_2O$
Calculated (%): C,67.52 ; H,7.46
Found (%): C,67.32 ; H,7.42

**Reference Example B129**

Production of ethyl 2-[6-[3-[4-[phenyl(2-thienyl)-methylamino]piperidino]propylamino]imidazo[1,2-b]pyridazin-2-yl]-2-methylpropionate trihydrochloride

4-[Phenyl(2-thienyl)methylamino]-1-piperidinepropanamine (1.44 g) and ethyl 2-(6-chloroimidazo[1,2-b]pyridazin-2-yl)-2-methylpropionate (585 mg) were dissolved in 1-methyl-2-pyrrolidone (3 ml), followed by stirring in an oil bath (170°C) for 4 hours. After cooling, ethanol and saturated aqueous sodium hydrogen carbonate solution were added thereto, followed by extraction with ethyl acetate; the extract was washed with brine and dried over magnesium

sulfate. The extract was concentrated under reduced pressure; the residue was subjected to silica gel column chromatography and eluted with ethyl acetate: methanol:triethylamine (50:5:1). The desired fractions were collected, concentrated, dissolved in ethyl acetate (5 ml); 4N-hydrogenchloride in ethyl acetate (0.86 ml) was added thereto, followed by concentration. Ethanol-ethyl acetate (1:4) was added to the residue; the crystals precipitated were collected by filtration and dried to give the title compound (609 mg).
melting point: 175-178°C
elemental analysis for $C_{31}H_{43}N_6O_2SCl_3 \cdot H_2O$
Calculated (%): C,54.11 ; H,6.59 ; N,12.21
Found (%): C,54.17 ; H,6.49 ; N,12.08

**Reference Example B130**

Production of ethyl 2-[6-[3-[4-(hydroxydiphenylmethyl)-piperidino]propylamino]imidazo[1,2-b]pyridazin-2-yl]-2-methylpropionate dihydrochloride

4-(Hydroxydiphenylmethyl)-1-piperidinepropanamine (427 mg) and ethyl 2-(6-chloroimidazo[1,2-b]pyridazin-2-yl)-2-methylpropionate (235 mg) were stirred at 160°C for 3.5 hours. After cooling, ethanol and saturated aqueous sodium hydrogen carbonate solution were added thereto, followed by extraction with ethyl acetate; the extract was washed with brine and dried over magnesium sulfate. The extract was concentrated under reduced pressure; the residue was subjected to silica gel column chromatography and eluted with ethyl acetate:methnol:triethylamine (50:5:1). The desired fractions were collected, concentrated, dissolved in ethyl acetate (5 ml); 4N-hydrogenchloride in ethyl acetate (0.23 ml) was added thereto, followed by concentration. Ethyl ether was added to the residue; the powder was collected by filtration and dried to give the title compound (216 mg).
Amorphous
elemental analysis for $C_{33}H_{43}N_5O_3Cl_2 \cdot H_2O \cdot 0.5Et_2O$
Calculated (%): C,61.49 ; H,7.37 ; N,10.24
Found (%): C,61.47 ; H,7.36 ; N,9.87

**Reference Example B131**

Production of ethyl 2-[6-[3-[3-(diphenylmethoxy)pyrrolidino]-propylamino]imidazo[1,2-b]pyridazin-2-yl]-2-methylpropionate dihydrochloride

3-(Diphenylmethoxy)-1-pyrrolidinepropanamine (1.53 g) and ethyl 2-(6-chloroimidazo[1,2-b]pyridazin-2-yl)-2-methylpropionate (660 mg) were dissolved in 1-methyl-2-pyrrolidone (3 ml), followed by stirring at 170°C for 8 hours. After cooling, saturated aqueous sodium hydrogen carbonate solution was added thereto, followed by extraction with ethyl acetate; the extract was washed with water and brine and dried over magnesium sulfate. The solution was concentrated under reduced pressure; the residue was subjected to silica gel column chromatography and eluted with ethyl acetate:methanol:triethylamine (50:5:1). The desired fractions were collected, concentrated, dissolved in ethyl acetate (5 ml); 4N-hydrogenchloride in ethyl acetate (0.81 ml) was added thereto, followed by concentration. Ethyl ether was added to the residue; the powder was collected by filtration and dried to give the title compound (877 mg).
Amorphous
elemental analysis for $C_{32}H_{41}N_5O_3Cl_2 \cdot H_2O$
Calculated (%): C,60.75 ; H,6.85 ; N,11.07
Found (%): C,60.50 ; H,6.55 ; N,10.81

**Reference Example B132**

Production of ethyl 2-[6-[3-[4-(10,11-dihydro-5H-dibenzo[a,d]cyclohepten-5-oxy)piperidino]propylamino]-imidazo [1,2-b]pyridazin-2-yl]-2-methylpropionate

4-(10,11-Dihydro-5H-dibenzo[a,d]cyclohepten-5-oxy)-1-piperidinepropanamine (1.69 g) and ethyl 2-(6-chloroimidazo[1,2-b]pyridazin-2-yl)-2-methylpropionate (645 mg) were dissolved in 1-methyl-2-pyrrolidone (3 ml), followed by stirring at 170°C for 7 hours. After cooling, saturated aqueous sodium hydrogen carbonate solution was added thereto, followed by extraction with ethyl acetate; the extract was washed with water and brine and dried over magnesium sulfate. The extract was concentrated under reduced pressure; the residue was subjected to silica gel column chromatography and eluted with ethyl acetate:methanol:triethylamine (50:5:1). The desired fractions were collected, concentrated, the residue was subjected to silica gel column chromatography again and eluted with methylene chloride:

methanol:triethylamine (100:1:2). The desired fractions were collected and concentrated to give the title compound (340 mg).
$^1$H-NMR(CDCl$_3$)δ ppm: 1.21(3H,t,J=7.0Hz), 1.52-2.20(8H,m), 1.64(6H,s), 2.43-2.60(2H,m), 2.70-2.92(2H,m), 2.95-3.10(2H,m), 3.28-3.62(5H,m), 6.14(2H,d,J=7.0Hz), 6.29(1H,d,J=9.4Hz), 6.40-6.50(1H,brs), 7.05-7.22(6H,m), 7.33-7.43(2H,m), 7.54(1H,d,J=9.4Hz).

**Reference Example A1**

Production of 4-(diphenylmethoxy)-1-piperidinepropanol

4-(Diphenylmethoxy)piperidine (2.67 g) was dissolved in N,N-dimethylformamide (20 ml); 3-bromopropanol (1.09 ml) and potassium carbonate (1.66 g) were added thereto, followed by stirring at room temperature for 40 hours. Ice water was added thereto, followed by extraction with ethyl acetate; the extract was washed with saline and dried over magnesium sulfate. After the extract was concentrated under reduced pressure, the residue was subjected to silica gel column chromatography and eluted with ethyl acetate:methanol:triethylamine (90:10:1). The desired fractions were collected and concentrated to give the title compound (2.32 g).
$^1$H-NMR(CDCl$_3$)δ ppm: 1.5-2.4(10H,m), 2.58(2H,t,J=5Hz), 3.3-3.6(1H,m), 3.78(2H,t,J=5Hz), 5.50(1H,s), 7.1-7.5(10H, m).

**Reference Example A2**

Production of 4-(diphenylmethoxy)-1-piperidinebutanol

4-(Diphenylmethoxy)piperidine (1.05 g) was dissolved in N,N-dimethylformamide (10 ml); 4-bromobutyl acetate (0.57 ml) and potassium carbonate (652 mg) were added thereto, followed by stirring at 50°C for 3 hours. Ice water was added thereto, followed by extraction with ethyl ether; the extract was washed with saline and dried over magnesium sulfate. After the extract was concentrated under reduced pressure, the residue was dissolved in ethanol (15 ml); 1 N aqueous solution of sodium hydroxide (8 ml) was added thereto, followed by stirring at room temperature. After the mixture was concentrated under reduced pressure, the residue was neutralized with 1 N hydrochloric acid, followed by extraction with ethyl acetate; the extract was washed with saline and dried over magnesium sulfate. After the extract was concentrated under reduced pressure, the crystals precipitate were collected, washed with ethyl ether and dried to give the title compound (1.21 g).
$^1$H-NMR(CDCl$_3$)δ ppm: 2.95(2H,t,J=5Hz), 1.6-3.4(13H,m), 3.74(2H,t,J=5Hz), 5.43(1H,s), 7.2-7.5(10H,m).

**Reference Example A3**

Production of 4-(diphenylmethoxy)-1-piperidinehexanol

4-(Diphenylmethoxy)piperidine (1.00 g) was dissolved in N,N-dimethylformamide (10 ml); 6-bromo-hexanol (0.49 ml), sodium iodide (0.56 g) and potassium carbonate (0.62 g) were added thereto, followed by stirring at 100°C for 1 hour. Ice water was added thereto, followed by extraction with ethyl acetate; the extract was washed with saline and dried over magnesium sulfate. After the extract was concentrated under reduced pressure, the residue was subjected to silica gel column chromatography and eluted with ethyl acetate:methanol:triethylamine (90:10:1). The desired fractions were collected and concentrated to give the title compound (1.24 g).
$^1$H-NMR(CDCl$_3$)δ ppm: 1.2-2.0(12H,m), 2.0-2.2(2H,m), 2.30(2H,t,J=8Hz), 2.6-2.9(2H,m), 3.3-3.6(1H,m), 3.63(2H,t, J=6Hz), 5.52(1H,s), 7.1-7.5(10H,m).

**Reference Example A4**

Production of 2-[2-[4-(diphenylmethoxy)piperidino]-ethoxy]ethanol

4-(Diphenylmethoxy)piperidine (1.30 g) was dissolved in N,N-dimethylformamide (10 ml); 2-(2-chloroethoxy) ethanol (0.52 ml), sodium iodide (0.73 g) and potassium carbonate (0.81 g) were added thereto, followed by stirring at 100°C for 1 hour. Ice water was added thereto, followed by extraction with ethyl acetate; the extract was washed with saline and dried over magnesium sulfate. After the extract was concentrated under reduced pressure, the residue was subjected to silica gel column chromatography and eluted with ethyl acetate:methanol:triethylamine (90:10:1). The desired fractions were collected and concentrated to give the title compound (1.47 g).
$^1$H-NMR(CDCl$_3$)δ ppm: 1.6-2.4(6H,m), 2.54(2H,t,J=6Hz), 2.6-3.0(2H,m), 3.3-3.5(1H,m), 3.5-3.8(6H,m), 5.50(1H,s),

7.1-7.5(10H,m).

**Reference Example A5**

Production of 2-[2-[4-(diphenylmethyl)-1-piperazinyl]-ethoxy]ethanol

1-(Diphenylmethyl)piperazine (1.00 g) was dissolved in N,N-dimethylformamide (10 ml); 2- (2-chloroethoxy) ethanol . (0.42 ml), sodium iodide (0.59 g) and potassium carbonate (0.66 g) were added thereto, followed by stirring at 100°C for 1 hour. Ice water was added thereto, followed by extraction with ethyl acetate; the extract was washed with saline and dried over magnesium sulfate. After the extract was concentrated under reduced pressure, the residue was subjected to silica gel column chromatography and eluted with dichloromethane:ethyl acetate:methanol (10:10: 1). The desired fractions were collected and concentrated to give the title compound (1.47 g).
$^1$H-NMR($CDCl_3$)δ ppm: 2.3-2.8(8H,m), 2.57(2H,t,J=6Hz), 3.5-3.8(6H,m), 4.21(1H,s), 7.1-7.5(10H,m).

**Reference Example A6**

Production of 2-tert-butyl-6-chloro[1,2,4]triazolo[1,5-b]pyridazine

step A:

N,N-Dimethylpivalamide (36 g) was dissolved in toluene (85 ml); under ice cooling conditions, phosphorus oxychloride (11.3 ml) was added thereto drop by drop, followed by stirring at room temperature for 24 hours. To this solution, 3-amino-6-chloropyridazine (12.0 g) was added, followed by stirring at 60-70°C for 24 hours. After cooling, ethyl acetate was added thereto; the mixture was washed with 2 N aqueous solution of sodium hydroxide and saline and dried over sodium sulfate. After the extract was concentrated under reduced pressure, the residue was subjected to silica gel column chromatography and eluted with hexane:ethyl acetate:triethylamine (50:50:2). The desired fractions were collected to give $N^2$-(6-chloropyridazin-3-yl)-$N^1$,$N^1$-dimethylpivalamidine (6.38 g). The mother liquor was purified by silica gel column chromatography to give the amidine (6.07 g). The amidine derivative obtained (12.5 g) was dissolved in methanol (60 ml); a solution of hydroxylamine hydrochloride (4.31 g) in methanol (40 ml) was s added thereto, followed by stirring at room temperature for 2 hours. The methanol was concentrated to half volume under reduced pressure; the crystals precipitated were collected by filtration, washed with water and ethyl ether and dried to give the title compound (10.44 g).
melting point: 128-130°C
elemental analysis for $C_9H_{13}N_4OCl$
Calculated (%): C,47.27 ; H,5.73 ; N,24.50
Found (%): C,47.28 ; H,5.59 ; N,24.34

step B:

N-(6-Chloropyridazin-3-yl)pivalamidoxime (4.07 g) was suspended in chloroform (170 ml); phosphorus oxychloride (8.3 ml) was added thereto drop by drop, followed by heating and refluxing for 5 hours. After cooling, ice water and 2 N aqueous solution of sodium hydroxide were added thereto, followed by extraction with chloroform; the extract was washed with saline and dried over magnesium sulfate. After the extract was concentrated under reduced pressure, the residue was subjected to silica gel column chromatography and eluted with hexane:ethyl acetate (5:1). The desired fractions were collected and concentrated to give the title compound (1.12 g).
melting point: 95-97°C
elemental analysis for $C_9H_{11}N_4Cl{\cdot}0.3H_2O$
Calculated (%): C,50.03 ; H,5.41 ; N,25.93
Found (%): C,50.23 ; H,5.12 ; N,25.90

**Reference Example A7**

Production of methyl 6-chloro[1,2,4]triazolo[1,5-b]pyridazine-2-carboxylate

step A: 6-chloro[1,2,4]triazolo[1,5-b]pyridazine-2-carboxylic acid

6-Chloro-2-methyl[1,2,4]triazdo[1,5-b]pyridazine (10.0 g) was added to concentrated sulfuric acid (55 ml) under ice cooling conditions; sodium dichromate dihydrate (19.4 g) was added little by little at the same temperature,

followed by stirring at room temperature for 4 days. Under ice cooling conditions, ice water (ca. 200 ml) was added thereto; the crystals precipitated were collected by filtration, washed with water and ethyl ether and dried to give the title compound (9.74 g).
melting point: 221°C (decomp.)
elemental analysis for $C_6H_3N_4O_2Cl$
Calculated (%): C,36.29 ; H,1.52 ; N,28.22
Found (%): C,35.96 ; H,1.59 ; N,28.12

step B:

6-Chloro[1,2,4]triazolo[1,5-b]pyridazin-2-carboxylic acid (3.02 g) was dissolved in N,N-dimethylformamide (50 ml); N-ethyldiisopropylamine (3.15 ml) was added thereto, followed by the addition of methyl iodide (1.14 ml) with stirring under ice water cooling conditions. After stirring at room temperature for 19 hours, ice water (ca. 200 ml) was added thereto; the crystals precipitated were collected by filtration and washed with water and ethyl ether. The mother liquor was purified by silica gel column chromatography; the crystals obtained were combined with the above washings and dried to give the title compound (2.91 g).
melting point: 208-209°C
elemental analysis for $C_7H_5N_4O_2Cl$
Calculated (%): C,39.55 ; H,2.37 ; N,26.35
Found (%): C,39.65 ; H,2.46 ; N,26.34

**Reference Example A8**

Production of ethyl 2-(6-chloroimidazo[1,2-b]pyridazin-2-yl)-2-methylpropionate

method A

step A:

3-Amino-6-chloropyridazine (11.2 g) was suspended in ethanol (150 ml); ethyl 4-chloroacetoacetate (28.6 g) was added thereto, followed by heating and refluxing for 24 hours. After cooling, the mixture was concentrated under reduced pressure; the residue was adjusted to pH 7 by the addition of an aqueous solution of sodium hydrogen carbonate, followed by extraction with ethyl acetate; the extract was washed with saline and dried over magnesium sulfate. The extract was concentrated under reduced pressure; the residue was subjected to silica gel column chromatography and eluted with hexane:ethyl acetate (2:3). The desired fractions were collected to give the title compound (12.7 g).
$^1H$-NMR($CDCl_3$)δ ppm: 1.29(3H,t,J=7Hz), 3.89(2H,s), 4.23(2H,q,J=7Hz), 7.05, 7.85(each 1H,d,J=9Hz), 7.95(1H,s).

step B:

Ethyl 6-chloroimidazo[1,2-b]pyridazin-2-acetate (6.8 g) was dissolved in N,N-dimethylformamide (50 ml); while the solution was stirred under ice water cooling conditions, 60% oily sodium hydride (2.46 g) was added little by little, followed by stirring at room temperature for 30 minutes. Under ice water cooling conditions, methyl iodide (4.36 ml) was added thereto, followed by stirring at room temperature for 2 hours. Ice water was poured thereto, followed by extraction with ethyl acetate; the extract was washed with saline and dried over magnesium sulfate. The extract was concentrated under reduced pressure; the residue was subjected to silica gel column chromatography and eluted with hexane:ethyl acetate (2:1). The desired fractions were collected and concentrated to give the title compound (4.06 g).
melting point: 64-65°C
elemental analysis for $C_{12}H_{14}N_3O_2Cl$
Calculated (%): C,53.84 ; H,5.27 ; N,15.70
Found (%): C,53.85 ; H,5.16 ; N,15.80

method B

The objective compound can be also synthesized by the following method.
3-Amino-6-chloropyridazine (80.0 g), ethyl 4-bromo-2,2-dimethyl-3-oxobutanoate (201 g) and disodium hydrogenphosphate (131 g) were suspended in ethanol (300 ml), followed by heating and refluxing for 8 hours. Water (300 ml) was added to the reaction mixture, followed by two extractions with ethyl acetate (300 ml). The organic layers

combined was washed with water (600 ml) twice and with brine (300 ml), followed by drying over magnesium sulfate, treating with activated carbons, filtration and concentrating under reduced pressure. The residue was dissolved in diisopropyl ether (200 ml); the insoluble substances were filtrated off and the filtrate was concentrated under reduced pressure. The residue was subjected to silica gel column chromatography (hexane:ethyl acetate 100:1, 2:1 and 1:1) and crystallized from hexane to give the title compound (99.3 g).

**Reference Example A9**

Production of methyl 2-(6-chloroimidazo[1,2-b]pyridazin-2-yl)-2-methylpropionate

3-Amino-6-chloropyridazine (10.1 g) was suspended in methanol (120 ml); methyl 4-chloroacetoacetate (23.5 g) was added thereto, followed by heating and refluxing for 20 hours. After cooling, the mixture was concentrated under reduced pressure; the residue was adjusted to pH 7 by the addition of an aqueous solution of sodium hydrogen carbonate, followed by extraction with ethyl ether; the extract was washed with saline and dried over magnesium sulfate. The extract was concentrated under reduced pressure; the residue was subjected to silica gel column chromatography and eluted with hexane:ethyl acetate (1:4). The desired fractions were collected to give methyl 6-chloroimidazo[1,2-b]pyridazin-2-acetate (9.15 g). Methyl 6-chloroimidazo[1,2-b]pyridazin-2-acetate (9.15 g) was dissolved in N,N-dimethylformamide (70 ml); while the solution was stirred under ice water cooling conditions, 60% oily sodium hydride (3.5 g) was added thereto little by little, followed by stirring at room temperature for 30 minutes. Under ice water cooling conditions, methyl iodide (6.3 ml) was added thereto, followed by stirring at room temperature for 5 hours. Ice water was poured thereto, followed by extraction with ethyl acetate; the extract was washed with saline and dried over magnesium sulfate. The extract was concentrated under reduced pressure; the residue was subjected to silica gel column chromatography and eluted with hexane:ethyl acetate (1:1). The desired fractions were collected and concentrated to give the title compound (14.1 g).
melting point: 92-93°C
elemental analysis for $C_{11}H_{12}N_3O_2Cl$
Calculated (%): C,52.08 ; H,4.77 ; N,16.56
Found (%): C,52.01 ; H,4.60 ; N,16.59

**Reference Example A10**

Production of 2-(6-chloroimidazo[1,2-b]pyridazin-2-yl)-2-methylpropionic acid

Methyl 2-(6-chloroimidazo[1,2-b]pyridazin-2-yl)-2-methylpropionate (1.40 g) was dissolved in tetrahydrofuran (15 ml); 1 N aqueous solution of sodium hydroxide (9 ml) was added thereto, followed by stirring at room temperature for 3 hours. After the mixture was concentrated under reduced pressure, the residue was adjusted to pH 4 by the addition of 1 N hydrochloric acid; the crystals precipitated were collected by filtration to give the title compound (1.06 g).
melting point: 159-161°C
elemental analysis for $C_{10}H_{10}N_3O_2Cl$
Calculated (%): C,50.12 ; H,4.21 ; N,17.53
Found (%): C,50.36 ; H,4.34 ; N,17.32

**Reference Example A11**

Production of tert-butyl 2-(6-chloroimidazo[1,2-b]pyridazin-2-yl)-2-methylpropionate

2-(6-Chloroimidazo[1,2-b]pyridazin-2-yl)-2-methylpropionic acid (0.863 g) was suspended in toluene (10 ml); N,N-dimethylformamide di-tert-butylacetal (2.6 ml) was added thereto, followed by stirring at 80°C for 1 hour. After cooling, the mixture was diluted with ethyl acetate, washed with an aqueous solution of sodium hydrogen carbonate and dried over magnesium sulfate. After the extract was concentrated under reduced pressure, ethyl ether was added to the residue; the precipitated crystals were collected and dried to give the title compound (0.52 g).
$^1$H-NNR($CDCl_3$)δ ppm: 1.43(9H,s), 1.64(6H,s), 7.02, 7.87(each 1H,d,J=9Hz), 7.84(1H,s).

**Reference Example A12**

Production of 6-chloro-2-methoxyimidazo[1,2-b]pyridazine

6-Chloro-2-hydroxyimidazo[1,2-b]pyridazine (2.69 g) was suspended in N,N-dimethylformamide (30 ml); 60%

oily sodium hydride (838 mg) was added thereto little by little, followed by stirring at room temperature for 30 minutes. Under ice water cooling conditions, methyl iodide (1.2 ml) was added thereto, followed by stirring at room temperature for 3 days. Ice water was added thereto, followed by extraction with ethyl acetate; the extract was washed with saline and dried over magnesium sulfate. After the extract was concentrated under reduced pressure, the residue was subjected to silica gel column chromatography and eluted with hexane:ethyl acetate (5:1). The desired fractions were collected and concentrated to give the title compound (1.05 g).
melting point: 134-136°C
elemental analysis for $C_7H_6N_3OCl$
Calculated (%): C,45.79 ; H,3.29 ; N,22.89
Found (%): C,45.68 ; H,3.27 ; N,22.79

**Reference Example A13**

Production of 4-(diphenylmethoxy)-1-piperidinepentaneamine

Potassium phthalimide (3.70 g) was dissolved in N,N-dimethylformamide (20 ml); 1,5-dichloropentane (5.4 ml) was added thereto, followed by stirring at room temperature for 15 hours. Ice water was added to the reaction mixture, followed by extraction with ethyl acetate; the extract was washed with saline and dried over magnesium sulfate. The extract was concentrated under reduced pressure; the residue was subjected to silica gel column chromatography and eluted with hexane-ethyl acetate (4:1). The desired fractions were collected to give N-(5-bromopentyl)phthalimide (4.68 g) as an oil. N-(5-Bromopentyl)phthalimide (4.68 g) and 4-(diphenylmethoxy)piperidine (4.25 g) were dissolved in N,N-dimethylformamide (30 ml); potassium carbonate (2.42 g) was added thereto, followed by stirring at room temperature for 15 hours. Ice water was added to the reaction mixture, followed by extraction with ethyl acetate; the extract was washed with saline and dried over magnesium sulfate. The extract was concentrated under reduced pressure; the residue was subjected to silica gel column chromatography and eluted with hexane-ethyl acetate-triethylamine (50:50:1). The desired fractions were collected to give N-[5-[4-(diphenylmethoxy)piperidino]pentyl]phthalimide (6.67 g) as an oil. N-[5-[4-(Diphenylmethoxy)piperidino]pentyl]phthalimide (6.6 g) was dissolved in ethanol (30 ml); hydrazine monohydrate (0.694 ml) was added thereto, followed by thermal refluxing for 3 hours. After cooling, the mixture was concentrated under reduced pressure; ethyl acetate was added to the residue; the crystals precipitated were collected, dissolved in 1 N aqueous solution of sodium hydroxide (15 ml) and water (20 ml) and extracted with ethyl acetate; the extract was washed with saline and dried over sodium sulfate. The extract was concentrated under reduced pressure; the crystals obtained were collected to give the title compound (3.29 g).
$^1$H-NMR($CDCl_3$)δ ppm: 1.2-2.9(18H,m), 3.3-3.6(1H,m), 5.52(1H,s), 7.1-7.4(10H,m).

**Reference Example A14**

Production of 1-amino-3-[4-(diphenylmethoxy)piperidino]-2-propanol

Potassium phthalimide (3.70 g) was dissolved in N,N-dimethylformamide (20 ml); epibromohydrin (2.58 ml) was added thereto, followed by stirring at room temperature for 15 hours. Ice water was added to the reaction mixture, followed by extraction with ethyl acetate; the extract was washed with saline and dried over magnesium sulfate. The extract was concentrated under reduced pressure; ethyl ether was added to the residue; the crystals precipitated were collected to give N-(2-oxylanylmethoxy)phthalimide (3.7 g).

N-(2-Oxylanylmethoxy)phthalimide (0.61 g) and 4-(diphenylmethoxy)piperidine (0.802 g) were dissolved in ethanol (10 ml), followed by thermal refluxing for 2 hours. The reaction mixture was concentrated under reduced pressure; the residue was subjected to silica gel column chromatography and eluted with ethyl acetate. The desired fractions were collected to give N-[3-[4-(diphenylmethoxy)piperidino-2-hydroxypropyl]phthalimide (1.30 g) as an oil. This oil was dissolved in ethanol (10 ml); hydrazine monohydrate (0.14 ml) was added thereto, followed by thermal refluxing for 3 hours. After cooling, the mixture was concentrated under reduced pressure; ethanol was added to the residue; the crystals precipitated were collected, dissolved in 1 N aqueous solution of sodium hydroxide (3 ml) and water (10 ml) and extracted with ethyl acetate; the extract was washed with saline and dried over sodium sulfate. The extract was concentrated under reduced pressure; the crystals obtained were collected to give the title compound (0.76 g).
$^1$H-NMR($CDCl_3$)δ ppm: 1.2-3.0(12H,m), 3.3-3.55(1H,m), 3.55-3.8(1H,m), 5.52(1H,s), 7.1-7.5(10H,m).

**Reference Example A15**

Production of 4-[bis(4-fluorophenyl)methoxy]-1-piperidinepropanamine

4,4'-Difluorobenzophenone (25 g) was dissolved in ethanol-tetrahydrofuran (180 ml-60 ml); sodium borohydride (2.16 g) was added thereto under ice cooling conditions, followed by stirring at room temperature for 30 minutes. The mixture was concentrated under reduced pressure; the residue was diluted with ice water and extracted with ethyl acetate; the extract was washed with saline and dried over magnesium sulfate. The extract was concentrated under reduced pressure; the oil (29.2 g) obtained was dissolved in toluene (800 ml); 4-hydroxypiperidine (11.6 g) and p-toluenesulfonic acid monohydrate (23.7 g) were added thereto, followed by thermal refluxing for 2 hours. After cooling, the mixture was concentrated under reduced pressure; ice water and 1 N aqueous solution of sodium hydroxide (130 ml) were added thereto, followed by extraction with ethyl acetate; the extract was washed with saline and dried over magnesium sulfate. The extract was concentrated under reduced pressure; the oily substance obtained (34.5 g) was dissolved in N,N-dimethylformamide (100 ml); N-(3-bromopropyl)phthalimide (16.3 g) and potassium carbonate (10.5 g) were added thereto, followed by stirring at room temperature for 20 hours. Ice water was added to the reaction mixture, followed by extraction with ethyl acetate; the extract was washed with saline and dried over magnesium sulfate. The extract was concentrated under reduced pressure; the residue was subjected to silica gel column chromatography and eluted with hexane:ethyl acetate (1:2). The desired fractions were collected to give N-[3-[4-[bis(4-fluorophenyl) methoxy]piperidino]propyl]phthalimide (20.5 g) as an oil. This oil (20.5 g) was dissolved in ethanol (150 ml); hydrazine monohydrate (2.02 ml) was added thereto, followed by thermal refluxing for 3 hours. After cooling, the mixture was concentrated under reduced pressure; ethanol was added to the residue; the crystals precipitated were collected, dissolved in 1 N aqueous solution of sodium hydroxide (40 ml) and extracted with ethyl acetate-tetrahydrofuran (2:1); the extract was washed with saline and dried over sodium sulfate. The extract was concentrated under reduced pressure to give the title compound (12.07 g) as an oil.
$^1$H-NMR($CDCl_3$)δ ppm: 1.5-2.2(10H,m), 2.36(2H,d,J=7Hz), 2.74(2H,d,J=7Hz), 3.3-3.5(1H,m), 5.47(1H,s), 6.9-7.4(8H,m).

**Reference Example A16**

Production of 4-[bis(4-methylphenyl)methoxy]-1-piperidinepropanamine

4,4'-Dimethylbenzophenone (25 g) was dissolved in ethanol-tetrahydrofuran (180 ml-60 ml); sodium borohydride (2.23 g) was added thereto under ice cooling conditions, followed by stirring at room temperature for 24 hours. The mixture was concentrated under reduced pressure; ice water was added to the residue; the crystals precipitated were collected and dried; the crystals obtained (30.5 g) were dissolved in toluene (800 ml); 4-hydroxypiperidine (11.9 g) and p-toluenesulfonic acid monohydrate (24.9 g) were added thereto, followed by thermal refluxing for 3 hours. After cooling, the mixture was concentrated under reduced pressure; ice water (100 ml) and 1 N aqueous solution of sodium hydroxide (140 ml) were added thereto, followed by extraction with ethyl acetate; the extract was washed with saline and dried over sodium sulfate. The extract was concentrated under reduced pressure; the residue was subjected to silica gel column chromatography and eluted with ethyl acetate-methanol-triethylamine (90;10:1). The desired fractions were collected to give 4-[bis(4-methylphenyl)methoxy]piperidine (32.8 g) as an oil. 4-[Bis(4-methylphenyl)methoxy] piperidine (16.4 g) was dissolved in N,N-dimethylformamide (100 ml); N-(3-bromopropyl)phthalimide (14.2 g) and potassium carbonate (8.15 g) were added thereto, followed by stirring at room temperature for 16 hours. Ice water was added to the reaction mixture, followed by extraction with ethyl acetate; the extract was washed with saline and dried over magnesium sulfate. The extract was concentrated under reduced pressure; the residue was subjected to silica gel column chromatography and eluted with hexane:ethyl acetate (1:2). The desired fractions were collected to give N-[3-[4-[bis(4-methylphenyl)methoxy]-piperidino]propyl]phthalimide (21.2 g) as an oil. This oil (20.5 g) was dissolved in ethanol (150 ml); hydrazine monohydrate (2.18 ml) was added thereto, followed by thermal refluxing for 3 hours. After cooling, the mixture was concentrated under reduced pressure; ethanol was added to the residue; the crystals precipitated were collected, dissolved in 1 N aqueous solution of sodium hydroxide (40 ml) and extracted with ethyl acetate-tetrahydrofuran (2:1); the extract was washed with saline and dried over sodium sulfate. The extract was concentrated under reduced pressure to give the title compound (10.5 g) as an oil.
$^1$H-NMR($CDCl_3$)δ ppm: 1.4-2.9(14H,m), 2.31(6H,s), 3.3-3.50(1H,m), 5.46(1H,s), 7.11, 7.22(each 4H,d,J=8Hz).

**Reference Example A17**

Production of N-(6-chloroimidazo[1,2-b]pyridazine-2-carbonyl)glycine ethyl ester

6-Chloroimidazo[1,2-b]pyridazine-2-carboxylic acid (0.593 g) was suspended in N,N-dimethylformamide (7.5 ml); N,N'-carbonyldiimidazole (0.535 g) and glycine ethyl ester hydrochloride (0.46 g) were added thereto, followed by stirring at room temperature for 30 minutes. To this mixture, triethylamine (0.457 ml) was added, followed by further stirring for 1 hour. Ice water was added to the reaction mixture; the crystals precipitated were collected by filtration, washed with water and dried to give the title compound (0.749 g).
melting point: 190-191°C
elemental analysis for $C_{11}H_{11}N_4O_3Cl$
Calculated (%): C,46.74 ; H,3.92 ; N,19.82
Found (%): C,46.70 ; H,4.03 ; N,19.75

**Reference Example A18**

Production of 2-(6-chloroimidazo[1,2-b]pyridazin-2-yl)-2-methylpropionamide

2-(6-Chloroimidazo[1,2-b]pyridazin-2-yl)-2-methylpropionic acid (1.20 g) was dissolved in N,N-dimethylformamide (8 ml); N,N'-carbonyldiimidazole (0.892 g) was added thereto, followed by stirring at room temperature for 30 minutes. To this mixture, ammonium chloride (0.321 g) and triethylamine (0.832 ml) were added thereto under ice cooling conditions, followed by stirring at room temperature for 3 hours. Ice water was added to the reaction mixture; the crystals precipitated were collected by filtration, washed with water and dried to give the title compound (0.697 g).
melting point: 194-195°C
elemental analysis for $C_{10}H_{11}N_4OCl$
Calculated (%): C.50.32 ; H,4.65 ; N,23.47
Found (%): C,50.34 ; H,4.60 ; N,23.43

**Reference Example A19**

Production of 2-(6-chloroimidazo[1,2-b]pyridazin-2-yl)-N,N,2-trimethylpropionamide

2-(6-Chloroimidazo[1,2-b]pyridazin-2-yl)-2-methylpropionic acid (0.959 g) was dissolved in N,N-dimethylformamide (6 ml); N,N'-carbonyldiimidazole (0.714 g) was added thereto, followed by stirring at room temperature for 60 minutes. To this mixture, dimethylamine hydrochloride (0.392 g) and triethylamine (0.665 ml) were added under ice cooling conditions, followed by stirring at room temperature for 3 hours. Saline was added to the reaction mixture, followed by extraction with ethyl acetate; the extract was washed with saline and dried over magnesium sulfate. The extract was concentrated under reduced pressure; the residue was subjected to silica gel column chromatography and eluted with ethyl acetate:methanol (95:5). The desired fractions were collected and concentrated; the crystals obtained were collected by filtration to give the title compound (0.608 g). melting point: 149-151°C
elemental analysis for $C_{12}H_{15}N_4OCl$
Calculated (%): C,54.04 ; H,5.67 ; N,21.01
Found (%): C,53.90 ; H,5.85 ; N,21.04

**Reference Example A20**

Production of 2-(6-chloroimidazo[1,2-b]pyridazin-2-yl)-2-methylpropanol

2-(6-Chloroimidazo[1,2-b]pyridazin-2-yl)-2-methylpropionic acid (0.719 g) was dissolved in tetrahydrofuran (15 ml); N,N'-carbonyldiimidazole (0.535 g) was added thereto, followed by stirring at room temperature for 60 minutes. To this mixture, tetra-n-butylammonium borohydride (1.15 g) was added under ice cooling conditions, followed by stirring at room temperature for 1 hour. 5 N hydrochloric acid (2 ml) was added to the reaction mixture, followed by concentration under reduced pressure. The residue was adjusted to pH 7 by the addition of aqueous sodium carbonate and extracted with ethyl acetate; the extract was washed with saline and dried over magnesium sulfate. The extract was concentrated under reduced pressure; the residue was subjected to silica gel column chromatography and eluted with ethyl acetate. The desired fractions were collected and concentrated; the crystals obtained were collected by filtration to give the title compound (0.488 g).
$^1H$-NMR($CDCl_3$)$\delta$ ppm: 1.39(6H,s), 3.72(2H,s), 7.04, 7.82(each 1H,d,J=9.5Hz), 7.76(1H,s).

**Reference Example A21**

Production of N-(6-chloroimidazo[1,2-b]pyridazine-2-carbonyl)-2,2-dimethylglycine ethyl ester

6-Chloroimidazo[1,2-b]pyridazine-2-carboxylic acid (1.28 g) was suspended in N,N-dimethylformamide (12 ml); N,N'-carbonyldiimidazole (1.16 g) was added thereto, followed by stirring at room temperature for 30 minutes. To the reaction mixture, 2-aminoisobutyric acid ethyl ester hydrochloride (1.20 g) and triethylamine (1.00 ml) were added, followed by stirring at room temperature for 16 hours. Water was added, the crystals precipitated were collected by filtration; the filtrate was extracted with ethyl acetate; the extract was washed with brine and dried over magnesium sulfate. The extract was concentrated under reduced pressure; the residue was combined with the above crystals collected by filtration and subjected to silica gel column chromatography and eluted with hexane:ethyl acetate (2:1). The desired fractions were collected and concentrated under reduced pressure to give the title compound (1.20 g).
$^1$H-NMR($CDCl_3$)δ ppm: 1.28(3H,t,J=7.2Hz), 1.70(6H,s), 4.25(2H,q,J=7.0Hz), 7.13(1H,d,J=9.4Hz), 7.87(1H,brs), 7.89 (1H,d,J=9.6Hz), 8.41(1H,s).

**Reference Example A22**

Production of ethyl 2-(3,6-dichloroimidazo[1,2-b]pyridazin-2-yl)-2-methylpropionate

Ethyl 2-(6-chloroimidazo[1,2-b]pyridazin-2-yl)-2-methylpropionate (4.07 g) was suspended in ethyl acetate (60 ml); N-chlorosuccinimide (2.13 g) was added thereto, followed by thermal refluxing for 4 hours. After cooling, water was added thereto, followed by extraction with ethyl acetate; the extract was washed with brine and dried over magnesium sulfate. The extract was concentrated under reduced pressure; the residue was subjected to silica gel column chromatography and eluted with hexane:ethyl acetate (5:1). The desired fractions were collected and concentrated under reduced pressure to give the title compound (4.48 g).
melting point: 66-67°C
elemental analysis for $C_{12}H_{13}N_3O_2Cl_2$
Calculated (%): C,47.70 ; H,4.34 ; N,13.91
Found (%): C,47.67 ; H,4.23 ; N,13.93

**Reference Example A23**

Production of methyl 2-(6-chloro-7-methylimidazo[1,2-b]pyridazin-2-yl)-2-methylpropionate

step A: Production of methyl 6-chloro-7-methylimidazo[1,2-b]pyridazine-2-acetate

6-Amino-3-chloro-4-methylpyridazine (15.3 g) was suspended in methanol (200 ml); methyl 4-chloroacetoacetate (25.0 ml) was added thereto, followed by thermal refluxing for 36 hours. After cooling, the mixture was concentrated under reduced pressure; the residue was adjusted to pH 7 by the addition of an aqueous solution of sodium hydrogen carbonate and extracted with ethyl acetate; the extract was washed with saline and dried over magnesium sulfate. The extract was concentrated under reduced pressure; the residue was subjected to silica gel column chromatography and eluted with hexane:ethyl acetate (1:4). The desired fractions were collected to give the title compound (14.3 g).
melting point: 98-99°C
elemental analysis for $C_{10}R_{10}N_3O_2Cl$
Calculated (%): C,50.12 ; H,4.21 ; N,17.53
Found (%) : C,50.07 ; H,4.25 ; N,17.74

step B:

60% Oily sodium hydride (4.8 g) was suspended in N,N-dimethylformamide (150 ml); while this suspension was stirred under ice cooling conditions, methyl 6-chloro-7-methylimidazo[1,2-b]pyridazin-2-acetate (11.4 g) was added thereto little by little; followed by stirring at room temperature for 30 minutes. Under ice cooling conditions, methyl iodide (7.5 ml) was added thereto, followed by stirring at room temperature for 6 hours. Ice water was poured thereto, followed by extraction with ethyl acetate; the extract was washed with saline and dried over magnesium sulfate. The extract was concentrated under reduced pressure; the residue was subjected to silica gel column chromatography and eluted with hexane:ethyl acetate (1:1). The desired fractions were collected and concentrated to give the title compound (9.17 g).

melting point: 109-110°C
elemental analysis for $C_{12}H_{14}N_3O_2Cl$
Calculated (%): C,53.84 ; H,5.27 ; N,15.70
Found (%): C,53.96 ; H,5.19 ; N,15.86

**Reference Example A24**

Production of isopropyl 1-(6-chloroimidazo[1,2-b]pyridazin-2-yl)cyclopentanecarboxylate

step A: Production of methyl 1-(6-chloroimidazo[1,2-b]pyridazin-2-yl)cyclopentanecarboxylate

Methyl 6-chloroimidazo[1,2-b]pyridazin-2-acetate (5.48 g) was dissolved in N,N-dimethylformamide (42 ml); while this solution was stirred under ice cooling conditions, 60% oily sodium hydride (1.07 g) was added thereto little by little; followed by stirring at room temperature for 1.5 hours. Under ice cooling conditions, 1,4-dibromobutane (3.19 ml) was added thereto drop by drop, followed by stirring at room temperature for 18 hours. Ice water was poured thereto, followed by extraction with ethyl acetate; the extract was washed with brine and dried over magnesium sulfate. The extract was concentrated under reduced pressure; the residue was subjected to silica gel column chromatography and eluted with ethyl acetate:hexane (1:3). The desired fractions were collected and concentrated under reduced pressure to give the title compound (1.72 g).
$^1$H-NMR($CDCl_3$)δ ppm: 1.63-1.85(4H,m), 2.10-2.38(2H,m), 2.42-2.68(2H,m), 3.69(3H,s), 7.02(1H,d,J=9.4Hz), 7.84 (1H,s), 7.86(1H,d,J=8.6Hz).

step B:

In 2-propanol (30 ml), concentrated sulfuric acid (0.81 ml) was dissolved; methyl 1-(6-chloroimidazo[1,2-b]pyridazin-2-yl)cyclopentanecarboxylate (1.7 g) was added thereto, followed by thermal refluxing for 7.5 hours. After cooling, the mixture was concentrated under reduced pressure, neutralized by the addition of aqueous sodium hydrogen carbonate solution and extracted with ethyl acetate. The extract was washed with brine and dried over magnesium sulfate. The extract was concentrated under reduced pressure; the crystals precipitated were collected by filtration, washed with hexane and dried to give the title compound (1.30 g). The filtrate was concentrated under reduced pressure; the residue was subjected to silica gel column chromatography and eluted with hexane:ethyl acetate (3:1). The desired fractions were collected, concentrated under reduced pressure and dried to give the title compound (356 mg).
$^1$H-NMR($CDCl_3$)δ ppm: 1.18(3H,s), 1.21(3H,s), 1.68-1.85(4H,m), 2.13-2.32(2H,m), 2.45-2.60(2H,m), 4.94-5.13(1H,m), 7.02 (1H,d,J=9.6Hz), 7.83(1H,s), 7.86(1H,d,J=9.4Hz).

**Reference Example A25**

Production of isopropyl 1-(6-chloroimidazo[1,2-b]pyridazin-2-yl)cyclopropanecarboxylate

step A: Production of methyl 1-(6-chloroimidazo[1,2-b]pyridazin-2-yl)cyclopropanecarboxylate

Methyl 6-chloroimidazo[1,2-b]pyridazin-2-acetate (5.93 g) was dissolved in N,N-dimethylformamide (45 ml); while this solution was stirred under ice cooling conditions, 60% oily sodium hydride (2.31 g) was added thereto little by little; followed by stirring at room temperature for 40 minutes. Under ice cooling conditions, 1,2-dibromoethane (2.49 ml) was added thereto drop by drop, followed by stirring at room temperature for 14 hours. Ice water was poured thereto, followed by extraction with ethyl acetate; the extract was washed with brine and dried over magnesium sulfate. The extract was concentrated under reduced pressure; the residue was subjected to silica gel column chromatography and eluted with hexane:ethyl acetate (2:1). The desired fractions were collected and concentrated under reduced pressure to give the title compound (3.67 g).
$^1$H-NMR($CDCl_3$)δ ppm: 1.60-1.68(2H,m), 1.70-1.85(2H,m), 3.75(3H,s), 7.00(1H,d,J=9.6Hz), 7.77(1H,d,J=9.6Hz), 8.28 (1H,s).

step B:

In 2-propanol (70 ml), concentrated sulfuric acid (1.82 ml) was dissolved; methyl 1-(6-chloroimidazo[1,2-b]pyridazin-2-yl)cyclopropanecarboxylate (3.44 g) was added thereto, followed by thermal refluxing for 7.5 hours. After cooling, the mixture was concentrated under reduced pressure, neutralized by the addition of aqueous sodium hydrogen carbonate solution and extracted with ethyl acetate. The extract was washed with brine and dried over magnesium

sulfate. The extract was concentrated under reduced pressure; the crystals precipitated were collected by filtration, washed with ether and hexane and dried to give the title compound (1.98 g). The filtrate was concentrated under reduced pressure; the residue was subjected to silica gel column chromatography and eluted with hexane:ethyl acetate (5:1). The desired fractions were collected, concentrated under reduced pressure and dried to give the title compound (650 mg).
melting point: 112-114°C
elemental analysis for $C_{13}H_{14}N_3O_2Cl$
Calculated (%): C,55.82 ; H,5.04 ; N,15.02
Found (%): C,55.75 ; H.5.17 ; N,14.99

**Reference Example A26**

Production of ethyl 2-(6-chloro-3-methylimidazo[1,2-b]pyridazin-2-yl)-2-methylpropionate

step A: ethyl 6-chloro-3-methylimidazo[1,2-b]pyridazine-2-acetate

3-Amino-6-chloropyridazine (2.44 g) was suspended in ethanol (37 ml); ethyl 4-bromo-3-oxopentanoate (8.40 g) was added thereto, followed by thermal refluxing for 18 hours. After cooling, the mixture was concentrated under reduced pressure; the residue was adjusted to pH 7 by the addition of aqueous sodium hydrogen carbonate solution; ethyl ether was added thereto; the precipitated was collected by filtration and extracted with ethyl ether; the extract was washed with brine and dried over magnesium sulfate. The extract was concentrated under reduced pressure; the residue was subjected to silica gel column chromatography and eluted with hexane:ethyl acetate (1:1). The desired fractions were collected to give the title compound (2.63 g).
$^1$H-NMR($CDCl_3$)δ ppm: 1.27(3H,t,J=7.1Hz), 2.54(3H,s), 3.85(2H,s), 4.19(2H,q,J=7.1Hz), 6.99(1H,d,J=9.6Hz) 7.82(1H, d,J=9.6Hz).

step B:

Ethyl 6-chloro-3-methylimidazo[1,2-b]pyridazin-2-acetate (5.41 g) was dissolved in N,N-dimethylformamide (40 ml); while this solution was stirred under ice cooling conditions, 60% oily sodium hydride (1.87 g) was added thereto little by little; followed by stirring at room temperature for 40 minutes. Under ice cooling conditions, methyl iodide (3.32 ml) was added thereto, followed by stirring at room temperature for 15 hours. Ice water was poured thereto, followed by extraction with ethyl acetate; the extract was washed with brine and dried over magnesium sulfate. The extract was concentrated under reduced pressure; the residue was subjected to silica gel column chromatography and eluted with hexane:ethyl acetate (3:1). The desired fractions were collected and concentrated under reduced pressure to give the title compound (2.69 g).
$^1$H-NMR($CDCl_3$)δ ppm: 1.21(3H,t,J=7.2Hz), 1.69(6H,s), 2.48(3H,s), 4.47(2H,q,J=7.2Hz), 7.21(1H,d,J=9.6Hz), 7.88 (1H,d,J=9.6Hz).

**Reference Example A27**

Ethyl 6-chloro-2-methylimidazo[1,2-b]pyridazine-3-carboxylate

3-Amino-6-chloropyridazine (12.9 g) was suspended in ethanol (250 ml); ethyl 2-chloro-3-oxobutanoate (18.1 g) was added thereto, followed by thermal refluxing for 6 hours. After cooling, the mixture was concentrated under reduced pressure; the residue was adjusted to pH 7 by the addition of aqueous sodium hydrogen carbonate solution; ethyl ether was added thereto; the precipitated was collected by filtration and extracted with ethyl ether; the extract was washed with brine and dried over magnesium sulfate. The extract was concentrated under reduced pressure; ethanol:1 N aqueous solution of sodium hydroxide (1:1) was added thereto; the crystals precipitated were collected by filtration; the filtrate was concentrated again. The residue was crystallized by the addition of ethyl acetate and collected by filtration to give the title compound (3.09 g).
$^1$H-NMR($CDCl_3$)δ ppm: 1.45(3H,t,J=7.2Hz), 2.74(3H,s), 4.18(2H,q,J=7.2Hz), 6.97(1H,d,J=9.4Hz), 7.85(1H,d, J=9.6Hz).

**Reference Example A28**

Production of N-(6-chloro[1,2,4]triazolo[1,5-b]pyridazine-2-carbonyl)glycine ethyl ester

6-Chloro[1,2,4]triazolo[1,5-b]pyridazine-2-carboxylic acid (2.86 g) and N-ethyldiisopropylamine (2.72 ml) were suspended in N,N-dimethylformamide (30 ml); N,N'-carbonyldiimidazole (2.63 g) was added thereto, followed by stirring at room temperature for 1 hour. To the reaction mixture, glycine ethyl ester hydrochloride (2.21 g) was added, followed by stirring at room temperature for 5 hours. Water was added thereto; the crystals precipitated were collected by filtration, washed with water and ether and dried to give the title compound (2.93 g).
melting point: 175-177°C
elemental analysis for $C_{10}H_{10}N_5O_3Cl$
Calculated (%): C,42.34 ; H,3.55 ; N,24.69
Found (%): C,42.40 ; H,3.56 ; N,24.76

**Reference Example A29**

Production of ethyl 2-(6-chloroimidazo[1,2-b]pyridazin-2-yl)-2-ethylbutanate

step A: Production of ethyl 4-bromo-2,2-diethyl-3-oxobutanate

Ethyl 2,2-diethyl-3-oxobutanoate (11.5 g) was dissolved in acetic acid (50 ml); 25% solution of hydrobromic acid in acetic acid (1 ml) was added thereto; a solution of bromine (3.50 ml) in acetic acid (10 ml) was added thereto drop by drop in a water bath. After stirring at room temperature for 3 hours, the mixture was concentrated under reduced pressure; the residue was dissolved in hexane, washed with water, saturated aqueous sodium hydrogen carbonate solution and brine, and dried over magnesium sulfate. The extract was concentrated under reduced pressure to give the title compound (16.4 g).
$^1H$-NMR($CDCl_3$)δ ppm: 0.65-0.90(6H,m), 1.28(3H,t,J=7.2Hz), 1.80-2.15(4H,m), 4.09(2H,s), 4.22(2H,q,J=7.2Hz).

step B:

Ethyl 4-bromo-2,2-diethyl-3-oxobutanoate (13.2 g), 3-amino-6-chloropyridazine (5.89 g) and sodium hydrogen carbonate (5.76 g) were suspended in ethanol (33 ml), followed by thermal refluxing for 1 day. After cooling, water was added thereto, followed by extraction with diisopropyl ether; the extract was washed with brine and dried over magnesium sulfate. The extract was concentrated under reduced pressure; the residue was subjected to silica gel column chromatography and eluted with hexane:ethyl acetate (4:1). The desired fractions were collected and concentrated under reduced pressure to give the title compound (5.20 g).
melting point: 68-70°C
elemental analysis for $C_{14}H_{18}N_3O_2Cl$
Calculated (%): C,55.85 ; H,6.13 ; N,14.21
Found (%) : C,55.86 ; H,6.07 ; N,13.99

**Reference Example A30**

Production of 4-(diphenylmethylamino)-1-piperidinepropanamine step A: Production of N-[3-[4-(diphenylaminomethyl)-piperidino]propyl]phthalimide

N-(3-Bromopropyl)phthalimide (7.38 g) and 4-(diphenylmethylamino)piperidine (7.07 g) were dissolved in N, N-dimethylformamide (80 ml); potassium carbonate (4.04 g) was added thereto, followed by stirring at room temperature for 17 hours. Ice water was added to the reaction mixture, followed by extraction with ethyl acetate; the extract was washed with saline and dried over magnesium sulfate. The extract was concentrated under reduced pressure; the residue was subjected to silica gel column chromatography and eluted with hexane:ethyl acetate:triethylamine (50: 50:2). The desired fractions were collected and concentrated under reduced pressure to give the title compound (9.65 g) as an oil.
$^1H$-NMR($CDCl_3$)δ ppm: 1.08-1.30(2H,m), 1.42-1.64(2H,m), 1.74-1.92(4H,m), 2.25-2.42(1H,m), 2.34(2H,t,J=7.2Hz), 2.65-2.83(2H,m), 3.73(2H,t,J=6.9Hz), 4.96(1H,s), 7.12-7.40(10H,m), 7.65-7.73(2H,m), 7.78-7.88(2H,m).

step B:

N-[3-[4-(Diphenylaminomethyl)piperidino]-propyl]phthalimide (9.65 g) was dissolved in ethanol (40 ml); hydrazine monohydrate (1.08 ml) was added thereto, followed by thermal refluxing for 3.5 hours. After cooling, diisopropyl ether was added to the reaction mixture; the crystals precipitated were collected, washed with diisopropyl ether, dissolved in 1 N aqueous solution of sodium hydroxide (45 ml), tetrahydrofuran (20 ml) and water (20 ml), and extracted with ethyl acetate. The extract was washed with water and brine and dried over sodium sulfate. The extract was concentrated under reduced pressure to give the title compound (4.02 g).
$^1$H-NMR($CDCl_3$)δ ppm: 1.23-1.67(4H,m), 1.82-1.98(4H,m), 2.29-2.36(2H,m), 2.32-2.52(1H,m), 3.73(2H,t,J=7.4Hz), 2.71(2H,d,J=6.8Hz), 2.73-2.9.(2H,brm), 5.02(1H,s), 7.10-7.57(10H,m).

**Reference Example A31**

Production of N-(3,6-dichloroimidazo[1,2-b]pyridazine-2-carbonyl)glycine ethyl ester

N-(6-Chloroimidazo[1,2-b]pyridazine-2-carbonyl)glycine ethyl ester (0.86 g) was suspended in ethyl acetate (30 ml); N-chlorosuccinimide (1.2 g) was added thereto, followed by thermal refluxing for 20 hours. After cooling, tetrahydrofuran (30 ml) was added thereto; the mixture was washed with an aqueous solution of sodium thiosulfate and saline and dried over magnesium sulfate. The extract was concentrated under reduced pressure; ethyl ether was added to the residue; the crystals precipitated were collected by filtration, washed with ethyl ether and dried to give the title compound (0.552 g).
$^1$H-NMR($CDCl_3$)δ ppm: 1.32 (3H,t,J=7.2Hz), 4.27 (2H,d,J=5.6Hz), 4.27(2H,q,J=7.2Hz), 7.21,7.91(each 1H,d, J=9.6Hz), 7.82(1H,t,J=5.6Hz).

**Reference Example A32**

Production of N-(6-chloroimidazo[1,2-b]pyridazine-2-carbonyl) β-alanine ethyl ester

6-Chloroimidazo[1,2-b]pyridazine-2-carboxylic acid (1.98 g) was suspended in N,N-dimethylformamide (25 ml); N,N'-dicarbonylimidazole (1.78 g) was added thereto, followed by stirring at room temperature for 1 hour. To this mixture, β-alanine ethyl ester hydrochloride (1.69 g) and triethylamine (1.53 ml) were added, followed by further stirring for 3 hours. Ice water was added to the reaction mixture; the crystals precipitated were collected by filtration, washed with water and dried to give the title compound (2.57 g).
melting point: 132-134°C
elemental analysis for $C_{12}H_{13}N_4O_3Cl$
Calculated (%): C,48.58 ; H,4.42 ; N,18.88
Found (%): C,48.43 ; H,4.33 ; N,18.68

**Reference Example A33**

Production of ethyl 6-chloro-3-methylimidazo[1,2-b]pyridazine-2-carboxylate

3-Amino-6-chloropyridazine (5.83 g) was suspended in ethanol (70 ml); methyl 3-bromo-2-oxobutyrate (9.75 g) and N-ethyldiisopropylamine (8.6 ml) were added thereto, followed by thermal refluxing for 5 hours. After cooling, the mixture was concentrated under reduced pressure; the residue was adjusted to pH 7 by the addition of an aqueous solution of sodium hydrogen carbonate and extracted with ethyl acetate:tetrahydrofuran (1:1); the extract was washed with brine and dried over magnesium sulfate. The extract was concentrated under reduced pressure; ethyl acetate was added to the residue; the crystals precipitated were collected by filtration, washed with ethyl acetate and dried to give the title compound (3.90 g).
melting point: 170-171°C
elemental analysis for $C_{10}H_{10}N_3O_2Cl$
Calculated (%): C,50.12 ; H,4.21 ; N,17.53
Found (%): C,50.28 ; H,4.18 ; N,17.23

**Reference Example A34**

Production of N-(6-chloro-3-methylimidazo[1,2-b]pyridazine-2-carbonyl)glycine ethyl ester

Ethyl 6-chloro-3-methylimidazo[1,2-b]pyridazine-2-carboxylate (3.9 g) was suspended in tetrahydrofuran (40 ml); 1 N aqueous solution of sodium hydroxide (30 ml) was added thereto, followed by stirring at room temperature for 3 hours. The mixture was concentrated under reduced pressure; the residue was adjusted to pH 4 by the addition of water (50 ml) and 1 N hydrochloric acid; the crystals precipitated were collected by filtration and dried to give 6-chloro-3-methylimidazo[1,2-b]pyridazine-2-carboxylic acid (2.55 g). This carboxylic acid (1.27 g) was dissolved in N,N-dimethylformamide (20 ml); N,N'-carbonyldiimidazole (1.07 g) was added thereto, followed by stirring at room temperature for 30 minutes. To this mixture, glycine ethyl ester hydrochloride (0.922 g) and triethylamine (0.915 ml) were added, followed by further stirring for 3 hours. Ice water (60 ml) was added to the reaction mixture; the crystals precipitated were collected by filtration, washed with water and dried to give the title compound (1.18 g).
melting point: 192-195°C
elemental analysis for $C_{12}H_{13}N_4O_3Cl$
Calculated (%): C,48.58 ; H,4.42 ; N,18.88
Found (%) : C,48.65 ; H.4.13 ; N,18.93

**Reference Example A35**

Production of 6-chloro-2-isopropylimidazo[1,2-b]pyridazine

To a solution of 3-methyl-2-butanone (5.17 g) in methanol (60 ml), bromine (3.1 ml) was added under ice cooling conditions, followed by stirring for 45 minutes. To this mixture, water (30 ml) was added, followed by stirring at room temperature for 30 minutes. To this mixture, water and hexane were added; the organic layer was separated; the water layer was extracted with hexane. The organic layers combined were washed with water and a saturated aqueous solution of sodium hydrogen carbonate, dried over magnesium sulfate, filtered and concentrated under reduced pressure. The residue was dissolved in ethanol (20 ml); 3-amino-6-chloropyridazine (5.18 g) and sodium hydrogen carbonate (6.30 g) were added thereto, followed by thermal refluxing for 3 hours. Water and ethyl acetate were added to the reaction mixture; the insoluble substances were filtered off; the organic layer was separated; the water layer was extracted with ethyl acetate. The organic layers combined were washed with water, treated with activated charcoal, filtered and concentrated under reduced pressure. The residue was dissolved in ethyl acetate; the insoluble substances were filtered and concentrated under reduced pressure. The residue was subjected to silica gel column chromatography (hexane:ethyl acetate 3:1) and recrystallized from hexane to give the title compound (1.37 g).
melting point: 106-108°C
elemental analysis for $C_9H_{10}N_3Cl$
Calculated (%): C,55.25 ; H,5.15 ; N,21.48 ; Cl,18.12
Found (%): C,55.35 ; H,5.10 ; N,21.50 ; Cl,18.03

**Reference Example A36**

Production of isopropyl 6-chloro[1,2,4]triazolo[1,5-b]pyridazine-2-carboxylate

6-Chloro[1,2,4]triazolo[1,5-b]pyridazine-2-carboxylic acid (2.14 g) and N-ethyldiisopropylamine (5.57 ml) were dissolved in N,N-dimethylformamide (30 ml); isopropyl iodide (3.23 ml) was added thereto, followed by stirring at room temperature for 10 hours and at 50°C for 3 hours. After cooling, water was added thereto, followed by extraction with ethyl acetate; the extract was washed with brine and dried over magnesium sulfate. The extract was concentrated under reduced pressure; the residue was subjected to silica gel column chromatography and eluted with ethyl acetate. The desired fractions were collected and concentrated under reduced pressure to give the title compound (2.21 g).
$^1$H-NMR($CDCl_3$)δ ppm: 1.46(3H,s), 1.49(3H,s), 5.33-5.53(1H,m), 7.52(1H,d,J=9.6Hz), 8.19(1H,d,J=9.6Hz).

**Reference Example A37**

Production of N-(6-chloro[1,2,4]triazolo[1,5-b]pyridazine-2-carbonyl)-2,2-dimethylglycine ethyl ester

6-Chloro[1,2,4]triazolo[1,5-b]pyridazine-2-carboxylic acid (1.52 g) and N-ethyldiisopropylamine (1.45 ml) were suspended in N,N-dimethylformamide (15 ml); N,N'-carbonyldiimidazole (1.37 g) was added thereto, followed by stirring at room temperature for 3 hours. To the reaction mixture, 2-aminoisobutyric acid ethyl ester hydrochloride (1.41

g) was added, followed by stirring at room temperature for 4 hour. Water was added thereto, followed by extraction with ethyl acetate:tetrahydrofuran (1:1); the extract was washed with brine and dried over magnesium sulfate. The extract was concentrated under reduced pressure; the crystals precipitated were washed with ethyl ether, collected by filtration and dried to give the title compound (1.48 g). The filtrate was concentrated under reduced pressure; the residue was subjected to silica gel column chromatography and eluted with ethyl acetate. The desired fractions were collected and concentrated under reduced pressure to give the title compound (450 mg).
$^1$H-NMR($CDCl_3$)δ ppm: 1.29 (3H,t,J=7.lHz), 1.73(6H,s), 4.26(2H,q,J=7.2Hz), 7.52(1H,d,J=9.4Hz), 7.94(1H.brs), 8.16 (1H,d,J=9.4Hz).

**Reference Example A38**

Production of isopropyl 2-(6-chloroimidazo[1,2-b]pyridazin-2-yl)-2-methylpropionate

In isopropanol (18 ml), concentrated sulfuric acid (995 mg) was dissolved; methyl 2-(6-chloroimidazo[1,2-b] pyridazin-2-yl)-2-methylpropionate (1.0 g) was added thereto, followed by thermal refluxing for 40 hours. After cooling, the mixture was concentrated under reduced pressure, neutralized by the addition of aqueous sodium hydrogen carbonate solution and extracted with ethyl acetate. The extract was washed with brine and dried over magnesium sulfate. The extract was concentrated under reduced pressure; the crystals precipitated were collected, washed with hexane and dried to give the title compound (794 mg). The filtrate was concentrated under reduced pressure; the residue was subjected to silica gel column chromatography and eluted with hexane:ethyl acetate (5:1). The desired fractions were collected and dried to give the title compound (215 mg).
melting point: 100-101°C
elemental analysis for $C_{13}H_{16}N_3O_2Cl$
Calculated (%): C,55.42 ; H,5.72 ; N,14.91
Found (%): C,55.46 ; H,5.53 ; N,14.94

**Reference Example B133**

Production of ethyl 2-[6-[3-[4-(diphenylmethoxy)piperidino]-propylamino]imidazo[1,2-b]pyridazin-2-yl]-2-methylpropionate disuccinate

Ethyl 2-[6-[3-[4-(diphenylmethoxy)piperidino]-propylamino]imidazo[1,2-b]pyridazin-2-yl]-2-methylpropionate (0.278 g) synthesized in Reference Example B40 was dissolved in ethanol (1 mL), and succinic acid (0.118 g) was added thereto for dissolution, which was followed by concentration under reduced pressure. To the residue was added tetrahydrofuran (0.5 mL) for dissolution. Ethyl acetate (2 mL) was added and the precipitated crystals were collected by filtration, washed with ethyl acetate and dried to give the title compound (0.382 g).
melting point: 98-101°C (decomp.)
elemental analysis for $C_{41}H_{53}N_5O_{11}\cdot 1/3CH_3CO_2C_2H_5$
Calculated (%) :C,61.92 ; H,6.83 ; N,8.53
Found (%) :C,61.54 ; H,6.83 ; N,8.50

**Reference Example B134**

Production of ethyl 2-[6-[3-[4-(diphenylmethoxy)piperidino]-propylamino]imidazo[1,2-b]pyridazin-2-yl]-2-methylpropionate citrate (1:1)

Ethyl 2-[6-[3-[4-(diphenylmethoxy)piperidino]-propylamino]imidazo[1,2-b]pyridazin-2-yl]-2-methylpropionate (1.667 g) synthesized in Reference Example B40 was dissolved in ethanol (8 mL) and citric acid monohydrate (0.631 g) was added thereto. The mixture was heated for dissolution and concentrated under reduced pressure. To the residue was added ethyl acetate (23 mL) and the precipitated crystals were collected by filtration, washed with ethyl acetate (12 mL). Methanol (30 mL) was added to the crystals, heated for dissolution and concentrated under reduced pressure. To the residue was added ethanol (30 mL) for dissolution. After standing, the precipitated crystals were collected by filtration, washed with ethanol (10 mL) and dried to give the title compound (2.01 g).
melting point: 176°C (decomp.)
elemental analysis for $C_{39}H_{49}N_5O_{10}$
Calculated (%) :C,62.64 ; H.6.60 ; N,9.36
Found (%) :C,62.50 ; H,6.56 ; N,9.43

**Reference Example B135**

Production method of 2-[6-[3-[4-(diphenylmethoxy)piperidino]-propylamino]imidazo[1,2-b]pyridazin-2-yl]-2-methylpropionic acid dihydrate

4-(Diphenylmethoxy)-1-piperidinepropaneamine (363.6 g, 1120 mmol), ethyl 2-(6-chloroimidazo[1,2-b]pyridazin-2-yl)-2-methylpropionate (200.0 g, 747 mmol) and sodium carbonate (158.4 g, 1490 mmol) were suspended in dimethyl sulfoxide (600 mL) and the mixture was heated in an oil bath (bath temperature 165-170°C) while passing a nitrogen gas, and stirred for 3.5 hours. The reaction mixture was cooled to room temperature and ethyl acetate (2000 mL) and water (2000 mL) were added for partitioning. The organic layer was washed twice with water (1000 mL) and the organic layer was concentrated under reduced pressure. To the residue was added ethanol (1000 mL) and the mixture was concentrated under reduced pressure to give crude ethyl 2-[6-[3-[4-(diphenylmethoxy)piperidino]propylamino]imidazo[1,2-b]pyridazin-2-yl]-2-methylpropionate (588 g) as an oil. This oil was dissolved in ethanol (1400 mL) and sodium hydroxide (59.8 g, 1490 mmol) dissolved in water (600 mL) was added. The reaction mixture was heated to 60°C (internal temperature) and the mixture was stirred for 1 hour. The reaction mixture was concentrated under reduced pressure and to the residue were added water (2000 mL) and ethyl acetate (2000 mL) for partitioning. The aqueous layer was washed twice with ethyl acetate (1000 mL) and ethanol (2000 mL) was added to the aqueous layer. 1N Hydrochloric acid (1000 mL) was added to adjust pH to about pH 6 and the precipitated crystals were collected by filtration, washed with water (800 mL) and ethanol:water (1:1)(800 mL) and dried to give the crude title compound (353.6 g). HPLC purity area percentage 97.7%, yield 82.0%.

To the crude title compound (353.6 g) obtained here was added ethanol (1240 mL) and the mixture was heated under reflux for 1 hour. The reaction mixture was stirred under ice-cooling and the precipitated crystals were collected by filtration, washed with cold ethanol (930 mL) and dried. The obtained crystals were suspended in water (2000 mL) and stirred with heating in a water bath (internal temperature 65-70°C) for 1 hour. The reaction mixture was cooled to room temperature and the precipitated crystals were collected by filtration, washed with water (1000 mL) and dried to give the title compound (276 g).
melting point: 203-205°C (started softening at 110-120°C and solidified again)
elemental analysis for $C_{31}H_{37}N_5O_3 \cdot 2H_2O$
Calculated (%) :C,66.05 ; H,7.33 ; N,12.42
Found (%) :C,66.35 ; H,7.29 ; N,12.39
powder X-ray diffraction analysis results

| spacing: d value (angstrom) | intensity $I/I_0$ (%) |
|---|---|
| 6.94 | 84 |
| 12.88 | 41 |
| 13.72 | 62 |
| 15.10 | 53 |
| 17.56 | 84 |
| 18.70 | 39 |
| 19.24 | 62 |
| 20.66 | 60 |
| 21.06 | 100 |
| 21.76 | 54 |
| 26.42 | 43 |
| 28.24 | 37 |

**Reference Example B136**

Production method of 2-[6-[3-[4-(diphenylmethoxy)piperidino]-propylamino]imidazo[1,2-b]pyridazin-2-yl]-2-methylpropionic acid (anhydride)

2-[6-[3-[4-(Diphenylmethoxy)piperidino]propylamino]-imidazo[1,2-b]pyridazin-2-yl]-2-methylpropionic acid dihydrate (3.20 g, 5.67 mmol) synthesized in Reference Example B135 was dissolved in ethanol (500 mL) by heating and concentrated at atmospheric pressure until the total amount was reduced to 250 mL. The reaction mixture was stood at room temperature, and the precipitated crystals were collected by filtration, washed with ethanol and dried to give the title compound (2.67 g).

melting point: 205-206°C (decomp.)
elemental analysis for $C_{31}H_{37}N_5O_3$
Calculated (%) :C,70.56 ; H,7.07 ; N,13.27
Found (%) :C,70.42 ; H,6.89 ; N,13.32
powder X-ray diffraction analysis results

| spacing: d value (angstrom) | intensity $I/I_0$ (%) |
|---|---|
| 3.20 | 32 |
| 3.48 | 100 |
| 11.62 | 30 |
| 15.46 | 35 |
| 16.60 | 37 |
| 17.56 | 33 |
| 18.46 | 72 |
| 19.26 | 33 |
| 20.12 | 30 |
| 20.58 | 38 |
| 23.38 | 32 |
| 23.40 | 33 |

**Reference Example B137**

Production method of 2-[6-[3-[4-(diphenylmethoxy)piperidino]-propylamino]imidazo[1,2-b]pyridazin-2-yl]-2-methylpropionic acid dihydrate

2-[6-[3-[4-(Diphenylmethoxy)piperidino]propylamino]-imidazo[1,2-b]pyridazin-2-yl]-2-methylpropionic acid (0.250 g, anhydride) synthesized in Reference Example B136 was suspended in water (10 mL) and the suspension was stirred in a water bath (bath temperature 80°C) for 3 hours. The reaction mixture was cooled to room temperature, and the precipitated crystals were collected by filtration, washed with water and dried to give the title compound (0.233 g). 2-[6-[3-[4-(Diphenylmethoxy)piperidino]propylamino]-imidazo[1,2-b]pyridazin-2-yl]-2-methylpropionic acid dihydrate obtained here showed the same data of melting point, elemental analysis, powder X-ray diffraction analysis and the like with the compound obtained in Reference Example B135, based on which the compound was determined to be identical.

**Reference Example B138**

Production of 2-[6-[3-[4-(diphenylmethoxy)piperidino]-propylamino]-3-methylimidazo[1,2-b]pyridazin-2-yl]-2-methylpropionic acid

4-(Diphenylmethoxy)-1-piperidinepropaneamine (17.0 g) and ethyl 2-(6-chloro-3-methylimidazo[1,2-b]pyridazin-2-yl)-2-methylpropionate (7.39 g) were dissolved in N-methyl-2-pyrrolidinone (25 mL) and the mixture was stirred at 160°C for 8.5 hours. After cooling, an aqueous layer was added and the mixture was extracted with ethyl acetate. The extract was washed with brine, dried over magnesium sulfate, and concentrated under reduced pressure. The residue was subjected to silica gel column chromatography and eluted with ethyl acetate:methanol:triethylamine (50: 5:1). The objective fractions were collected, and concentrated under reduced pressure to give ethyl 2-[6-[3-[4-(diphenylmethoxy)-piperidino]propylamino]-3-methylimidazo[1,2-b]pyridazin-2-yl]-2-methylpropionate (10.4 g) as an oil. This oil (10.4 g) was dissolved in ethanol (100 mL) and 5N aqueous sodium hydroxide solution (18.3 mL) was added. The mixture was heated under reflux for 2 hours. After cooling, the reaction mixture was concentrated under reduced pressure. The residue was diluted with water, washed with diisopropyl ether and 5N hydrochloric acid was added to the aqueous layer to adjust its pH to 5. The precipitated crystals were collected by filtration, recrystallized from methanol-water (10:1) and dried to give the title compound (4.09 g).
melting point: 219-220°C
elemental analysis for $C_{32}H_{39}N_5O_3$
Calculated (%) :C,70.95 ; H.7.26 ; N,12.93
Found (%) :C,70.85 ; H,7.00 ; N,13.20

**Experimental Example 1**

Effect on histamine-induced increases in microvascular permeability in skin in guinea pigs

Male Hartley guinea pigs weighing about 500 g were used. After the dorsal hair was shaved under ether anesthesia, 1 ml of a 2.5% pontamine sky blue solution was injected intravenously, and then 0.1 ml of histamine at 3 μg/ml was injected intradermally into 2 sites (left and right) in the back. Thirty minutes after the injection of histamine, animals were fainted by knocking in the head, decapitated and killed by bleeding, after which the skin of the back was removed. The long and short diameters (mm) of each blue spot were measured and multiplied; the mean product was taken as the vascular permeability index. Test compounds were suspended in a 5% gum arabic solution and orally administered in a volume of 0.2 ml/100 g body weight at 1 hour before histamine administration. Animals in the control group received the same volume of a 5% gum arabic solution. The suppression rate of the sample for this reaction was calculated using Equation 1 and the results are given in Table 1.

Equation 1

Inhibition (%) of histamine-induced increases in microvascular permeability in skin = 100 × (1-vascular permeability index in the presence of drug/vascular permeability index in control group)

TABLE 1

| Compound | Inhibition (%) of histamine-induced increases in microvascular permeability, 3 mg/kg oral administration |
|---|---|
| Reference Example B 6 | 91 |
| Reference Example B12 | 91 |
| Reference Example B18 | 91 |
| Reference Example B20 | 92 |
| Reference Example B21 | 91 |
| Reference Example B37 | 92 |
| Reference Example B41 | 92 |
| Reference Example B45 | 91 |
| Reference Example B135 | 91 |

**Experimental Example 2**

1) Preparation of Guinea Pig Eosinophils

To male Hartley guinea pigs, 2 ml of equine serum (Bio-Whittaker, Inc.) was intraperitoneally administered once weekly for 8 consecutive weeks. At 48 hours after final administration, 75 ml of physiological saline was intraperitoneally injected, after which the recovered solution was centrifuged at 400 × g for 5 minutes. The resulting sediment was suspended in 5 ml of Percoll solution (density d=1.07) and layered on top of the discontinuous density layer of Percoll solution (density d=1.112, 5 ml; d=1.095, 10 ml; d=1.090, 10 ml; d=1.085, 5 ml), followed by centrifugation at 1,000 × g for 25 minutes (20°C). The cell layer formed at the interface between density d= 1.112 and density d=1.095 was collected. Erythrocytes present in the collected cell sediment were removed by hypotonic treatment (suspended in water for 30 minutes).

The cell sediment was washed 3 times with Hanks' solution containing 10 mM Hepes (Dojin Kagaku) (Hanks-Hepes) and suspended in a Hanks-Hepes solution containing 2% human serum albumin (Wako Pure Chemical Industry or Sigma) (Hanks-Hepes-HSA) to a concentration of $5.56 \times 10^6$ cells/ml. Eosinophil purity was 90%, viability was over 98%.

2) Determination of Chemotactic Reaction Suppression

To a 24-well culture dish, which serves as a lower chamber, 600 µl of Hanks-Hepes-HSA solution containing $LTB_4$ (final concentration $10^{-8}$ M, Cascade Biochemical Ltd.), was transferred, followed by incubation at 37°C for 30 minutes in a carbon dioxide incubator. Separately, 200 µl of eosinophil suspension ($5\times10^6$ cells/ml), previously incubated at 37°C for 15 minutes, was added to Chemotaxicell (polycarbonate membrane, pore size 3 µm, thickness 10 µm), which serves as an upper chamber, after the upper chamber was attached to the 24-well culture dish. After 2 hours of reaction in the carbon dioxide incubator, the Chemotaxicell was removed; 60 µl of 2% (w/v) EDTA solution in physiological saline was added to the liquid in the lower chamber. After ice-cooling, the cells migrating into the inner solution of the lower chamber were counted using a blood cell counter [Coulter Counter (trade name)]. The test drug, dissolved in N,N-dimethylformamide (DMF), was added to both the upper and lower chambers to a final concentration of $10^{-5}$ M.

Equation 2

Chemotactic reaction suppression rate = [1-(number of migrating cells in the presence of drug/number of migrating cells in the absence of drug)] × 100

suppression rates of $LTB_4$ -induced chemotactic reaction by test drug ($1 \times 10^{-5}$ M concentration) were calculated. The results are shown in Table 2.

TABLE 2

| Compound | Suppression Rate (%) |
|---|---|
| Reference Example B2 | 54 |
| Reference Example B6 | 64 |
| Reference Example B20 | 61 |
| Reference Example B34 | 50 |
| Reference Example B35 | 52 |
| Reference Example B36 | 64 |
| Reference Example B47 | 54 |
| Reference Example B51 | 80 |
| Reference Example B59 | 54 |
| Reference Example B61 | 50 |
| Reference Example B62 | 52 |

**Experimental Example 3**

Effect on intranasal pressure and pituita secretion

1) drug administration

Animals were anesthetized with pentobarbital sodium (30 mg/kg, i.p.), and the compound of Reference Example B135 and solvent (0.1% sodium dihydrogenphosphate + 0.1% Tween 80) were administered into the both nasal cavities of guinea pig in 20 µL. The animals underwent the following test in about 0.5 hour.

2) measurement of intranasal pressure and nasal secretion

The animals under anesthesia was held in the supine position. The neck was opened by median incision to expose the air tube. For maintenance of respiration, a polyethylene tube was inserted down into the air tube. Another polyethylene tube was inserted from the air tube incision into the rhinopharynx, and connected to a respirator via a silicon tube, through which the air was supplied into the nasal cavity at a volume of 4 mL/stroke and a frequency of 70 strokes/min. To prevent the air leak into the oral cavity, incisive canal was closed with Aron alfa A (trademark, Sankyo,

Tokyo). The intranasal pressure was measured with a transducer connected to a side branch of the tube inserted into the nasal cavity. Histamine 1000 μg/mL solution was aerosolized using an ultrasonic nebulizer provided between the respirator and the tube in the nasal cavity, and the air was supplied into the nasal cavity for 3 minutes. After the histamine air supply, the intranasal pressure was depicted on an oscillograph for 15 minutes and the area under the curve was determined.

The pituita flowed out from the nasal cavity or pooled in the nasal cavity during the 15 minutes after histamine air supply was wiped with a cotton swab, which was weighed on an electronic weighing machine.

Where necessary, aerosolized physiological saline as a negative control was supplied into the nasal cavity, and the intranasal pressure and nasal secretion were measured.

3) results

The percent inhibition of increase in the intranasal pressure by the administration of 0.1 and 1.0% nasal drop solutions of the compound of Reference Example B135 is shown in Table 3.

TABLE 3

| | |
|---|---|
| percent inhibition of increase in intranasal pressure by nasal administration of 0.1% solution | 70% |
| percent inhibition of increase in intranasal pressure by nasal administration of 1.0% solution | 62% |

The percent inhibition of increase in the nasal secretion by the nasal administration of 0.1% and 1.0% solutions of the compound of Reference Example B135 is shown in Table 4.

TABLE 4

| | |
|---|---|
| percent inhibition of nasal secretion by nasal administration of 0.1% solution | 72% |
| percent inhibition of nasal secretion by nasal administration of 1.0% solution | 78% |

From these results, the compound of Reference Example B135 was found to have reduced increase in the intranasal pressure and nasal secretion.

**Experimental Example 4**

Penetrability into the brain

1) measurement method

The compound of Reference Example B135 was orally administered (10 or 100 mg/kg) to male Hartley guinea pigs and, one hour later, the guinea pigs were slaughtered by decapitation. The cerebral cortex and lung tissues were removed and homogenized in phosphate buffer solution, followed by centrifugation at 4°C and 1000×g for 10 minutes. The supernatant was further centrifuged at 5000×g for 10 minutes to give crude membrane fractions. The membrane fractions were suspended in a buffer and, after adjusting the protein concentration to 1.0 mg/mL, subjected to binding test with tritium-labeled pyrilamine (2 nM). The inhibitory action on *ex vivo* binding test measured specific binding of pyrilamine in the brain and lung membrane fractions was calculated and used as an index of selectivity to peripheral histamine $H_1$ receptor in the body upon oral administration of the agent.

2) results

The percent inhibition upon oral administration of 100 mg/kg of the compound of Reference Example B135 is shown in Table 5.

TABLE 5

| | |
|---|---|
| percent inhibition of specific binding of pyrilamine in lung membrane fraction | 95% |
| percent inhibition of specific binding of pyrilamine in brain membrane fraction | 44% |

The percent inhibition upon oral administration of 10 mg/kg of the compound of Reference Example B135 is shown in Table 6.

TABLE 6

| | |
|---|---|
| percent inhibition of specific binding of pyrilamine in lung membrane fraction | 95% |
| percent inhibition of specific binding of membrane fraction pyrilamine in brain | -2.9% |

From these results, the compound of Reference Example B135 was found to have poor penetrability into the brain.

**Example 1**

| | |
|---|---|
| Reference Example B135 compound | 1.0 mg active component |
| sodium hydrogenphosphate | 10 mg buffer agent |
| crystalline sodium dihydrogenphosphate | 1.65 mg buffer agent |
| polyvinylpyrrolidone K30 | 50 mg solubilizer |
| con. glycerine | 13 mg isotonicity agent |
| propyl p-oxybenzoate | 0.35 mg antiseptics |
| butyl p-oxybenzoate | 0.1 mg antiseptics |
| sodium hydroxide | appropriate amount pH adjusting agent |
| purified water | to total amount of 1 ml |

The above-mentioned components are admixed to give a nose drop.

**Example 2**

| | |
|---|---|
| Reference Example B136 compound | 1.0 mg active component |
| sodium hydrogenphosphate | 10 mg buffer agent |
| crystalline sodium dihydrogenphosphate | 1.65 mg buffer agent |
| polyvinylpyrrolidone K30 | 50 mg solubilizer |
| con. glycerine | 13 mg isotonicity agent |
| propyl p-oxybenzoate | 0.35 mg antiseptics |
| butyl p-oxybenzoate | 0.1 mg antiseptics |
| sodium hydroxide | appropriate amount pH adjusting agent |
| purified water | to total amount of 1 ml |

The above-mentioned components are admixed to give a nose drop.

**Example 3**

| | |
|---|---|
| Reference Example B135 compound | 1.0 mg active component |
| lactose | 7.0 mg excipient |

The above-mentioned components are admixed to give a nose drop.

**Example 4**

| | |
|---|---|
| Reference Example B136 compound | 1.0 mg active component |

(continued)

| | |
|---|---|
| lactose | 7.0 mg excipient |

The above-mentioned components are admixed to give a nose drop.

**Reference Example C1**

| | |
|---|---|
| Reference Example B135 compound | 0.05 g active component |
| boric acid | 1.2 g buffer agent |
| sodium L-glutamate | 0.2 g buffer agent |
| sodium edetate | 0.005 g stabilizer |
| dibutylhydroxytoluene | 0.005 g stabilizer |
| chlorobutanol | 0.1 g preservative |
| benzalkonium chloride (10 w/v%) | 0.05 mL preservative |
| 1-menthol | 0.008 g refrigerant |
| macrogonal 4000 | 0.4 g solubilizer |
| sodium hydroxide | appropriate amount pH adjusting agent |
| purified water | to total amount of 100 ml |

The above-mentioned components are admixed to give a nose drop.

**Reference Example C2**

| | |
|---|---|
| Reference Example B136 compound | 0.05 g active component |
| boric acid | 1.2 g buffer agent |
| sodium L-glutamate | 0.2 g buffer agent |
| sodium edetate | 0.005 g stabilizer |
| dibutylhydroxytoluene | 0.005 g stabilizer |
| chlorobutanol | 0.1 g preservative |
| benzalkonium chloride (10 w/v%) | 0.05 mL preservative |
| 1-menthol | 0.008 g refrigerant |
| macrogonal 4000 | 0.4 g solubilizer |
| sodium hydroxide | appropriate amount pH adjusting agent |
| purified water | to total amount of 100 ml |

The above-mentioned components are admixed to give a nose drop.

**Industrial Applicability**

A nose drop containing the compound (I), a salt thereof or a prodrug thereof is useful as a superior agent for the prophylaxis or treatment of allergic rhinitis, pollinosis and the like.

**Claims**

**1.** A nose drop agent containing a compound represented by the formula

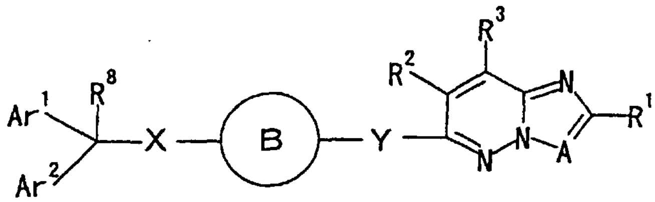

(I)

wherein

| | |
|---|---|
| $Ar^1$ and $Ar^2$ | are each an aromatic group optionally having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally having substituents, an acyl group or a hydroxy group optionally having substituents), |
| $R^1$, $R^2$ and $R^3$ | are the same or different and each is a hydrogen atom, a halogen atom, a hydrocarbon group optionally having substituents, an acyl group or a hydroxy group optionally having substituents, and |
| $R^8$ | is a hydrogen atom, a hydroxy group optionally substituted by a lower alkyl group or a carboxyl group, |

provided that the nitrogen-containing heterocycle represented by ring B is not a heterocycle represented by the formula

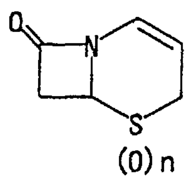

wherein n is 0 or 1, or a salt thereof or a prodrug thereof.

2. The agent of claim 1, which is an antihistaminic and/or eosinophil chemotaxis-inhibiting agent.

3. The agent of claim 1, which is an antiallergic agent.

4. The agent of claim 1, which is an agent for the prophylaxis or treatment of allergic rhinitis.

5. The agent of claim 1, which contains 2-[6-[3-[4-(diphenylmethoxy)piperidino]propylamino]imidazo[1,2-b]pyridazin-2-yl]-2-methylpropionic acid or a salt thereof.

6. The agent of claim 1, wherein the 2-[6-[3-[4-(diphenylmethoxy)piperidino]propylamino]imidazo[1,2-b]pyridazin-2-yl]-2-methylpropionic acid is a dihydrate.

7. A method for the prophylaxis or treatment of allergic rhinitis, which comprises administering, to a mammal, an effective amount of a nose drop comprising a compound represented by the formula

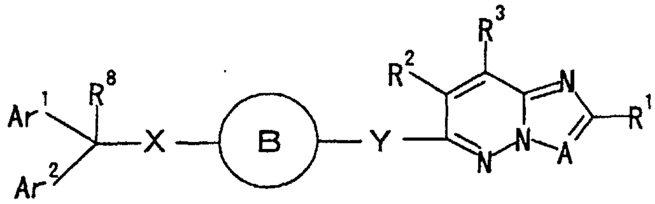

(I)

wherein

| | |
|---|---|
| $Ar^1$ and $Ar^2$ | are each an aromatic group optionally . having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally having substituents, an acyl group or a hydroxy group optionally having substituents), |
| $R^1$, $R^2$ and $R^3$ | are the same or different and each is a hydrogen atom, a halogen atom, a hydrocarbon group optionally having substituents, an acyl group or a hydroxy group optionally having substituents, and |
| $R^8$ | is a hydrogen atom, a hydroxy group optionally substituted by a lower alkyl group or a carboxyl group, |

provided that the nitrogen-containing heterocycle represented by ring B is not a heterocycle represented by the formula

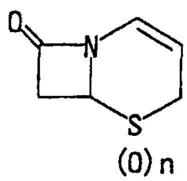

wherein n is 0 or 1, or a salt thereof or a prodrug thereof.

**8.** Use of a compound represented by the formula

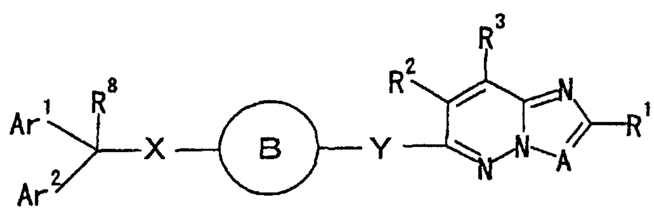

(I)

wherein

| | |
|---|---|
| $Ar^1$ and $Ar^2$ | are each an aromatic group optionally having substituents, $Ar^1$ and $Ar^2$ optionally form a condensed cyclic group together with the adjacent carbon atom, |
| ring B | is a nitrogen-containing heterocycle optionally having substituents, |
| X and Y | are the same or different and each is a bond, an oxygen atom or S(O)p (p is an integer of 0 to 2), $NR^4$ ($R^4$ is a hydrogen atom or a lower alkyl group) or a divalent linear lower hydrocarbon group optionally having substituents and containing 1 to 3 heteroatoms, |
| A | is a nitrogen atom or $CR^7$ ($R^7$ is a hydrogen atom, a halogen atom, a hydrocarbon group optionally |

having substituents, an acyl group or a hydroxy group optionally having substituents),

$R^1$, $R^2$ and $R^3$ are the same or different and each is a hydrogen atom, a halogen atom, a hydrocarbon group optionally having substituents, an acyl group or a hydroxy group optionally having substituents, and

$R^8$ is a hydrogen atom, a hydroxy group optionally substituted by a lower alkyl group or a carboxyl group,

provided that the nitrogen-containing heterocycle represented by ring B is not a heterocycle represented by the formula

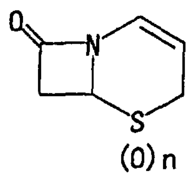

wherein n is 0 or 1, or a salt thereof or a prodrug for the production of a nose drop.

## INTERNATIONAL SEARCH REPORT

International application No.
PCT/JP00/07719

A. CLASSIFICATION OF SUBJECT MATTER
Int.Cl$^7$ A61K31/5025, 9/08, A61P11/02, 43/00, 37/08//C07D521/00, 487/04

According to International Patent Classification (IPC) or to both national classification and IPC

B. FIELDS SEARCHED

Minimum documentation searched (classification system followed by classification symbols)
Int.Cl$^7$ A61K31/5025, 9/08, A61P11/02, 43/00, 37/08//C07D521/00, 487/04

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO, 98/49167, A1 (Takeda Chemical Industries, Ltd.), 05 November, 1998 (05.11.98) & JP, 11-310581, A & EP, 979231, A1 | 1-6,8 |
| Y | JP, 10-226648, A (Towa Yakuhin K.K.), 25 August, 1998 (25.08.98) (Family: none) | 1-6,8 |
| Y | EP, 316633, A1 (ASTA Pharma Aktiengesellschaft), 24 May, 1989 (24.05.89) & US, 5164194, A1 & JP, 1-153639, A | 1-6,8 |
| A | JP, 11-240882, A (Takeda Chemical Industries, Ltd.), 07 September, 1999 (07.09.99) (Family: none) | 1-6,8 |
| A | US, 5482939, A (Takeda Chemical Industries, Ltd.), 09 January, 1996 (09.01.96) & JP, 8-198878, A | 1-6,8 |
| A | EP, 865790, A2 (Takeda Chemical Industries, Ltd.), 23 September, 1998 (23.09.98) & US, 5922712, A & JP, 7-149649, A | 1-6,8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 January, 2001 (16.01.01) | 30 January, 2001 (30.01.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/07719

**Box I Observations where certain claims were found unsearchable (Continuation of item 1 of first sheet)**

This international search report has not been established in respect of certain claims under Article 17(2)(a) for the following reasons:

1. ☒ Claims Nos.: 7
because they relate to subject matter not required to be searched by this Authority, namely:
The invention of claim 7 falls under the category of "methods for treatment of the human body by therapy" as provided for in Rule 39.1(iv) of the Regulations under the PCT.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)